# EUROPEAN PATENT SPECIFICATION

(11) **EP 4 785 339 B1**
(45) Date of publication and mention of the grant of the patent: **09.09.2026**
(21) Application number: 24728306.2
(22) Date of filing: 09.05.2024
(51) Int. Cl.: G16H 20/10, G16B 25/10, G01N 33/50

(54) **VALIDATION OF NATURAL MATRICES FOR THERAPEUTIC USE ON HUMANS, ANIMALS AND PLANTS ON BIO-PHYSICAL BASES AS AN ALTERNATIVE TO VALIDATION THROUGH CHEMICALLY DEFINABLE SUBSTANCES DERIVED FROM SAID MATRICES BY ISOLATION AND/OR SYNTHESIS PROCESSES AND ALSO AS AN ALTERNATIVE TO PRODUCTS COMPRISING NATURAL MATRICES VALIDATED ON THE BASIS OF THEIR TRADITIONAL USE**
NATURMATRICES FÜR THERAPEUTIKA UND PFLANZEN AUF UNSTERBLICHER BASIS
VALIDATION DE MATRICES NATURELLES À USAGE THÉRAPEUTIQUE SUR DES HUMAINS, ANIMAUX ET PLANTES SUR DES BASES BIOPHYSIQUES COMME ALTERNATIVE À LA VALIDATION PAR DES SUBSTANCES CHIMIQUEMENT DÉFINISSABLES DÉRIVÉES DESDITES MATRICES PAR DES PROCÉDÉS D'ISOLEMENT ET/OU DE SYNTHÈSE ET ÉGALEMENT EN TANT QU'ALTERNATIVE À DES PRODUITS COMPRENANT DES MATRICES NATURELLES VALIDÉES SUR LA BASE DE LEUR UTILISATION CLASSIQUE

(30) Priority: 11.01.2024 WO PCT/IB2024/050280
(43) Date of publication of application: 05.08.2026
(73) Proprietor: Bios-Therapy, Physiological Systems for Health S.p.A., 52037 Sansepolcro (AR) (IT)
(72) Inventor: MERCATI, Valentino, 52037 Sansepolcro (AR) (IT); LUCCI, Jacopo, 52037 Sansepolcro (AR) (IT)
(74) Representative: Predazzi, Valentina
(86) International application number: PCT/IB2024/054526
(87) International publication number: WO 2025/149786

(56) References cited:
- US-B2- 11 612 628
- LU X ET AL: "INHIBITION OF PROLIFERATION AND EXPRESSION OF AR/PSA BY HERBAL SUPPLEMENT EQUIGUARDTM IN LNCAP CELLS CULTURED IN ANDROGEN-PROFICIENT FBS AND ANDROGEN-DEFICIENT CHARCOAL-STRIPPED FBS IS CORRELATED WITH INCREASED SERINE-15 PHOSPHORYLATION OF THE TUMOR SUPPRESSOR GENE P53", ANTICANCER RESEARCH, INTERNATIONAL INSTITUTE OF ANTICANCER RESEARCH, GR, vol. 23, no. 3B, 1 May 2003 (2003-05-01), pages 2489 - 2498, XP009063311, ISSN: 0250-7005
- LIN ET AL: "Growth inhibitory and apoptosis inducing effect of Perilla frutescens extract on human hepatoma HepG2 cells", JOURNAL OF ETHNOPHARMACOLOGY, ELSEVIER IRELAND LTD, IE, vol. 112, no. 3, 4 July 2007 (2007-07-04), pages 557 - 567, XP022142073, ISSN: 0378-8741, DOI: 10.1016/J.JEP.2007.05.008
- LIU LANG ET AL: "Integrated Network Pharmacology and Experimental Validation Approach to Investigate the Mechanisms of Radix Rehmanniae Praeparata - Angelica Sinensis - Radix Achyranthis Bidentatae in Treating Knee Osteoarthritis", DRUG DESIGN, DEVELOPMENT AND THERAPY, vol. Volume 18, 1 May 2024 (2024-05-01), United Kingdom, pages 1583 - 1602, XP093219989, ISSN: 1177-8881, DOI: 10.2147/DDDT.S455006
- CHEN RONGQIN ET AL: "Trends in digital detection for the quality and safety of herbs using infrared and Raman spectroscopy", FRONTIERS IN PLANT SCIENCE, vol. 14, 21 March 2023 (2023-03-21), CH, XP093219992, ISSN: 1664-462X, DOI: 10.3389/fpls.2023.1128300
- LI SIYUE ET AL: "Spectrum-effect relationship analysis based on HPLC-FT-ICR-MS and multivariate statistical analysis to reveal the pharmacodynamic substances of Ling-Gui-Zhu-Gan decoction on Alzheimer's disease", JOURNAL OF PHARMACEUTICAL AND BIOMEDICAL ANALYSIS, vol. 237, 115765, 1 January 2024 (2024-01-01), AMSTERDAM, NL, pages 1 - 17, XP093243133, ISSN: 0731-7085, DOI: 10.1016/j.jpba.2023.115765
- LIANG SHAOYU ET AL: "Development and Validation of a Rapid and Specific UHPLC-MS/MS Method for Simultaneous Determination of 21 Bioactive Components in Tiantai No. 1 Pill and Rat Plasma", CHROMATOGRAPHIA, vol. 81, no. 3, 17 January 2018 (2018-01-17), DE, pages 447 - 456, XP093242699, ISSN: 0009-5893, DOI: 10.1007/s10337-018-3470-z
- PATHOMWICHAIWAT THANIKA ET AL: "Alkaline phosphatase activity-guided isolation of active compounds and new dammarane-type triterpenes from Cissus quadrangularis hexane extract", JOURNAL OF ETHNOPHARMACOLOGY, vol. 160, 1 February 2015 (2015-02-01), IE, pages 52 - 60, XP093243695, ISSN: 0378-8741, DOI: 10.1016/j.jep.2014.11.026
- INDRAN INTHRANI RAJA ET AL: "Preclinical studies and clinical evaluation of compounds from the genusEpimediumfor osteoporosis and bone health", PHARMACOLOGY & THERAPEUTICS, ELSEVIER, GB, vol. 162, 25 January 2016 (2016-01-25), pages 188 - 205, XP029556714, ISSN: 0163-7258, DOI: 10.1016/J.PHARMTHERA.2016.01.015
- TILTON ROBERT ET AL: "A comprehensive platform for quality control of botanical drugs (PhytomicsQC): a case study of Huangqin Tang (HQT) and PHY906", CHINESE MEDICINE, BIOMED CENTRAL LTD, LO, vol. 5, no. 1, 30, 20 August 2010 (2010-08-20), pages 1 - 15, XP021081700, ISSN: 1749-8546, DOI: 10.1186/1749-8546-5-30

## Description

The present invention relates to a new method for the compliance validation of batches by defining acceptability values of the spectroscopy spectra by means of spectroscopy analysis of therapeutic or beneficial products, the products comprising or consisting of one or more natural matrices. Products comprising or consisting of one or more natural matrices are characterised by the fact that the matrix or matrices themselves show distinctive emerging properties, i.e. properties that are different from those represented by the sum of the properties of their individual components. The invention also relates to a new process for the compliance validation (batches compliance control in industrial production processes of the product) of one or more batches of said products.

The present invention relates to new batch control methods for products comprising or consisting of a natural matrix or mixtures of vegetal matrices, said products being characterised by the fact that the matrix or matrices themselves show distinctive emerging properties different from those represented by the sum of the properties of their individual components.

### STATE OF THE ART

The historical concept under which patents are granted for the benefit of the public, particularly in matters of health and safety, has roots that date back centuries. The underlying principle is that, while patents provide inventors with a temporary monopoly on their creations, the ultimate aim is to serve the greater good of society.

In contemporary times, this historical concept is reflected in various legal provisions and policies that govern patents. It underscores the understanding that while inventors deserve recognition and protection for their contributions, society as a whole should ultimately benefit from these innovations, particularly in areas critical to public health and safety.

In other words, the humanitarian basis of the patent system lies in its goal to strike a balance between fostering innovation and ensuring that the benefits of that innovation are shared for the betterment of society as a whole. In particular, the patent system should ensure a knowledge sharing and the promotion of progress for the scope indicated above.

In particular, the patent system can play a crucial role in addressing humanitarian and global challenges. For instance, it can incentivize the development of sustainable medicines, environmentally sustainable technologies, and solutions for pressing issues like clean energy and water scarcity.

From the beginning of the 16th century until today, particularly in the field of medical and beneficial products, has been possible to standardise, and hence to validate only artificial substances produced with chemically definable alchemical processes.

This path, which although very reductionist has proven to be of great value, allowing many diseases to be eradicated in the past 5 centuries, is now encountering its limits, which derive from the extraneous nature of chemical substances to vital processes.

Concerning the development of new sustainable medicaments, it is now also ascertained that artificial (in particular, chemically synthesised) therapeutical products are generating harmful impacts on biodiversity and native immune systems.

It is well known that synthetic APIs can enter ecosystems through various routes, primarily through the discharge of pharmaceutical waste from manufacturing plants and improper disposal of unused or expired medications. This can lead to bioaccumulation of artificial and poorly biodegradable substances in aquatic and terrestrial organisms, potentially disrupting food chains and threatening biodiversity.

Studies have shown adverse effects on aquatic organisms, such as altered behaviour, reproduction, and even mortality, because of exposure to synthetic APIs. (Boxall, A. B. et al (2012). Pharmaceuticals and personal care products in the environment: what are the big questions?. Environmental health perspectives, 120(9), 1221-1229); Fick, J., & Lindberg, R. H. (2015). Tysklind, M. and Larsson, D. G. J. (2015). Predicted critical environmental concentrations for 500 pharmaceuticals. Regulatory Toxicology and Pharmacology, 73(1), 607-616.)

It is well known that many synthetic APIs are designed to be biologically active and particularly stable, which, as a result, can hinder their natural degradation processes. Consequently, these molecules persist in the environment for extended periods, potentially accumulating in soils and waters. This reduced biodegradability raises concerns about long-term environmental impacts and the potential for bioaccumulation in organisms [Kasprzyk-Hordern, B., et al (2008). The removal of pharmaceuticals, personal care products, endocrine disruptors and illicit drugs during wastewater treatment and its impact on the quality of receiving waters. Water research, 43(2), 363-380; Verlicchi P., et al (2012). Occurrence of pharmaceutical compounds in urban wastewater: Removal, mass load and environmental risk after a secondary treatment-A review. Science of the total environment, 429, 123-155].

Furthermore, there is growing concern about the potential effects of synthetic APIs on human and animal immune systems. Some pharmaceuticals have been found to interfere with immune activity, either directly or indirectly, leading to altered immune responses or increased susceptibility to infections. This can have significant implications for both individual health and population-level immunity [Vos T. et al (2016). Global, regional, and national incidence, prevalence, and years lived with disability for 310 diseases and injuries, 1990-2015: a systematic analysis for the Global Burden of Disease Study 2015. The Lancet, 388(10053), 1545-1602; Calabrese, E. J., & Baldwin, L. A. (2003). Toxicology rethinks its central belief. Nature, 421(6924), 691-692].

In conclusion, while synthetic APIs have undoubtedly contributed to advancements in healthcare, their environmental and health impacts should be carefully considered. Efforts to develop greener pharmaceuticals, improve waste management, and monitor environmental contamination are crucial steps towards mitigating these concerns.

In addition, it has to be noted that, while being chemically analogous to their synthetic counterparts if taken in isolation, natural molecules within a natural matrix are likely to possess distinct fingerprints with respect to their synthetic analogues, due to the totally different synthetic pathway in terms of primary metabolites, reactants, reaction temperatures, energy sources, catalysts etc., potentially influencing their physicochemical behaviour and reactivity, therefore their biological function.

According to the conventional paradigm, from a chemical-structural viewpoint, the identity of a molecule is embedded in its atomic composition and the geometric arrangement thereof. By way of example, estragole (1-allyl-4-methoxybenzene), which in nature is prominently identified in essential oils such as those derived from *Ocimum basilicum* and *Artemisia dracunculus* is known in the art for its potential aromatic and medicinal application. The molecular constitution and associated energy states of estragole, contingent upon its origin, have been a subject of robust scientific deliberation. While traditional perspectives postulate uniform molecular attributes, a more rigorous scrutiny suggests nuanced differences.

Given this premise, estragole, whether procured from botanical sources via distillation or synthesized in laboratory confines, should ideally be congruent in its inherent physicochemical attributes.

However, it's paramount to distinguish between the pathways of production. In botanical matrices, biosynthesis of estragole is orchestrated by a series of enzymatic reactions, commencing with primary metabolites, and culminating in this specific secondary metabolite. It is known in the art that each of these enzymatic transformations operates within a distinct energy landscape, potentially conferring to the molecule a unique energy state.

Conversely, the laboratory synthesis of estragole hinges on chemical reactions steered by different precursors and conditions (such as temperatures not compatible with the life of a plant). The energy dynamics of such synthetic routes, governed by the thermodynamics and kinetics intrinsic to the reactions, are highly likely to deviate from the plant-mediated enzymatic pathways.

In addition, it is evident that also the isotopic abundances resulting from the two different pathways (natural and synthetic) are unlikely to be the same. Isotopic abundances, even if subtly varied, are known to exert tangible influences on vibrational frequencies, bond strengths, and consequentially, the energy states of the molecule itself [Bigeleisen, J. (1996). Nuclear spin conversion in polyatomic molecules. Journal of Chemical Physics, 105(18), 8121-8129]. Given the likely isotopic disparities between botanical sources and synthetic reagents, the resultant estragole molecules are likely to harbour differential energy imprints and biological activities. In the light of the above, while being chemically analogous, molecules from natural and synthetic origins are reasonably likely to possess distinct energetic fingerprints, potentially influencing their physicochemical properties, reactivity and therefore their biological function. Indeed, the difference between the activity of synthetic and natural estragole has been reported in the art (Suzanne M.F. et al. "Basil extract inhibits the sulfotransferase mediated formation of DNA adducts of the procarcinogen 1'-hydroxyestragole by rat and human liver S9 homogenates and in HepG2 human hepatoma cells" Food and Chemical Toxicology, 2008, 46 (6) 2296-2302, https://doi.org/10.1016/j.fct.2008.03.010.).

In addition, synthetic molecules (intended as molecules obtained through a production carried out by man through chemical synthesis laboratory/industrial processes) are designed in order to provide the desired interaction with a specific given target molecule, said design not taking into account all the interactions that the said molecule may and will have within a natural matrix, with the environment, and with the whole receiving network of the organism in which they will be used. On the other hand, native biosynthesised molecules, being produced in natural, non-artificial settings, will intrinsically carry all the essential features to exist and exert their functions in an epigenetically determined contest whose description is inaccessible when a conventional deterministic chemical approach is used. A possible deciphering of natural matrices and biosynthesised molecules may be provided applying quantum biology.

Products obtained from natural sources have been used for thousands of years to prevent and cure human diseases. In this context, many studies have been limited to characterizing their chemical composition at the monomolecular level and the monomolecular activities, while the spontaneous assemblies, interactions and supramolecular organisation of all the components in said natural products have not been fully investigated and thus understood. Since the development of modern chemistry, the reductionist approach focusing on the isolation of single molecules from natural products and the subsequent artificial synthesis of molecules of therapeutic interest, the aim has been to develop selected active principles that act on a given target following the key-lock paradigm.

This had led to the conviction that research in the field of life sciences was to be aimed at substances that can be chemically validated, with data such as quantities of the individual substances at a molecular level, generating very powerful and effective artificial products, with linear dynamics. It is now becoming evident that this direction is also generating harmful impacts on biodiversity and native immune systems.

The inability to standardise products deriving from natural sources, including, in particular, products that are prepared by humans, but that consist mainly or only of components originating from natural raw (starting) sources i.e. that consist 100% of non-artificial matter, has been one of the major difficulties for technology, thereby opening the doors to the current API-based, pharmacological approach in order to ensure the batch to batch validation of products deriving from natural sources intended for medical or beneficial application.

By way of example, natural matrices, such as plant matrices, are complex systems characterized by many molecular components belonging to different phytochemical classes that interact with each other already in the plant in order to determine the plant's biology. This interaction continues also in the processing phases and different processing techniques affect the post-processing interactions of said components. These compounds can interact at the functional and structural level. Supramolecular aggregates as well as their chemical-physical and structural characteristics that result in both structural and functional networks are dynamic interactions and can be modulated by environmental conditions and, as one can expect, these interactions affect the reactivity of the individual components and, through the so called "matrix effect", result in properties typical of the distinct entity represented by the matrix and are different from the sum of the properties of its single molecular components. Such properties are defined as "emerging properties". This phenomenon has been described and attributed specifically to living matter, which has a drive to self-assemble and self-organize to form supramolecular complex entities [Jean-Marie Lehn Toward complex matter: Supramolecular chemistry and self-organization. PNAS, 2002, 99 (8) 4763-4768 https://doi.org/10.1073/pnas.072065599]. This inherent complexity leads to the fact that individual molecules within a natural matrix cannot be considered as to be contained in isolated and fixed packages, as mutual non-covalent and dynamic interactions continuously occur between them. Such interactions are intra- and intermolecular and occur both among molecules of the same type as well as among molecules belonging to different chemical classes. This introduces the need to consider that the ability of a natural matrix to exert a therapeutic activity on the human body depends not only on the quali-quantitative composition of the matrix, which is by its own nature prone to be variable per se, but also on the presence of such interactions between same and different molecules, including small molecules as well as more complex ones such as proteins, polysaccharides, lipids, RNA, etc.

In this context, the classical validation of therapeutic products based on the pharmacological relationship between structure and activity (SAR) which is the most relevant relationship in classical pharmacological activities between an active pharmaceutical ingredient (API) and the receptor targeted by said API, which is considered at the level of single molecules, is unlikely to be respected across different batches of the same natural matrix.

These considerations appear to profoundly distinguish the study of the interaction between a self-assembled natural matrix and a biological system, imbued with complexity at the molecular and supramolecular level, from the interaction which would be established by an API and its target receptor cellular structure. In fact, the latter is unequivocally defined by the exquisitely deterministic canons of the key-lock mechanism by fixing, also and above all in structural terms defined in both a quantitative and qualitative manner the interaction between the API and its specific target. This concept is so pervasive from a conceptual point of view that it then translates into the possibility of controlling the reproducibility of the biological function of the API through the sole control of the reproducibility of its molecular structure based on their structure-activity relationship (SAR). This is evidently not applicable to natural matrices or products comprising them, due to the characteristics discussed above.

It therefore appears necessary to note fundamental differences between natural self-assembled matrices and APIs:
- the first, which are eubiotic with respect to man and the environment, are characterized by a physiological interconnection at the molecular level with the receiver's biological system and, precisely by virtue of their complexity, are clearly inappropriately described and characterised through the use of deterministic tools such as the key-lock model;
- the latter, which are xenobiotes with respect to man and the environment, are instead characterized by the clear possibility of describing their interaction with the receiver's biological system according to totally deterministic canons, typically summarized by the "key and lock" model.

Therefore, knowledge of the identity and amount of each and every molecule in a natural matrix is not sufficient to predict the dynamic and kinetic properties, as well as the therapeutic effectiveness, of the matrix itself. The opposite happens when selected single molecules, such as APIs, are considered, whereby the SAR, the pharmacodynamic and pharmacokinetic properties are intrinsically related to the chemical identity of the active principle, and to the pharmacodynamic inertia of the excipients. For this reason, the canonical concepts of pharmacodynamics and pharmacokinetics make sense solely when referring to a single molecule (the active principle), or a representative thereof (a functional marker).

The network established among all components of the matrix yielding "the matrix effect" makes it impossible to identify a single marker as representative of the network, because no single component is capable of conveying alone all the properties specific to the matrix, since no single component reflects the interaction between the matrix and the target living organism.

The matrix effect confers the specific and unique properties of the matrix itself or of a mixture of matrices that result in a new different matrix, called emergent properties, which cannot be reconducted to the properties of any of the components taken in isolation. This perfectly reflects the impossibility to correctly study such properties through deterministic chemical methods, commonly used in classical pharmacological chemistry, which, as said above, are well adequate only for single active principles and excipients in pharma settings.

Rather, the dynamic and kinetic behaviour of the matrix is the result of the dynamic network of interactions taking place within the matrix, showing:
- the presence of a great number of components,
- the inability to reconduct the properties of the matrix to the sum of the properties of the single substances
- the impossibility to describe the interaction between the matrix and the receiving organism according to the key-lock paradigm (model), which is the foundation of SAR. At present, human beings are becoming aware that the response to most problems lies in nature itself, and that there is the need of developing processes and methods that allow to understand, and therefore somehow standardise, natural complex entities self-organizing their supramolecular networks, such as natural matrices. These entities are the only ones physiologically compatible with everything that forms creation. Therefore, the need of moving from the Enlightenment-reductionist based validation to a probabilistic approach inspired to the latest evolutions in scientific thinking, thus eventually opposing linear dynamics to circular dynamics. The deterministic chemical approach is therefore not adequate to investigate and monitor matrix properties and quality. Approaches that assess the interactions within complex systems are necessary, to allow, as required, the identification of the features relevant for the reproducibility of the therapeutic properties of the matrix.

Hence the need to turn to approaches inspired by Systems Theory.

The study of such properties, previously not achievable, requires tools such as the "omics" sciences, among which transcriptomics and metabolomics can be identified as of pivotal importance.

Concerning products comprising or consisting of natural matrices, having a therapeutic effect, it is herein reminded that the Medical Device (MD) EU Regulation 2017/745 (Regulation) was officially published in Europe May on 5th, 2017 [REGULATION (EU) 2017/745 OF THE EUROPEAN PARLIAMENT AND OF THE COUNCIL of 5 April 2017 on medical devices, amending Directive 2001/83/EC, Regulation (EC) No 178/2002 and Regulation (EC) No 1223/2009 and repealing Council Directives 90/385/EEC and 93/42/EEC] and introduced a completely new governance into all aspects of the lifecycle of a MD.

The term Medical Device, according to the Regulation, comprises products which do achieve a therapeutic effect but not with a pharmacological, immunological, or metabolic (Ph.IM) mode of action (MOA). The Ph.I.M MOA is the mode of action characterized by a key-lock model where the selected API obeys the rules of SAR and acts on its target receptor. Thus, products comprising or consisting of complex systems such as natural matrices can comply with the Regulation. In particular, the Regulation also indicates that a product which modifies a pathological or physiological state or process through a non-Ph-IM mechanism of action is a MD.

Therefore, the Regulation poses a double problem to be solved: on one side, MDs consisting of materials of natural origin, such as plant matrices and the like, need to undergo a quality control validation in order to be available for therapy, on the other side, there is the need to demonstrate that the therapeutic effect of said products is achieved by means of non-Ph-IM mechanisms of action.

The current validation of products for therapeutic use, based exclusively on reproducibility of their chemical composition, is applicable exclusively to products acting with a Ph-IM mechanisms of action.

Although taking in account the complexity of the natural matrices, the present state of the art is based on the validation of products comprising or consisting of natural matrices for the treatment of a pathological condition (i.e., a product falling within the definition of MD according to EU Regulation) at the chemical level as for APIs. This is in striking contrast with the intimate nature of such products for all the reasons provided above. Tilton et al, Chinese Medicine, Vol 5, no. 1, 2010, Article 30 pages 1-15 disclose a comprehensive platform for quality control of botanical drugs (PhytomicsQC) based on a case study wherein the quality control is performed using analytically sensitive and comprehensive chemical and biological fingerprinting. There is hence the need in the art to develop validation procedures that are based on the acknowledgement of said complexity and that are not limited to the evaluation of the reproducibility of such products based uniquely on their molecular composition. In addition, the present state of the art does not provide methods for the assessment of the mechanism of action of said product.

There is at present a strongly felt need in finding alternatives to the traditional pharmacology but at the same time guaranteeing the validation and standardization of products, comprising or consisting of one or more natural matrices, used for therapeutic purposes.

The provision of methods validating the quality of a product comprising or consisting of natural matrices that are not based on the mere chemical composition thereof, would allow the use in therapy of products of natural origin, having a circular dynamic rather than a linear one, i.e., acting on the overall physiological state altered by a pathological condition rather than on a single alteration. This would result in the development of new research fields and in the possibility of using a whole network (such as a natural matrix) in therapy, said network operating on the network represented by the receiving treated subject i.e., with a circular dynamic rather than a single compound, acting on a single point of the receiving network i.e., with a linear dynamic.

The present invention solves the first problem summarised in the paragraph above.

### SUMMARY OF THE INVENTION

The present invention provides a new method, as defined in the appended claims, for defining the acceptability values of a spectroscopy analysis for the compliance validation of one or more batches of a product, for the treatment of a pathological condition, comprising or consisting of one or more natural matrices; comprising a step in which said acceptability values are calculated on the spectroscopy spectra of a reference standard of said product, said reference standard having an ascertained therapeutic effect in the treatment of said pathological condition, and of one or more different batches of said product, wherein said spectra are defined as acceptable or not acceptable on the basis of selected biological activities exerted in at least one cellular-based assay by said reference standard and said one or more different batches on one or more hallmarks of said pathological condition and not on the mere chemical composition thereof.

In other words, the acceptability values are defined based on the deviation of said aforementioned spectra from the average spectrum obtained from them.

The products that can be validated with the method of the invention are preferably prepared according to good manufacturing practices, hence following standardised procedures to obtain *a priori* a high degree of homogeneity between batches despite the fact that they contain or consist of natural matrices and are therefore obtained from natural products.

As disclosed above, due to the complex interactions within natural matrix or matrices and therapeutic or beneficial (for health) products comprising or consisting of said matrices and the impossibility to ascribe the therapeutical or beneficial effects to specific APIs, there is a clear need in the art, not only for a best practice and standardisation through the whole process of preparation of eubiotic products comprising or consisting of natural matrices, but also for a batch control model of said products that is not based on the identification, quantification and evaluation of the individual chemical entities contained in said matrix or matrices as their therapeutic or beneficial emerging properties are not ascribable to the mere sum of the properties of their individual components of said matrix or matrices. This control model should therefore consider the whole system, allowing batch assessment within context of mutual interactions among all components. This different assessment method is necessary because the properties (emerging properties) of products comprising or consisting of a natural matrix or matrices, in particular a vegetal matrix, an animal matrix or mixtures thereof are due to the very interconnection among all the components of the natural matrices present in this kind of products. This means that, as explained above, as opposed to classical pharmaceutical products, whose activity is defined by a specific API, it is not possible to ascribe the emerging properties typical of natural matrices to a single or few components functional interactions. Indeed, the properties of a natural matrix stem not only from each and every single component therein but also from the supramolecular interconnection among said components including the way said components self-assemble themselves at the supramolecular level, which results in a network of interactions among all the components of the matrix. In other words, in products comprising or consisting of a natural matrix there is not an "active principle" responsible for the therapeutic features of the product, or "excipients" responsible for not interfering with the features of the active principle, as in a typical pharmaceutical product, but there are multiple interconnected and interacting components which are all responsible for the emergent properties of the matrix. "Emergent" is the term most often used to describe the observed integrated features of a system.

Characteristic is also the interaction between the natural matrix and the receiving organism, by way of example, the human body i.e., interactions between the donor network (the matrix or natural material according to the specification) and the receiving network (the body of the subject to whom the product is administered e.g.: the human body). Such interactions bring to the modification of a multitude of interconnected biological pathways in a way that is distinctive of each biological matrix. As opposed to specific APIs, the effect of a therapeutical product comprising or consisting of one or more natural matrices is broad and encompasses so many aspects of the physiology, that the result is that the product affects the overall pathological state rather than modifying a single function contributing to the pathological state. The modification of the overall pathological state is the result of the multitude of biological components constituting the natural material consisting of or comprising one or more natural matrices (i.e., the product), acting in a coordinated way (i.e., emergent properties or matrix effect) by means of both functional and structural interactions.

As discussed above, it is herein also reminded that, while being chemically analogous to their synthetic counterparts if taken in isolation, natural molecules within a natural matrix are likely to possess distinct fingerprints with respect to their synthetic analogues, due to the totally different synthetic pathway in terms of primary metabolites, reactants, reaction temperatures, energy sources, catalysts etc., potentially influencing their physicochemical behaviour and reactivity, therefore their biological activity.

For the reasons depicted above, a preferred standardised eubiotic protocol is used to produce the natural matrices, in particular plant matrices and of the final products to be validated with the methods of the invention. The eubiotic protocol preferably starts from the agricultural production to the final transformation of raw sources designed such as to preserve the basic natural programmatic rules, which have allowed the interconnection between all the components of living things, organic and inorganic, for millions of years is provided herein.

The Applicant's research disclosed herein demonstrates that the classical approach used for standard pharmaceutical products (i.e., quali-quantitative characterisation of the matrix) is not applicable to products providing therapeutic and/or health benefits, when the product comprises or consists of complex natural systems (i.e. natural matrices). As known in the art, one of the major problems linked to the validation of therapeutical product comprising or consisting of one or more natural matrices, lies in that a natural matrix extracted, e.g., from a given plant, is never totally identical to "the same" natural matrix extracted in the same way from another plant of the same species or even variety, from the point of view of the molecular components.

Due to the very nature of natural matrices, contrary to the classical quality control acceptance parameters used for classical pharmaceutical products based on a specific API rule, a degree of variability in the quali-quantitative composition of products comprising or consisting of natural matrices, must be tolerated as a manifestation of the most intimate nature of such entities and their mode of action on the receiving organism; the problem is however to identify how to assess said acceptable degrees of variability.

The present application describes a new and reliable method for assessing spectroscopy or spectrophotometry acceptability values suitable for the quality processes of products, comprising or consisting of complex natural systems, having a therapeutic or healthy effect the acceptability values being based on the biological activities of said kind of products on hallmarks of a given pathological state rather than on the quali-quantitative analysis of specific chemical substances in said products.

The authors of the present invention surprisingly found that, notwithstanding the quali-quantitative differences among natural matrices obtained from different members of the same source (even through the same production procedures), the different molecular entities in said matrices appear to act in a redundant manner with each other both functionally and structurally. This redundance results in a clear maintenance of the biological activity exercised by products comprising or consisting of natural matrices even when the quali-quantitative composition of different batches of said products would not be considered acceptable using classical batch control protocols at present demanded by the legislations designed to regulate deterministically acting APIs. Without being bound to theories, the observed maintenance of the biological activity is likely due to the fact that, as said above, the emerging properties of a natural matrix are due to the matrix network acting as a whole entity with distinctive properties and may not be ascribable to each single molecule as if it were in isolation.

The inventors have hence discovered that different batches of products comprising or consisting of complex natural systems, that, according to the common standard quality control validation techniques based on their quali-quantitative compositions resulted in a determination of non-compliance, surprisingly maintained equivalent biological effects resulting in their desired biological activity notwithstanding their different composition.

Therefore, for therapeutic products comprising or consisting of one or more natural matrices, instead of basing the acceptability values of the quality control on the identification and evaluation of selected individual chemical entities, and/or performing statistical preclearing of spectroscopy data in order to discard outliers classically accepted for APIs, the applicant developed a new method that bases the assessment of quality control parameters, for validating different batches said product, on the analysis of selected parameters that are representative of its biological effect, which is the pivotal feature of a medical device constituted by natural matrices. The assessment of the acceptability values and validation method herein provided, are adequate for the quality control of biological material with therapeutic activity as defined by Regulation 2017/745, reflect product conformity to GSPR 1 of Annex I of said Regulation, specifically in the first lines reciting: "Devices shall achieve the performance intended by their manufacturer and shall be designed and manufactured in such a way that, during normal conditions of use, they are suitable for their intended purpose".

The objects of the invention are defined in the appended claims,

The present invention therefore provides
a method for the compliance validation of one or more batches of a product for the treatment of a pathological condition or for adjuvating homeostasis in an altered physiological state, said product consisting of one or more natural matrices, the method comprising
(a) performing at least one *in vitro* cell-based assay on a reference standard batch of said product having a known therapeutic effect for treatment of said pathological condition, and on one or more test batches of said product, wherein the read-out of said cell-based assay is representative of the modulation of one or more biological activity associated with one or more hallmarks of said pathological condition or of a pathological condition that can develop from said altered physiological state,
   (a1) determining for each of said one or more biological activities the modulation trend thereof for restoring a healthy physiological state and,
   (a2) performing a transcriptomic analysis determining the genes and modulation trend thereof underlying the modification detectable in said pathological condition in both diseased and healthy physiological state for each of said biological activities;
(b) quantifying in terms of numerical values the modulation of said one or more biological activities induced by each batch of step (a) for each cell-based assay read-out and defining as reference values the values calculated for said reference standard batch;
(c) defining as acceptable the test batches whose values calculated in (b) induce a modulation of each measured biological activity in said cell-based assay whose modulus is ≥ to the modulus of the reference value calculated in (b) and defining as non-acceptable the batches whose values calculated in (b) induce a modulation of at least one of said biological activities in said cell-based assay whose modulus is < to the modulus of the reference value calculated in (b);
(d) performing a NIR or RAMAN spectroscopy on said reference standard batch and on said one or more different test batches; and
(e) defining the acceptability values of the NIR or RAMAN spectroscopy spectra thereby obtained, as the variability range of the NIR or RAMAN spectroscopy spectra values of all of said acceptable batches and of said reference standard batch, preferably refined by the spectra of said non-acceptable batches;
(f) performing a NIR or RAMAN spectroscopy analysis of further batches of said product, and
(g) validating each of said further batches for which the obtained spectrum satisfies the acceptability values defined in step (e).

### GLOSSARY

A **"natural matrix"** in the present application refers to a material consisting of a network represented by a broad number of components/constituents obtained (e.g. extracted) directly from a member of the natural kingdom or a naturally occurring portion thereof (i.e. from a natural raw source), without significant processing or synthetic alteration, wherein "without significant processing or synthetic alteration" is intended as processed only by manual, mechanical or gravitational means e.g. by dissolution in water or other naturally occurring solvents, such as water, water-alcohol solutions etc.; by flotation; by extraction with water or other naturally occurring solvents; by steam distillation or by heating solely to remove water or any other naturally occurring solvent; or extracted from air by any means and with the provision that "natural matrix" excludes said member of the natural kingdom or a naturally occurring portion thereof as such. In other words, a natural matrix, or a mixture of natural matrices, are materials obtained from entities that are self-assembled in nature and processed while preserving their native bio-physical characteristics which determine their physiological interaction with other living organisms, such as the human organism. Their emerging properties can be expressed by contributing to the rebalancing of metabolic processes or states of the receiving organism and/or of some organs or tissues alongside the physiological actions that will be activated in each specific context. According to the present invention the natural matrix can be from a material obtained from any source in the life kingdoms i.e., Monera, Protista, Fungi, Plantae and Animalia. The term hence encompasses a plant natural matrix, an animal natural matrix, a fungi natural matrix a protista (archaea or bacteria) natural matrix a monera natural matrix. A natural matrix may also comprise natural inorganic materials such as minerals extracted from natural raw materials. A synonym of natural matrix or one or more natural matrices in the present description is "natural material" as defined below.

An example of naturally occurring portion of an organism may be represented by e.g. roots, leaves, bark, fruit, flower, of a plant or sections thereof, organs, tissues.

In any part of the description the general term natural matrix can be substituted with:
a plant natural matrix or a natural matrix obtained from a plant,
an animal natural matrix or a natural matrix obtained from an animal,
a fungi natural matrix or a natural matrix obtained from a fungus,
a protista natural matrix or a natural matrix obtained from a protista,
a monera natural matrix or a natural matrix obtained from a monera,
or with a plant material and/or extract, an extract from an animal tissue or organ, fungi and/or a fungi extract, or a mixture thereof. In addition, a natural matrix may contain minerals or components obtained from minerals.

Plant is synonymous with herb.

For example, in the context of products comprising or consisting of material of plant origin (obtained from plants) such as herbal supplements a natural matrix would comprise a part of the original, not intentionally altered, preferably unaltered, constituents that are naturally present in the source materials (e.g., extracts of the source material) and therefore contain various organic and inorganic constituents that are found in the aforementioned kingdoms.

The term "natural" matrix emphasizes the retaining the integrity and complexity of a network of constituents/components as in the original natural source, rather than isolating, purifying, or extracting specific molecules or molecular classes through extensive processing or chemical modification.

Due to the supramolecular self-assembly of the constituents/components of a natural matrix, the whole matrix behaves as a complex network that does not interact with a single target molecule but that interacts with a network of recipients (also organised as a network) in the receiving organism. Therefore, the interaction natural matrix-receiving organism is not, as for common pharmaceutical APIs the result of a point-to-point interaction, but the result of an "interactor" networks (i.e. the matrix) "receiver" network (i.e. the organism to whom the matrix is administered) interaction.

The term natural matrix can be also substituted in any part of the description and claims with complex natural system.

Nowhere in the description and in the claims the term natural matrix can be interpreted as "a product of nature", rather, a natural matrix is a product obtained from a natural organism and processed (e.g., extracted) therefrom by techniques that do not substantially alter biological structure and the supramolecular interconnections among the components within the matrix.

**Emerging properties** according to the present description and to the art, the term defines the properties of a natural matrix or of a natural material according to the present specification, i.e. properties that are not represented by the mere sum of properties of each singled out constituent/component of said matrix/material but by the intermolecular interactions among all constituents/components of the matrix/material that are the result of the supramolecular self-assembly of said components/constituents within the matrix/material itself.

"Emerging properties" hence refer to technical effects, such as therapeutic or homeostasis - adjuvating properties (i.e., beneficial effect), that the interactions and relationships among the constituents/components of a natural matrix exert on a receiving living system. Emergent properties are not immediately evident or predictable based solely on the individual characteristics of each constituent/component of the matrix. Instead, they "emerge" as all the constituents/component of the matrix network interact with one another and with the living system receiving network in a dynamic and complex way. Emerging properties have been broadly discussed in the art in various scientific and systems-oriented fields, including physics, chemistry, biology, and complex systems theory.

Key points about emerging properties include:
System Complexity: Emerging properties are associated with systems that exhibit a certain level of complexity. In simple systems, the interactions between constituents/components are limited, and properties are more easily deducible from the properties of individual constituents. In complex systems, however, the interactions between components and their supramolecular organisation can give rise to novel and unexpected features.
Nonlinearity: Emergent properties often result from nonlinear interactions, where the relationship between cause and effect is not proportional.
Holism: The concept of emerging properties emphasizes a holistic perspective, recognizing that the whole system is more than the sum of its parts.

A **product comprising or consisting of one or more natural matrices** (alias "a product comprising or consisting of complex natural system/s") according to the present invention is a product that comprises or consists of one or more natural matrices, herein also defined as a "natural material" i.e., a "material obtained/manufactured/processed from a natural raw source (raw material) or from a member of the natural kingdom" cfr. below.

In any part of the description and the claims "a product comprising or consisting of one or more natural matrices" can be replaced by "a product comprising or consisting of one or more plant matrix" or by "a product comprising or consisting of complex natural system/s" "a natural material or a material of natural origin".

Furthermore, the term "product comprising or consisting of one or more natural matrices" according to the present description can be an intermediate, or the final formulation for intended use (e.g. resuspended dry product) or, in particular when the formulation for intended use is in liquid form, the term can define a dry or a lyophilised or a concentrated form thereof to which water will be added by the user or by the physician in order to prepare the formulation for administration.

Nowhere in the description and in the claims "a product comprising or consisting of one or more natural matrices" can be intended as a product of nature as such. In addition, when a single natural matrix is present in or consists of said product, said natural matrix is obtained (e.g. extracted) from an organism as defined above by technological means; when a mixture of natural matrices is comprised in or consists of the product, said mixture is a mixture of selected natural matrices made by man, and said mixture cannot be found as such in any of the natural products of origin of each matrix contained therein. Therefore, when the product comprises or consists of a plurality of natural matrices, said natural matrices have been combined by man and the resulting product is endowed of new emergent properties.

According to the present description, the expression "**biological activities related to a pathological condition**" refers to a set of biological activities associated to a distancing/deviation from homeostasis which may or may not reach the onset, progression, worsening, of a pathological condition. Hence, the expression "biological activities modifications related to a pathological condition" refers to modulations or changes in the normal (healthy) physiological state of biological activities (processes) within an organism (preferably a human) that are directly associated to an alteration/impairment of homeostasis, up to a pathological state or disease. In other words, it describes the specific adjustments or deviations from the healthy physiological state of a set or "network" of biological activities that occur as a result or that concur to the onset, progression, worsening, of a pathological condition or disease.

By way of example, in the case of diabetes, biological activities related to glucose metabolism, insulin production and management are modified in ways that are directly associated to the pathological condition of diabetes and are hence related to the pathological condition or state of diabetes according to the present description.

**Synthetic** according to the present description has the meaning conventionally accepted in chemistry.

Conventionally, in chemistry, the term "synthetic" refers to the origin or source of a material or substance. Synthetic substances or materials are produced by man through artificial synthesis i.e., through laboratory chemical reactions usually by reacting simpler chemicals to create more complex ones through processes that often use different pathways, temperature conditions, pressure conditions, energy sources and/or catalysers from those used by living organisms.

Examples: Synthetic substances or materials include plastics, pharmaceutical drugs, and many industrial chemicals. For example, nylon is a synthetic polymer made through chemical synthesis, and aspirin is a synthetic drug produced through specific chemical reactions.

An ***in vitro* cell-based assay,** in the present description has the meaning conventionally used in the art, in particular, it refers to an analytical procedure based on cells, for evaluating the cell behaviour and reaction to insults or stimuli, in the context of a disease or of a pathological or pre-pathological condition or of a condition wherein homeostasis is altered. This type of assay is designed to study the biological response of cells in a controlled environment, often in a petri dish or a well plate. According to the invention, suitable *in vitro* cell-based assays are assays whose read out is associated with the modulation of biological activities associated to one or more hallmark of a given pathological or pre-pathological condition or of a condition wherein homeostasis is altered (altered physiological state).

The cells can be derived from humans, animals, or cell lines that mimic specific tissues or organs.

In the context of a disease or pathological/pre-pathological condition, or of a condition wherein homeostasis is altered, a cell-based assay is specifically designed to simulate or mimic conditions related to the disease or pathological/pre-pathological condition or to the condition wherein homeostasis is altered. In particular, cell-based assays can be used to evaluate *in vitro* the therapeutic, adjuvating and/or beneficial activity of different compounds or products. It can involve exposing cells to factors known to be associated with the disease or pathological condition, to potential therapeutic or adjuvating products adjuvating the restoration of the physiological state or using cells that are or have been genetically modified to carry disease or pathological condition-specific traits. The selected cells can be treated to induce the pathological, pre-pathological or altered homeostasis condition or can be cells already presenting the desired altered phenotype.

A **healthy physiological state** refers to the condition of an organism's body, organ, apparatus, system or body district, and its internal processes when they are functioning optimally and within normal parameters for that individual, i.e. the state to which homeostasis tends. A healthy physiological state, in the context of biological activities known to contribute to hallmarks of a given disease or pathological condition, refers to the condition in which said various biological activities are operating optimally and within normal (healthy) parameters. This state is characterized by the absence of significant aberrant cellular or molecular processes associated with the specific disease under consideration.

The term can refer to the hallmarks of a particular disease, which are distinctive features or characteristics that are typically observed in individuals affected by that disease. These hallmarks can include specific cellular behaviours, molecular pathways, or physiological responses that play a key role in the development or progression of the disease.

In summary, a healthy physiological state in the context of a specific disease or pathological condition is a state in which the biological activities related to the known hallmarks of that disease or pathological condition are modulated in a direction that is consistent with a non-diseased (non-pathological) state, in other words, opposed to the diseased (pathological) state.

A healthy physiological state, therefore, also indicates the direction of the modulation of biological activities that are known hallmarks of a pathological condition in homeostasis, i.e., before the onset of a pathological condition, in other words the homeostatic direction of the modulation of a set of biological activities ascribed to a specific system, district, apparatus or organ of a healthy subject. In the present description healthy physiological state and healthy state are used as aliases.

Altered physiological states and altered homeostasis are closely related concepts that describe deviations from the normal functioning and balance of the body's internal environment. While they overlap, there are some distinctions between the two terms:
Altered Physiological States: This term encompasses a broad range of changes in the body's normal functioning, including disruptions in organ systems, biochemical processes, and cellular functions. Altered physiological states can result from various factors such as disease, injury, medication, environmental factors, and psychological stress. Examples include fever, inflammation, hormonal imbalances, and impaired organ function.

Altered Homeostasis: Homeostasis refers to the body's ability to maintain a stable internal environment despite external changes. This stability is achieved through regulatory mechanisms that control variables such as body temperature, blood pressure, pH balance, and blood glucose levels within narrow ranges. Altered homeostasis occurs when these regulatory mechanisms fail to maintain balance, leading to deviations from the body's normal set points. These deviations can be temporary or chronic and may involve compensatory mechanisms to restore balance.

In summary, altered physiological states describe the observable changes in the body's normal functioning, while altered homeostasis refers to the underlying disruption of the body's regulatory mechanisms that maintain internal stability.

An altered homeostasis underlies altered physiological states, as disruptions in homeostatic mechanisms that can lead to physiological imbalances and manifestations of illness or dysfunction.

**Hallmark** of a disease or of a pathological or medical condition according to the present description has the meaning conventionally used in the art. According to the state of the art, hallmarks of a disease are indicators that can mark the progression or control of a given disease or pathological condition. These hallmarks (also called 'key indicators') are typically a set of features or patterns that a physician would monitor, over time, to track the progression or regression of a particular illness. In summary, a hallmark of a disease is a defining feature or characteristic whose modification is indicative of a given medical condition, aiding in its identification, diagnosis, monitoring and understanding. By way of example, for neurodegenerative diseases (NDDs) at least the following eight hallmarks of NDD are known in the art: (pathological protein) aggregation, synaptic and neuronal network (dysfunction), (aberrant) proteostasis, cytoskeleton (abnormalities), (altered) energy homeostasis, DNA and RNA (defects), inflammation (increase), and neuronal cell death (increase). In cancer research, the hallmarks of cancer are a set of distinctive characteristics that are commonly found in cancer cells. These hallmarks include (sustained) proliferative signalling, (evasion of) growth suppressors, (resistance to) cell death, (enabling) replicative immortality, (inducing) angiogenesis, and (activating) invasion and metastasis.

Hallmarks of a disease, biological activities associated to said hallmarks, parameters whose analysis allows to assess the modifications of said biological activities etc. are a framework to study a disease or a pathological or medical condition using an integrated/ holistic approach.

The expression "**natural material**," according to the present description, refers to materials consisting of one or more natural matrices with the provision that said material/s are not found in nature *as such* but are the result of a technical human intervention (such as, e.g., extraction processes, filtration, and the like, i.e., elaborates). These materials are typically obtained from plants, animals, fungi or microorganisms, and minerals through preparation processes that aim to preserve the integrity of the networks within the natural raw sources from which the natural material is prepared. In the present description "natural material" is considered as a synonym of one or more natural matrices. A natural material according to the present description can therefore be a product, such as a product with a therapeutic effect, consisting only of or comprising different natural matrices selectively assembled (that are not combined in such combination in nature) in order to produce a given therapeutic effect thereby forming an "interactor network" whose administration to a subject (i.e. a receiving living network) shows emerging properties providing a therapeutic effect or a homeostasis adjuvating effect, i.e. an effect beneficial to the health of said subject.

"**Having Therapeutic effect**": according to the present description a product having a therapeutic effect is a product which, upon administration to a subject affected by a pathological condition, reduces the severity of the subject's condition (i.e., the severity is at least partially reduced or mitigated), and/or provides some alleviation, mitigation or decrease in at least one clinical symptom and/or in a delay in the progression of said condition or reinstates (completely or in part) a healthy physiological condition in the district affected by said pathological condition.

Having beneficial effect according to the present description encompasses a product whose administration to a healthy subject or a subject which is healthy but not in homeostasis, or to an *in vitro* cell assay representing an adequately healthy status, results in an *in vitro* or *in vivo* evidence of a reinstatement or adjuvation of homeostasis upon administration in said cell assay or in the recipient's system/district/apparatus/organ of interest.

The terms **"prevent," "preventing**,**"** and **"prevention of"** (and grammatical variations thereof) refer to reduction and/or delay of the onset and/or progression of a disease, disorder and/or a clinical symptom(s) in a subject and/or a reduction in the severity of the onset and/or progression of the disease, disorder and/or clinical symptom(s) relative to what would occur in the absence of the methods of the invention. The prevention can be complete, e.g., the total absence of the disease, disorder and/or clinical symptom(s). The prevention can also be partial, such that the occurrence of the disease, disorder and/or clinical symptom(s) in the subject and/or the severity of onset and/or the progression is less than what would occur in the absence of a composition according to the present invention.

Having a "beneficial/healthy/beneficial to health" effect according to the present description encompasses a product whose administration to a healthy subject or to a healthy subject who is not in homeostasis or to an *in vitro* cell assay representing an adequately healthy status, results in an *in vitro* or *in vivo* evidence of a reinstatement or adjuvation of homeostasis upon administration in said cell assay or in the recipient's system/district/apparatus/organ of interest.

The term "**product having (ascertained) therapeutic properties comprising one or more natural matrices**" or "product for the treatment of a pathological condition, said product comprising one or more natural matrices" according to the present invention is a product as defined above wherein the emerging properties of said product provide a therapeutic effect as defined below in this glossary.

The term can be replaced in any part of the description and of the claims by "product having therapeutic activities comprising or consisting of complex natural systems" "formulation having therapeutic properties comprising (or consisting of) complex natural systems" or with "composition having therapeutic properties comprising (or consisting of) complex natural systems" or with "mixture having therapeutic properties comprising (or consisting of) complex natural systems" wherein the term "complex natural systems" can be substituted with "one or more natural matrices" or with "natural material".

The definition applies, mutatis mutandis, to the term "**product having beneficial/healthy/beneficial to health properties comprising one or more natural matrices**".

According to the present description the term **homeostasis** has the meaning conventionally accepted in the art, and refers therefore, to the physiological process by which living organisms maintain a stable internal environment despite external changes. This stability is crucial for the proper functioning of cells, tissues, and organs. The goal of homeostasis is to ensure that the internal conditions of an organism remain within optimal ranges for survival and proper physiological functioning (healthy physiological state). Homeostasis is obtained by organisms by the modulation of a set of biological activities and processes aimed to maintain a healthy physiological state.

A product adjuvating homeostatic processes is a product that modulates biological activities in the direction of a healthy physiological state, hence, a product that is suitable for a healthy individual and that supports the homeostatic mechanisms that contribute to the healthy physiological state and that can be used by a healthy individual to adjuvate the homeostatic regulations of biological activities associated to hallmarks of a given pathological condition.

**Medical device,** also MD, according to the present description is a product as defined above, according to the definition in EU Regulation 2017/745 Article 2 (1) indents 1-3, necessarily used for therapeutic purposes hence, 'medical device' means any ... [omissis] ... material intended by the manufacturer to be used, alone or in combination, for human beings for one or more of the following specific medical purposes:
- treatment or alleviation of disease,
- treatment, alleviation of, or compensation for, an injury or disability,
- modification of a physiological or pathological process or state,
... omissis...
and which does not achieve its principal intended action by pharmacological, immunological, or metabolic means, in or on the human body, but which may be assisted in its function by such means.
'performance' of a medical device means the ability of a medical device as herein defined to achieve its intended purpose as stated by the manufacturer;
'Clinical performance' of a medical device means the ability of a medical device as herein defined, resulting from any direct or indirect medical effects which stem from its technical or functional characteristics, including diagnostic characteristics, to achieve its intended purpose as claimed by the manufacturer, thereby leading to a clinical benefit for patients, when used as intended by the manufacturer;
'Clinical benefit' of the medical device means the positive impact of a device as herein defined on the health of an individual, expressed in terms of a meaningful, measurable, patient-relevant clinical outcome(s), including outcome(s) related to diagnosis, or a positive impact on patient management or public health.

Also, if a composition, (e.g. a contrivance, such as product having therapeutic properties comprising one or more natural matrices), is intended to have a medical purpose, such as diagnosis, treatment, mitigation, or prevention of a disease or to affect the structure or function of the body, and it meets the criteria outlined in the definition, it may be classified as a medical device by the US FDA.

Per Section 201(h)(1) of the Food, Drug, and Cosmetic Act, a device is:
An instrument, apparatus, implement, machine, contrivance, implant, *in vitro* reagent, or other similar or related article, including a component part, or accessory which is:
(A) recognized in the official National Formulary, or the United States Pharmacopoeia, or any supplement to them,
(B) intended for use in the diagnosis of disease or other conditions, or in the cure, mitigation, treatment, or prevention of disease, in man or other animals, or
(C) intended to affect the structure or any function of the body of man or other animals, and which does not achieve its primary intended purposes through chemical action within or on the body of man or other animals and which is not dependent upon being metabolized for the achievement of its primary intended purposes. The term "device" does not include software functions excluded pursuant to section 520(o).

The classification of medical devices within a risk class is typically based on factors such as intended use, indications for use, and risk associated with the device.

A product considered as **having an ascertained or known therapeutic effect** according to the present invention means that it is a product for which at least one batch (hereinafter: reference standard) of said product that shows the desired therapeutic effect at least *in vitro,* e.g., in laboratory settings using cells, organoids, tissues and/or *in vivo* on animal models or clinical trials (i.e. a batch whose desired therapeutic activity has been validated at least *in vitro*) exists.

The expression **having an ascertained or known beneficial/healthy/beneficial effect** according to the present invention means that it is a product for which at least one batch (hereinafter: reference standard) of said product that shows the desired effect of reinstatement/adjuvation of homeostasis in the at least *in vitro,* e.g., in laboratory settings using cells, organoids, tissues and/or *in vivo* on animal models or clinical trials (i.e. a batch whose desired beneficial activity has been validated at least *in vitro*) exists.

A **subject "in need thereof"** as used herein refers to a subject that can benefit from the therapeutic and/or prophylactic effects of the therapeutical compositions. Such a subject can be diagnosed with a disease or disorder, a subject suspected of having or developing a disorder or, and/or a subject determined to be at increased risk of having or developing a disease or disorder.

By the terms **"treat," "treating," or "treatment of"** (and grammatical variations thereof) it is meant that the severity of the subject's condition is reduced, at least partially improved or ameliorated, and/or that some alleviation, mitigation or decrease in at least one clinical symptom is achieved and/or there is a delay in the progression of the disease or disorder.

**Compliance validation** refers to the validation of the batch-to-batch compliance in the production process of a given product, i.e., the different batches obtained in production maintain their compliance with the reference standard (also defined as "gold standard" in industrial production processes) desired therapeutic or beneficial activities.

**Acceptability values** (also known as **acceptability ranges or cutoffs**) of the spectroscopy or spectrophotometry spectra are defined as the variability acceptable range from the average spectrum obtained from the reference standard batch spectrum of a product and the spectra of the acceptable batches of said product and are preferably refined by spectra of non-acceptable batches according to (c) of the method of the invention, under which the deviation value calculated by overlapping spectrum of unknown batch of product tested (not validated through cell-based assay) on the average spectrum is deemed to maintain its compliance with a reference standard of said product, i.e. to maintain the desired formulation and therapeutic/beneficial activity. As already stated in the description above, they are defined based on the deviation of said aforementioned spectra (of c) from the average spectrum obtained from them.

The **'weakest performance'** of a reference standard vs. a reference drug (i.e., a drug commonly prescribed for the treatment of the same pathological condition) refers to the least therapeutic or least beneficial modulation value of a given biological activity calculated when comparing the same modulation value in a cell-based assay according to the invention.

**A pathophysiological or physio pathological state** refers to an abnormal physiological condition or process within the body that is typically associated with disease or dysfunction. In the present description and claims it is also defined "pathological state". It involves the study of the functional changes that occur as a result of disease or injury and how these alterations manifest at the cellular, tissue, organ, and systemic levels. Pathophysiology encompasses the understanding of both the underlying mechanisms of disease and the body's response to these disruptions to diagnose, treat, and manage various health conditions. Understanding pathophysiological states is crucial in medicine for both research and clinical practice.

Pathophysiology involves the study of how various factors, such as genetic abnormalities, environmental influences, or disease processes, disrupt the normal physiological functions of the body, leading to the development of health disorders or diseases. Understanding physio pathological states is essential for diagnosing, treating, and managing a wide range of medical conditions across different specialties in healthcare.

**Different batches or lots of a product** in the present description as in the state of the art, refer to distinct groups of items produced or manufactured at different times or under different conditions, but still belonging to the same product line. In different batches, the starting raw materials can be from the same stocks or from different stocks. Each batch or lot typically receives a unique identifier which distinguishes it from other batches. These identifiers help in quality control, inventory management, and traceability throughout the production process and supply chain.

Batches of products comprising one or more natural matrix or consisting of one or more natural matrix are expected to vary in their qualitative and quantitative chemical composition due to factors like raw material variations. In addition, the batches can vary as any other product batch, due to production conditions, or equipment used.

### DETAILED DESCRIPTION OF THE FIGURES

Legend of the batches:
Figures 2-7 and 12:
   Product Arté-Gx in lyophilised form (see detailed composition of the product in Example 1):
   Batch 20B1955 also L 20B1955 reference standard (indicated in the figures as Gold Standard)
   Batch 20B0596 also L 20B0596
   Batch 20I1297 also L 20I1297
   Batch 21E1640 also L 21E1640
   Batch 20J1770 also L 20J1770
   Batch Dest 21E1640
Figure 9 Product B reference standard (indicated in the figures as Gold Standard)
Figure 11 Product C reference standard (indicated in the figures as Gold Standard) (see detailed composition of the products in Example 1).

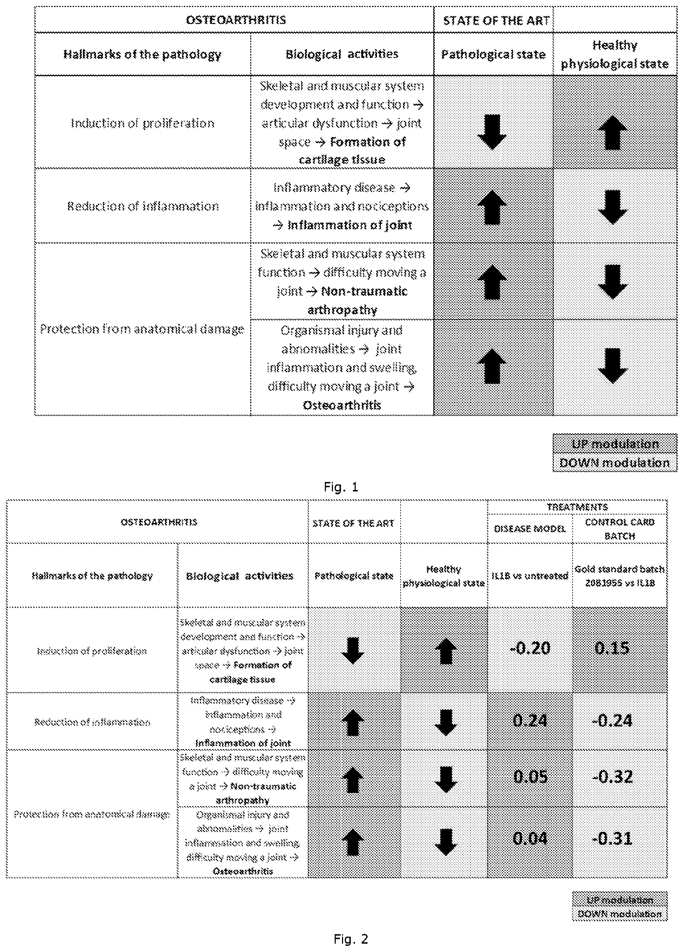

In the figures 2-4, 9 and 11, the modulations values were calculated as Z-scores as described in the specification and in the examples).

Figure 1 Exemplification of hallmarks of osteoarthritis including the trend of the hallmark representing an improvement of the diseased state, (column 1), biological activities concurring to each disease (column 2) and modulations thereof in the pathological conditions (column 3) and modulation of each of said biological activity representative of a healthy physiological state (column 4) dark grey: up modulation light grey: down modulation.

Figure 2 Modulation of selected biological activities in a chondrocytes cell-based assay in osteoarthritis: column 1 hallmarks including the trend of the hallmark representing an improvement of the diseased state, column 2 biological activities, column 3 predicted modulation of biological activities in pathological state, column 4 desired modulation of biological activities in healthy physiological state, column 5 cell-based assay without therapeutic treatment, representative of the biological activities modulation of the pathological state, column 6 modulation induced by the reference standard of the tested product . The cell-based assay shows that the reference standard modulates the selected activities according to the healthy physiological state. The numbers reported in each square represent a Z-score calculated with the method of the invention representing the modulation of each biological activity observed.

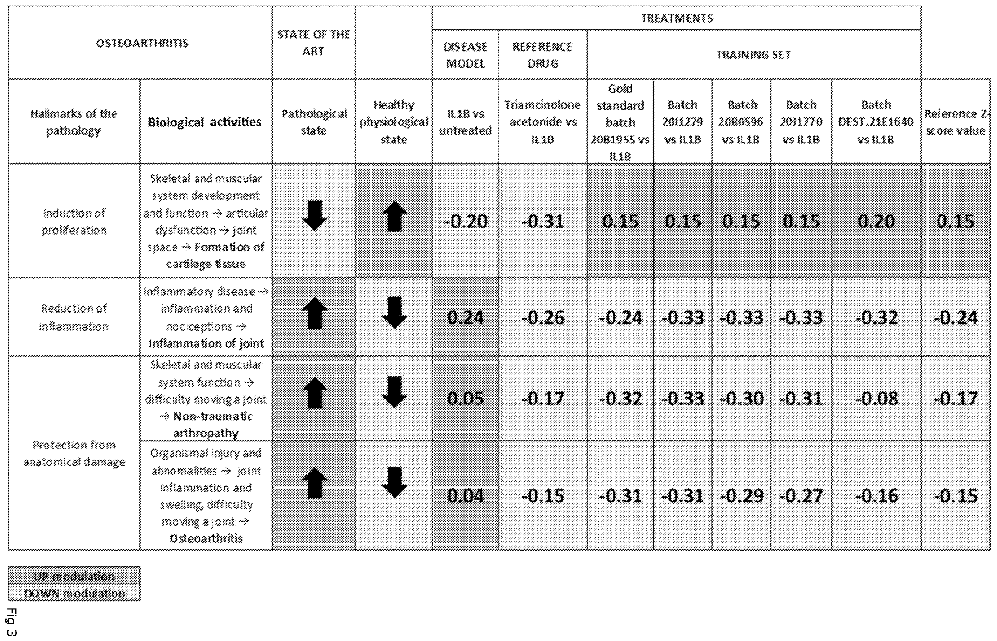

Figure 3 Modulation of selected biological activities in a chondrocytes cell-based assay representative of osteoarthritis: column 1 hallmarks including the trend of the hallmark representing an improvement of the diseased state, column 2 biological activities, column 3 predicted modulation of biological activities in pathological state, column 4 desired modulation of biological activities in healthy physiological state, column 5 cell-based assay without therapeutic treatment, representative of the biological activities modulation of the pathological state, column 6 modulation induced by a reference drug (Triamcinolone Acetonide), column 7 modulation induced by the reference standard of the tested product, columns 8-11 modulation induced by four different batches of the tested product. Column 11 represents the modulation induced by an intentionally destabilised batch (DEST 21E1640) showing weaker therapeutic activity. The cell-based assay shows that the reference standard and the additional batches similarly modulate the selected activities according to the healthy physiological state. The numbers reported in each square represent the Z-score value calculated representing the modulation of each biological activity observed.

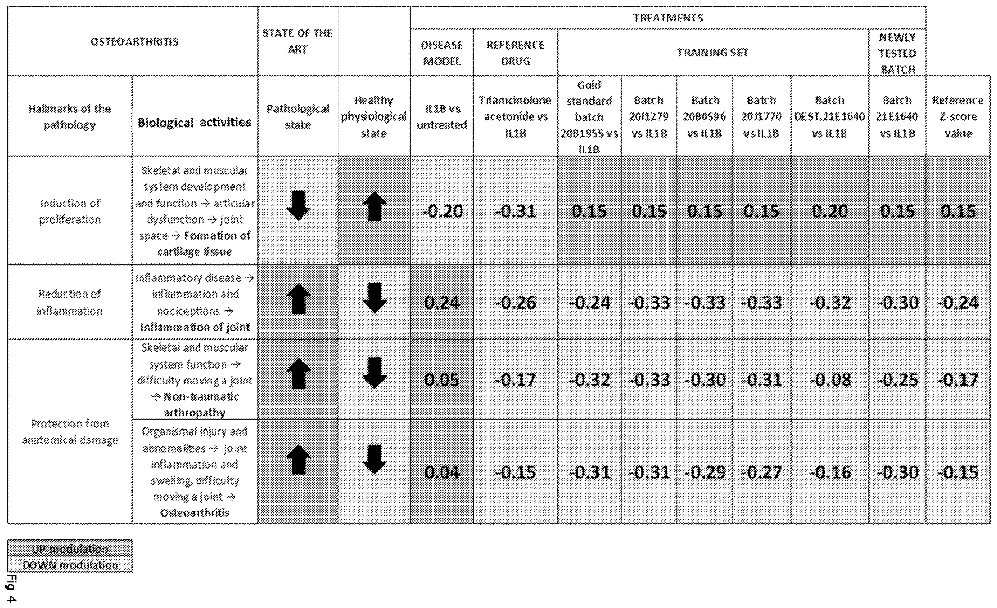

Figure 4 Corresponds to figure 3 plus column 12 showing the control analysis of the modulation induced by the non destabilised batch (21E1640) that was validated as compliant according to the process of the invention. The cell-based assay shows that the reference standard and the additional batches similarly modulate the selected activities according to the healthy physiological state and therefore confirms the compliance of the batch that was validated with the method of the invention. The numbers reported in each square represent the Z-score value (modulation value) calculated representing the modulation of each biological activity observed. The reference Z score values were calculated according to the invention considering the Reference standard Z-score values (column 8 of figure 4) and the drug Z-score values (column 9 of figure 4) selecting as reference Z-score value (column 13 of figure 4) for each modulated biological activity the Z-score value associated to the weakest performance as depicted in the table below (gold standard=reference standard):

| **Hallmarks of the pathology** | **Biological functions** | **Reference drug Triamcinolone acetonide Drug Z-score values** | **Gold standard Z-score values** | **Reference Z-score values** |
|---|---|---|---|---|
| Induction of proliferation | Skeletal and muscular system development and function → articular dysfunction → joint space → **Formation of cartilage tissue** | -0.31 | **0.15** | **0.15** |
| Reduction of inflammation | Inflammatory disease → inflammation and nociceptions → **Inflammation of joint** | -0.26 | **-0.24** | **-0.24** |
| Protection from anatomical damage | Skeletal and muscular system function → difficulty moving a joint → **Non-traumatic arthropathy** | **-0.17** | -0.32 | **-0.17** |
| | Organismal injury and abnomalities → joint inflammation and swelling, difficulty moving a joint → **Osteoarthritis** | **-0.15** | -0.31 | **-0.15** |

As the therapeutic effect is ascertained both for the product as well as for the drug, it is correct to accept, when both the reference drug and the reference standard provide modulation of a selected biological activity in the same direction of the healthy physiological state, the weakest performance z-score as reference cut-off for that modulation.

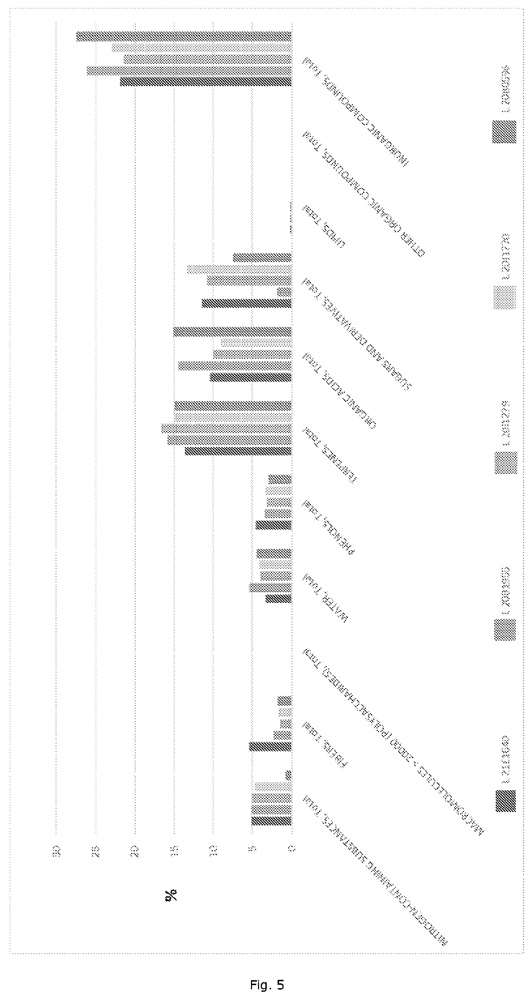

Figure 5 Targeted metabolomics of five batches (including reference standard) of Arté Gx main chemical classes. The figure clearly shows that each batch tested differs from each other and from the reference standard in the quali-quantitative composition. Comparison with tests of the selected biological activities of the same batches on the cell-based-assay (figures 3 and 4), shows that the quali-quantitative analysis of different batches of a product for the treatment of a pathological condition, the product comprising or consisting of one or more natural matrices does not allow to correctly estimate its activity profile.

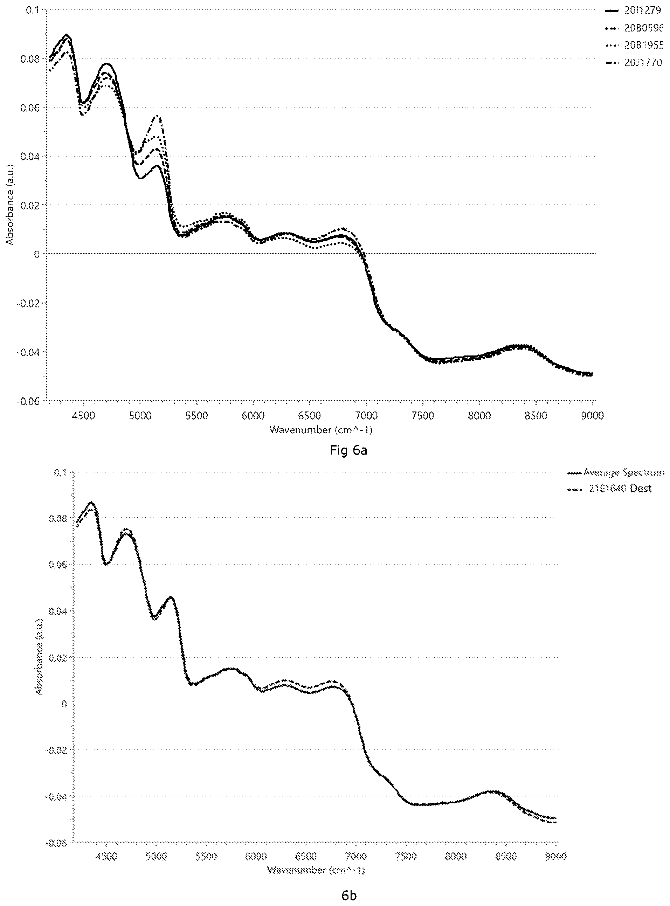

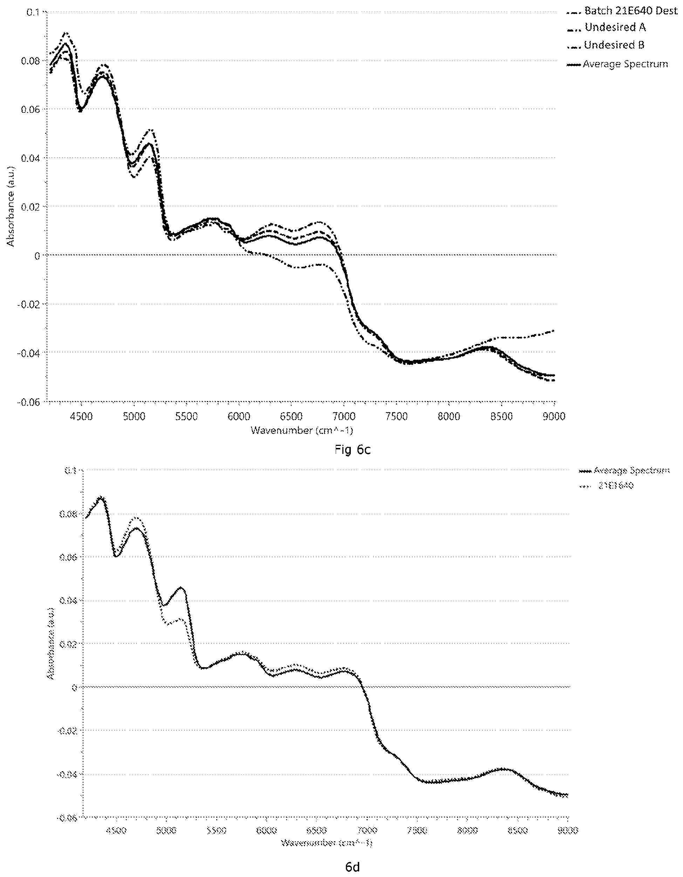

Figure 6 6a. NIR spectrum of the reference standard and three different positive batches selected in iv) in the wavelength range of interest. 6b. NIR average spectrum of the four batches of 6a. and NIR spectrum of negative batch 21E1640 Dest, 6c. NIR average spectrum of the four batches of 6a.and two a priori undesired spectra (undesired A Centella asiatica extract, undesired B Echinacea extract) 6d. NIR average spectrum of the four batches of 6a. and NIR spectrum of unassessed batch 21E1640 (not destabilised).

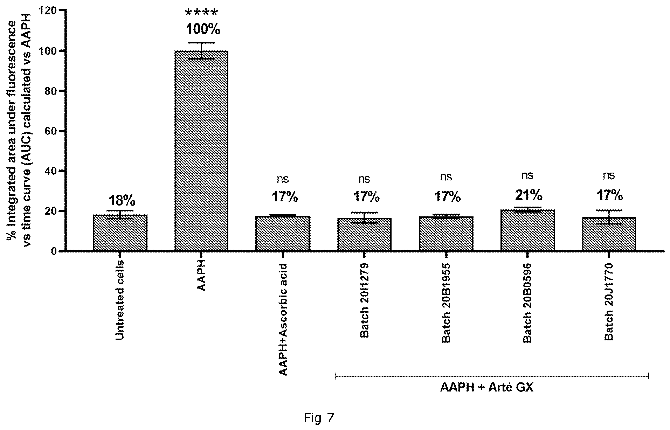

Figure 7 ROS scavenging activity of 4 validated batches including reference standard of Arté-Gx. The quantity of ROS detected in samples treated with AAPH was considered as the maximum release of ROS (100%) and the quantity of ROS detected in the other samples was calculated as percentage with respect to the maximum release induced by AAPH. One-way ANOVA and Dunnett's post-test were applied. Only values capable of returning p<0.05 were considered significant (*p<0.05, **p<0.01, ***p<0.001 , ****p<0.0001). Statistical significance of all samples vs untreated cells.

The figure demonstrates that the validated batches are, indeed, inducing ROS scavenging activity as desired for the intended therapeutic use.

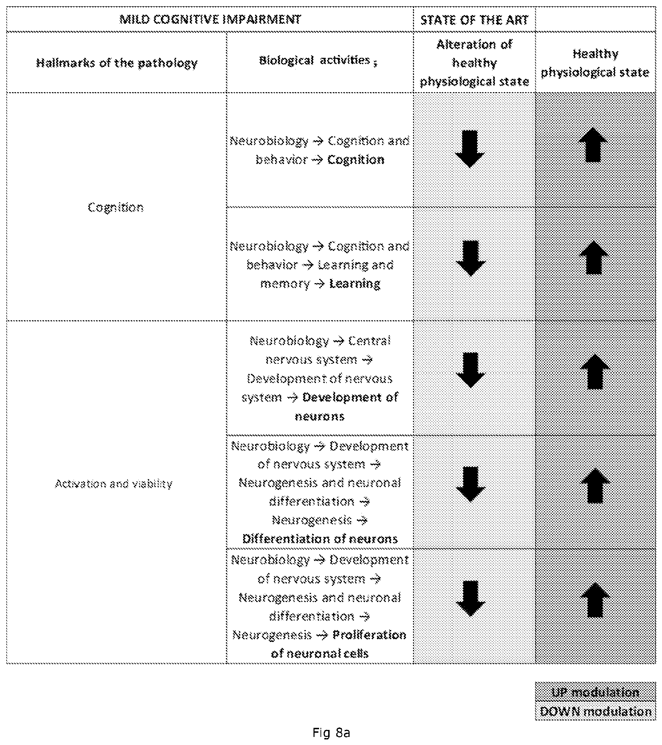

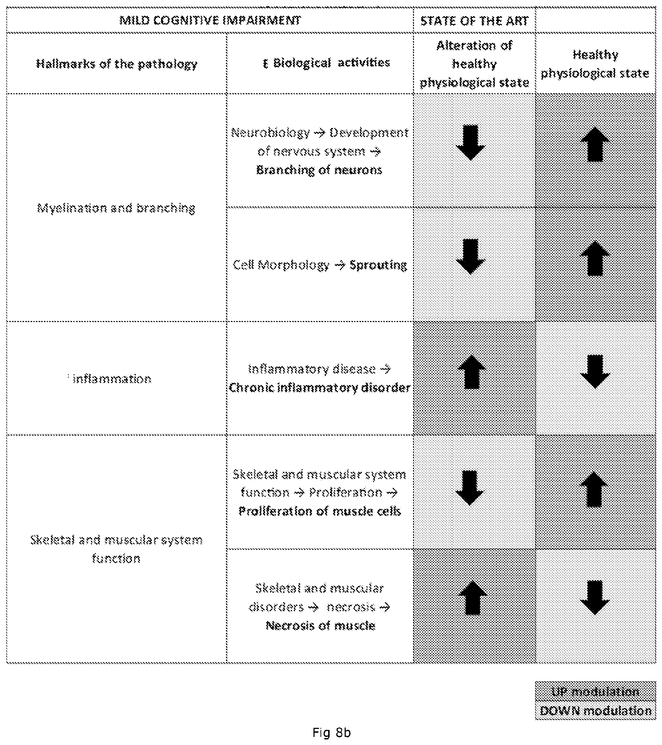

Figures 8a and 8b Exemplification of hallmarks of mild cognitive impairment (MCI), (column 1), biological activities concurring to each disease (column 2) and modulations thereof in the pathological conditions (expressed as alteration of healthy physiological state) (column 3) and modulation of each of said biological activities representative of a healthy physiological state (column 4) dark grey: up modulation light grey: down modulation.

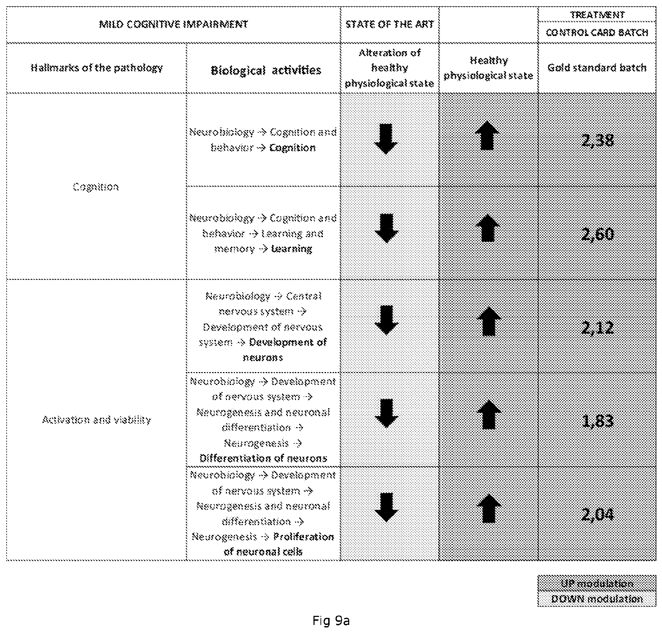

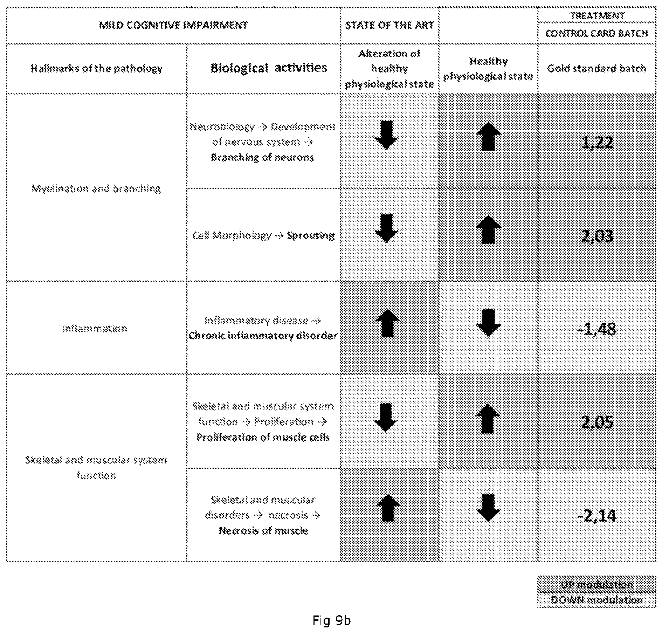

Figures 9a and 9b MCI. Modulation of selected biological activities in a human neuronal cell-based assay (cells SH-SY5Y): column 1 hallmarks, column 2 biological activities, column 3 predicted modulation of biological activities in pathological state, column 4 desired modulation of biological activities in healthy physiological state, column 5 modulation induced by the reference standard of the tested product B. The cell-based assay shows that the reference standard modulates the selected activities according to the healthy physiological state. The numbers reported in each square represent a Z-score calculated with the method of the invention representing the modulation of each biological activity observed.

In figures 9a and 9b the tested product B used is described in example 1.

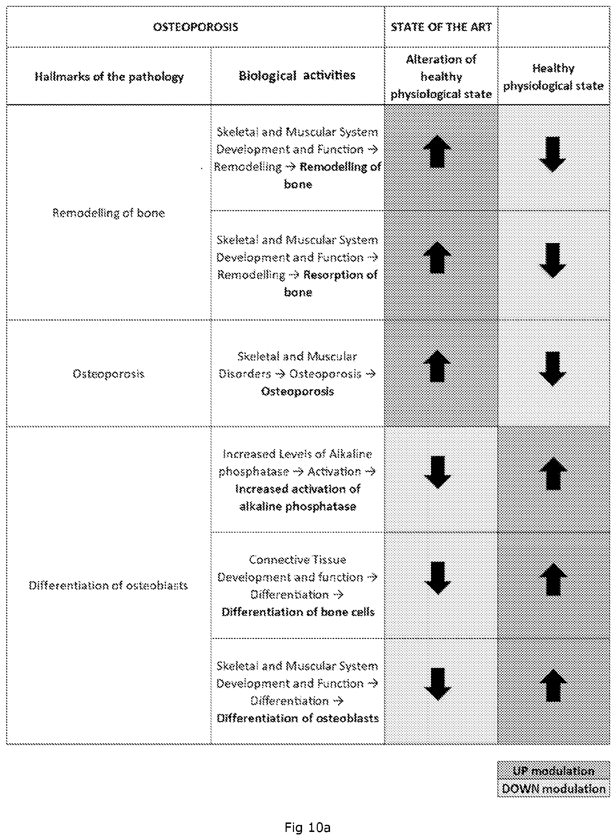

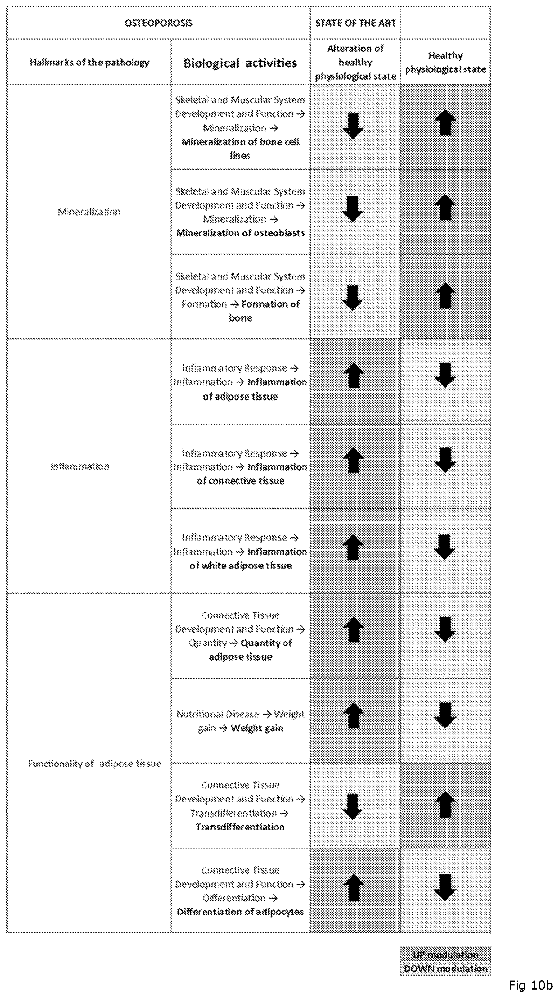

Figures 10a and 10b Exemplification of hallmarks of osteoporosis (OP) including the trend of the hallmark representing an improvement of the diseased state, (column 1), biological activities concurring to each hallmark (column 2) and modulations thereof in the pathological conditions (expressed as alteration of healthy physiological state) (column 3) and modulation of each of said biological activities representative of a healthy physiological state (column 4) dark grey: up modulation light grey: down modulation.

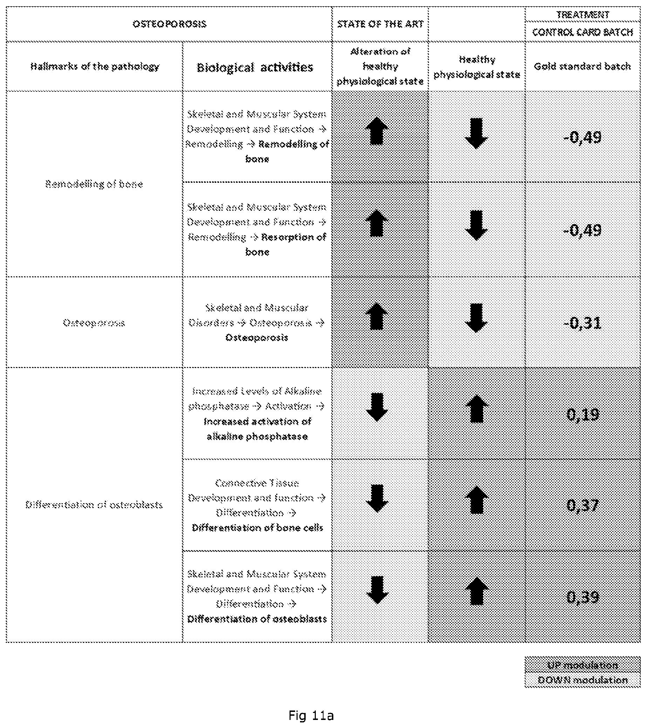

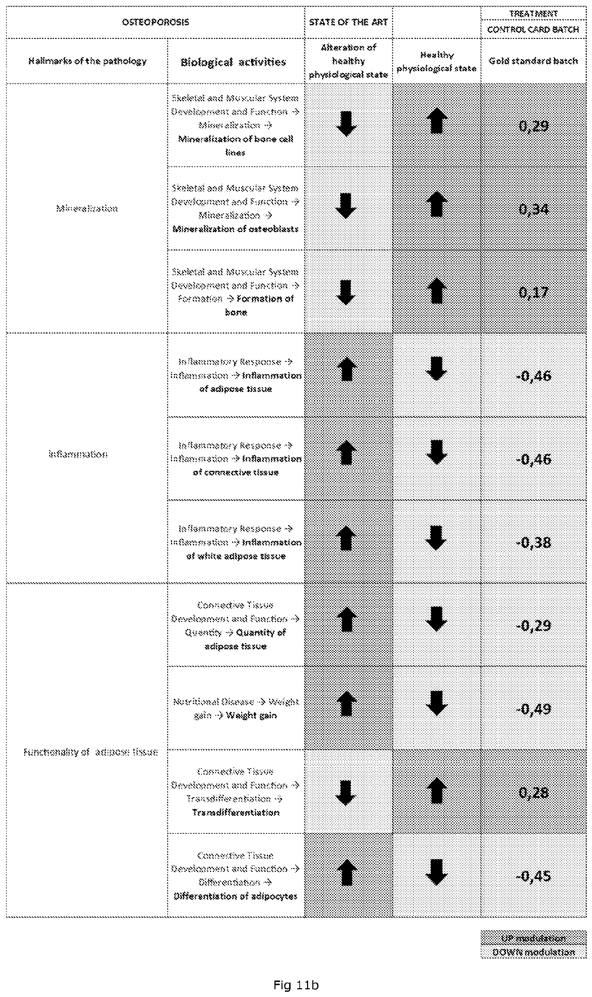

Figures 11a and 11b OP. Modulation of selected biological activities in adipocyte-derived, mesenchymal stem cell lines (hADMSC), capable of differentiating into osteoblasts and mineralize the extracellular matrix (ECM): column 1 hallmarks, column 2 biological activities, column 3 predicted modulation of biological activities in pathological state, column 4 desired modulation of biological activities in healthy physiological state, column 5 modulation induced by the reference standard of the tested product C. The cell-based assay shows that the reference standard modulates the selected activities according to the healthy physiological state. The numbers reported in each square represent a Z-score calculated with the method of the invention representing the modulation of each biological activity observed.

In figures 11a and 11b the tested product C used is described in example 1

Figure 12
modulation of the biological activity oxidative stress (hallmark inflammation) of 4 validated batches including reference standard of Arté-Gx. Ascorbic acid has been used as positive control and insult with AAPH as negative control representative of pathological state. As clear from the figure, also the use of a different parameter for monitoring the modulation of the biological activity causally related to the pathological hallmark of osteoarthritis (OA) is suitable for validating the batches as an alternative to transcriptomics parameters.

### Figure 13

Biophysical characterization of biological vegetal material total RNA.

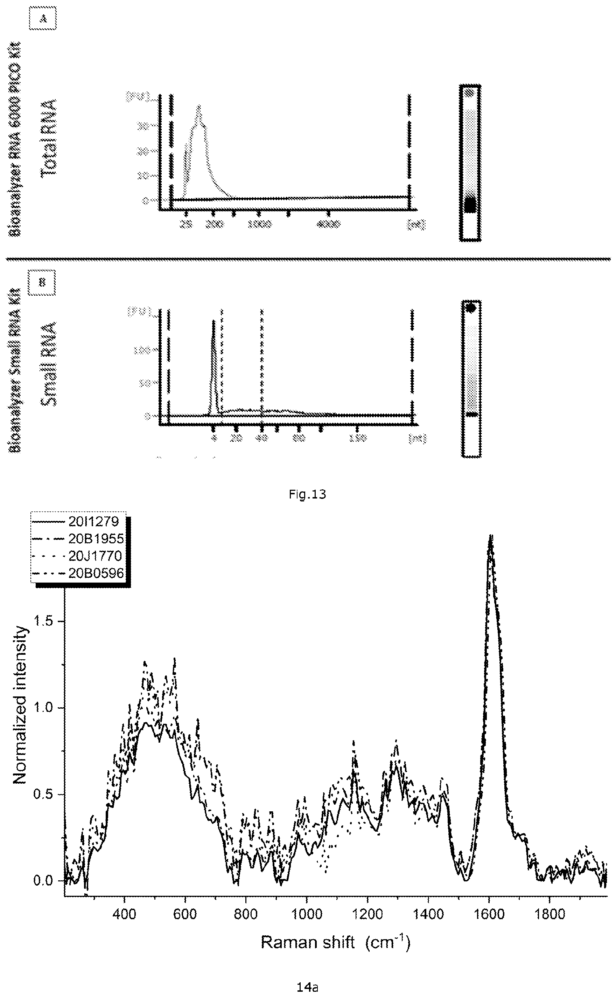

Figure 13 shows the electropherogram of the production intermediate of product A, Arté GX, i.e., the Centella asiatica and Echinacea water coextract in the proportions depicted in example 1, before ultrafiltration, panel "A" shows the size distribution of the total RNA and panel "B" shows the RNA size distribution between 4 and 150 nt.

### Figure 14

14a RAMAN spectrum of the reference standard and three different positive batches selected in iv) in the wavelength range of interest. 14b. RAMAN average spectrum of the four batches of 14a. and RAMAN spectrum of negative batch 21E1640 Dest, 14c. RAMAN average spectrum of the four batches of 14a.and two a priori undesired spectra (undesired A Centella asiatica extract, undesired B Echinacea extract) 14d. RAMAN average spectrum of the four batches of 14a. and NIR spectrum of unassessed batch 21E1640 (not destabilised).

### DETAILED DESCRIPTION OF THE INVENTION

The present invention provides methods and processes as defined in the appended claims for the batch to batch validation of the quality compliance of products comprising or consisting of natural matrices, such as products consisting of 100% natural material and exerting therapeutic or beneficial activity ( the latter by allowing the homeostatic rebalancing of physio-pathological states or slight imbalances in living beings such as the human organism), through the provision of spectroscopy (NIR or RAMAN) acceptability values based on measurements of the modification of specific biological activities concurring to the therapeutic or beneficial effect. The Applicant herein hence provides for the first time a method and a process which will allow to standardise said kind of products and therefore allow to comply with the regulatory frameworks for granting a viable space for innovative uses of said products in therapy. In the past decades the Applicant did pursue an intersectoral research path which, along with scientific innovations, resulted in the development of eubiotic protocols for agricultural production and transformation processes of raw sources (starting materials) to preserve their basic natural programmatic rules, which have allowed interconnection for millions of years among all the components of living things, organic and inorganic.

Following the teachings of the present invention it is now possible to assess precise parameters to validate the batch-to-batch compliance of products with emerging properties resulting in a physiological therapeutic or beneficial activity, when the product comprises or consists of one or more natural matrices.

This is of particular interest for all the products for therapeutic/beneficial use that are not based on classical pharmacological formulation "APIs plus excipients", such as products that comprise or consists of one or more natural matrices. The main problem with this kind of products is that, notwithstanding the important therapeutic/beneficial effects exerted, in the absence of repeatable and precise methods for a batch to batch validation, ensuring the compliance of the product with the claimed therapeutic/beneficial effect, which, due to their very origin (nature) present an intrinsic variability in their quali-quantitative composition, cannot be correctly assessed by classical validation processes based on a mere quali-quantitative composition. Up to now, in fact, it has not been possible to bring products comprising or consisting of one or more natural matrices in a regulatory framework that would take this into account and allow for a high degree of innovation. At present, this kind of products are validated following standard procedures such as quantification of one or more chemical class of compounds which does not represent the complexity and full effectiveness of products with emerging properties such as natural matrices-based products. This problem is solved by the present invention.

As the method and process of the invention, as defined in the appended claims, are designed for products comprising or consisting of natural matrices, it is preferred that the production of these products is carried out standardising the entire production process both in the agricultural and manufacturing processes.

The inventors herein demonstrate that an approach based exclusively on reproducibility assays with classical targeted metabolomics, such as the quali-quantitative analysis of a number of chemical classes or of specific chemical compounds of a product with therapeutical or beneficial effects, judged using criteria that are designed for the management of single APIs acting via the deterministic key-lock mechanism thanks to the existence of a clear SAR, s not suitable for a quality control when the product comprises or consists of one or more natural matrices i. In fact, the results obtained by the inventors from the analysis of the chemical classes of different batches of the same product, when confronted with selected biological activities (underlying the therapeutic or beneficial effect) of each batch, show that the quantitative fluctuations of the individual chemical classes of substances that are present in said different batches, if used as the means for assessing the quality of said batches according to criteria applied to APIs would lead to an a priori assumption that these batches have a different biological activity from one another (see example 3.1 second table and figure 5). The experiments carried out by the inventors on different batches of a therapeutic product comprising one or more natural matrices (cfr. example 3.1 and, in particular, figures 3, 4 and 5) on the contrary, demonstrate that, notwithstanding their variable quali-quantitative chemical composition, said batches nevertheless entail the same therapeutic activity, with a resilient behaviour possibly due to the presence of redundancy among the single components at both structural and functional level.

Thus, the experiments carried out by the inventors (in particular, cfr. figures 3, 4 and 5) show that, when considered alone, the quali-quantitative analysis of different batches of a product for the treatment of a pathological condition, does not allow to correctly estimate the activity profile when the product comprises or consists of one or more natural matrices. Hence, the data provided herein demonstrate that, contrary to pharmaceutical products based on traditional (i.e., synthetic chemical) APIs, the quali-quantitative analysis of the individual constituents of a therapeutical product comprising or consisting of one or more natural matrices (i.e. providing a therapeutic effect based on a network to network interaction), when performed as a method for assessing the therapeutic and quality reproducibility of a non-SAR-based entity, is not suitable to evaluate the correct compliance batch to batch of said product.

In fact, given the very nature of a natural matrices, the batch to batch quantitatively variable chemical profiles, would prompt the validator to consider various batches of a product that comprises or consists of one or more natural matrices as a "different material" with respect to a given reference standard of said product, and therefore to discard said batches, while, on the contrary, the results provided by the inventors herein, demonstrate that in biological systems, quali-quantitatively different batches of a given product exert the same relevant effect for their intended therapeutical use. This is another demonstration that the therapeutic or beneficial activity of a complex matrix cannot be traced back to the sum of the activities of each single molecule within the matrix itself and that, when natural matrices are involved, differently from classical APIs, the reproducibility of the therapeutic or beneficial activity of the matrix does not exclusively depend on reproducibility of the identity and quantity of the molecular components which constitute it.

The results obtained by the inventors demonstrate that there are both structural and functional redundancy mechanisms within natural matrices that confer to said matrices a particular functional resilience, and that that matrix per se should not be perceived as a compilation of molecules acting independently from one another as if they were still subject to the SAR that is commonly attributed to such molecules when they are studied in isolation.

As shown in the present description (see examples and figures), different batches of a tested product that comprises or consists of one or more natural matrices, with ascertained differences in their quali-quantitative composition one from the other and from a reference standard of said product, can retain the ability to mediate the same therapeutic or beneficial activity notwithstanding their different quantitative compositions at the molecular level.

The authors of the invention therefore, developed a new method, as defined in the appended claims, for defining the acceptability values of a spectroscopy (NIR or RAMAN) analysis that are suitable for the compliance validation of one or more batches of a product for the treatment of a pathological condition, wherein the product comprising or consisting of one or more natural matrices; and a process for the compliance validation (e.g. quality control in industrial production processes) of different batches of a product comprising or consisting of one or more natural matrices, that is based on the conservation of selected intrinsic characteristics of the matrices (the modulation of selected biological features), rather than on the absolute identity of their molecular components. The method of the invention allows the definition of validation acceptability values based on the analysis of selected parameters to which the maintenance of a desired overall biological activity is, as demonstrated in the present description, effectively correlated.

The present description discloses in the examples experimental data on a model product herein indicated as Arté-GX or product A (see examples for the composition of the product), which is for the treatment of osteoarthritis (disclosed in WO 2018/138678) and substantially consists of plant derived natural matrices. Faced with the problem of satisfying the regulatory requirements for bringing the aforementioned product into therapy, the inventors tested the classical validation methods and realised that said methods (based on the analysis of the quali-quantitative chemical composition) were not suitable for correctly assessing the therapeutic effect of the product (compare figures 3, 4 and 5).

The inventors therefore developed the method and process herein disclosed and found results consistent with the ones reported herein also on other different products, with therapeutical effects, comprising or consisting of one or more natural matrices.

All the products tested disclosed in the present description consist of or comprise natural matrices and are therapeutic or beneficial products. The matrices comprised therein were obtained from plant biological material suitably processed and formulated to obtain a final natural material (as defined in the glossary) which is able to modify, upon administration, a pathological state or an altered physiological state and to promote the restoration of healthy physiological conditions. For all the tested products, a reference standard with a known and verified therapeutic or beneficial effect was available. A detailed description of the tested products is provided in the examples section.

In the tested products, the hundreds of components coming from the coarse raw plant parts have not been deprived of the ability, characteristic of materials of natural, biological origin, to establish multiple combinations of molecular and supramolecular interactions among themselves and with the target tissues and therefore maintain therapeutic emerging properties.

In particular, the products tested by the Applicant have been prepared, starting from the preparation of the soil to the production of the final product, following eubiotic protocols that have been selected and standardised by Applicant throughout more than 40 years of experience to render, *a priori,* the final products as homogeneous as possible and therefore increase the efficiency in terms of yield of valid batches. The eubiotic protocol developed by the applicant, has been designed in order to standardise as much as possible each step leading to the desired final product respecting the eubiotics of each step, so to provide a final product which is 100% natural as opposed to a synthetic or partially synthetic product (i.e. a product that does not comprise a single component obtained by artificial chemical synthesis) wherein each component is produced under eubiotic conditions. It is possible to develop a fully eubiotic, standardised, production process with the integration of inter-sectoral technologies and research in several different fields, from the agricultural one to -omics and mathematical sciences ones by comparing, or rather integrating, the reductionist approach discussed above with that of systems theory and quantum biology. Preferably, according to the invention, the tested products comprising or consisting of one or more natural matrices do not comprise molecules of chemical synthesis nor comprise natural matrices that have been put in contact and that may have internalised molecules of chemical synthesis, thereby allowing to provide natural matrices and final products consisting of 100% natural ingredients.

An object of the invention is a method for the compliance validation of one or more batches of a product for the treatment of a pathological condition or for adjuvating homeostasis in an altered physiological state, said product consisting of one or more natural matrices, the method comprising
(a) performing at least one *in vitro* cell-based assay on a reference standard batch of said product having a known therapeutic effect for treatment of said pathological condition, and on one or more test batches of said product, wherein the read-out of said cell-based assay is representative of the modulation of one or more biological activity associated with one or more hallmarks of said pathological condition or of a pathological condition that can develop from said altered physiological state,
   (a1) determining for each of said one or more biological activities the modulation trend thereof for restoring a healthy physiological state and,
   (a2) performing a transcriptomic analysis determining the genes and modulation trend thereof underlying the modification detectable in said pathological condition in both diseased and healthy physiological state for each of said biological activities;
(b) quantifying in terms of numerical values the modulation of said one or more biological activities induced by each batch of step (a) for each cell-based assay read-out and defining as reference values the values calculated for said reference standard batch;
(c) defining as acceptable the test batches whose values calculated in (b) induce a modulation of each measured biological activity in said cell-based assay whose modulus is ≥ to the modulus of the reference value calculated in (b) and defining as non-acceptable the batches whose values calculated in (b) induce a modulation of at least one of said biological activities in said cell-based assay whose modulus is < to the modulus of the reference value calculated in (b);
(d) performing a NIR or RAMAN spectroscopy on said reference standard batch and on said one or more different test batches; and
(e) defining the acceptability values of the NIR or RAMAN spectroscopy spectra thereby obtained, as the variability range of the NIR or RAMAN spectroscopy spectra values of all of said acceptable batches and of said reference standard batch, preferably refined by the spectra of said non-acceptable batches;
(f) performing a NIR or RAMAN spectroscopy analysis of further batches of said product, and
(g) validating each of said further batches for which the obtained spectrum satisfies the acceptability values defined in step (e).

Depending on the pathological condition of interest, one or more hallmarks can be selected. Preferably a plurality of hallmarks is selected, although in the case of cancers, for example, the skilled person skilled person is well aware that the most relevant hallmark is the proliferation of cancer cells. Therefore, the elected cell-based assay can be in this case, an assay verifying the viability of neoplastic cells or of tumour masses upon administration of the product of interest. Still, according to the invention it is preferred to use a single cell-based assay however, more than one cell-based assay can also be used. In a non-limiting example, when for the analysis of different hallmarks different cell-based assays are deemed more suitable, additional cell-based assays can be carried out.

According to the invention the cell-based assay is designed to simulate conditions related to said pathological condition or to said altered physiological state.

The main difference between the product validation procedures of the present invention and those of the state of the art, is based on the demonstration that, although informative, the quali-quantitative characterisation of a product comprising or consisting of one or more natural matrices, due to the dynamic interactions of all its constituents that result in new emerging properties, is not suitable for assessing the real effectiveness of these kind of products and therefore the validation procedures have to be carried out on a basis that differs from that which underlies the validation procedures used for classical API based therapeutical products. Indeed, a quali-quantitative validation, in case of products comprising or consisting of natural matrices, as shown also by the data provided in the examples and in the figures, is not sufficient nor suitable to ensure the therapeutic effectiveness of the product.

As a result, the applicant has developed new methods and processes that base the validation procedures on the definition of validation parameters that are truly representative of the therapeutic effectiveness of the product.

Additional metabolomics analysis may nevertheless still be desirable for the manufacturers e.g., to evaluate the toxicological profile of the product.

Depending on the pathological condition of interest, the hallmarks can be one or more, when available, preferably more hallmarks are selected, however, e.g. in the case of cancers, the skilled person is well aware that the most relevant hallmark is the proliferation of the cancer cells, therefore the skilled person can limit the method of the invention to this single hallmark and the elected cell-based assay will be in this case, assays verifying the viability of neoplastic cells or of tumour masses upon administration of the product of interest.

The expression "value/s calculated in (b)" can be substituted in any part of the description and of the claims with "modulation value/s" or "modulation value/s calculated in (b).

When the same hallmark can be associated with biological activities that can be monitored through different biological parameters, the skilled person can decide to carry out more than one cell-based assay to monitor said activity. In the example section and in the figures the definition of the acceptability values with different parameters is provided, the results show that in each case, although different parameters for analysing the modulation of the biological activity/ies were selected, the result is consistent and, when the same spectroscopy technique is used, the resulting spectroscopy spectra variability range defining the acceptability values is the same.

The skilled person will be able to select the more suitable parameters depending on the selected hallmarks of the pathological condition of interest.

Said parameters can be, by way of example, genes and their expression patterns, ROS, oxidative stress, cell viability and others.

The method can be applied, mutatis mutandis, to beneficial products (adjuvants of homeostasis) comprising one or more natural matrices. Hence the invention also encompasses a method according to claim 1 for defining the acceptability values of a spectroscopy (NIR or RAMAN) analysis for the compliance validation of one or more batches of a product for adjuvating homeostasis in an altered physiological state, wherein the product comprises one or more natural matrices; said method comprising:
(a) performing at least one *in vitro* cell-based assay on a reference standard batch of said product, wherein the reference standard has a known beneficial effect for adjuvating homeostasis in an altered physiological condition, and on one or more test batches of said product, wherein the read-out of said cell-based assay is representative of the modulation of one or more biological activities associated with one or more hallmarks of a pathological condition that can develop from said altered physiological condition;
(b) quantifying in terms of numerical values the modulation of said one or more biological activities induced by each batch of step (a) for each cell-based assay read-out defining as reference values the values calculated for the reference standard batch;
(c) defining as acceptable the test batches whose values calculated in (b) induce a modulation of each measured biological activity in said cell-based assay whose modulus is ≥ to the modulus of the reference value calculated in (b) and defining as non-acceptable the batches whose modulation values calculated in (b) induce a modulation of at least one of said biological activities in said cell-based assay whose modulus is < to the modulus of the reference value calculated in (b);.
(d) performing a spectroscopy on said reference standard batch and on said one or more different test batches; and
(e) defining the acceptability values of the spectroscopy spectra as the variability range of the spectroscopy spectra values of all of said acceptable batches and of said reference standard batch, preferably refined by the spectra of said non-acceptable batches.

According to the invention, step a) can comprise:
a. retrieving from the state of the art a list of the hallmarks of said pathological condition;
b. identifying for each of said hallmarks a set of biological activities modifications detectable in said pathological condition and determining the modulation of each of said activities concurring to the desired therapeutic effect thereby designing the modulation pattern of each of said activities representing a healthy physiological state;
c. identifying the parameters and the modulation pattern thereof (such as genes and expression pattern thereof) underlying the modification detectable in said pathological condition for each of said biological activities and setting for each of said parameters the modulation pattern opposite to the one identified as the expression pattern indicative of said healthy physiological state;
step b) can comprise:
analysing the modulation pattern of each of the parameters (such as genes and expression thereof) identified in a) c. induced by a reference standard of the analysed product in a suitable *in vitro* cell-based assay, determining the quali-quantitative modulation of + each of said parameters induced by said reference standard with respect to the pathophysiological state control of said *in vitro* cell-based assay, and calculating the reference standard modulation value for each of said biological activities induced by said reference standard and selecting each of said reference standard modulation values as reference values (cut-offs) indicative of said desired therapeutic effect;
and analysing the modulation pattern of the parameters (such as genes and expression thereof) identified in a) c. induced by further different batches of said product, in said *in vitro* cell-based assay and determining the quali-quantitative modulation of each of said parameters induced by each of said batches thereby calculating the modulation value induced by each of said batches for each of said biological activities;
step c) can comprise:
comparing the modulation value of each of said biological activities induced by each of said batches calculated in b) with the corresponding reference values and defining as acceptable the test batches (preferably at least three) whose modulation value calculated in b) complies with the corresponding reference values (i.e. the reference values are intended as cut-offs and a batch is defined as acceptable when the reference cut-off is reached for each biological activity) and preferably at least one negative batch for which at least one modulation value calculated in b) does not comply (hence does not reach the desired cut-off) with the corresponding reference modulation value; the method also comprises steps
d) carrying out a NIR or RAMAN spectroscopy analysis of said reference standard and of the batches selected in c) and
e) defining the acceptability values of the NIR or RAMAN spectroscopy as the variability range of the spectra values resulting by the acceptable batches, preferably refined by the spectra of said non-acceptable batches. In fact, when non-acceptable batches are present, their spectra will define non-acceptable values when determining the compliance variability range. Therefore, the method provides the acceptability values of the NIR or RAMAN spectroscopy spectra.

As stated above, the reference modulation value is intended as a cut-off value (positive in case of up-modulation and negative in case of down-modulation) that must be reached for each selected biological activity by a test batch in order to be defined as acceptable.

The range of variability in each point of the spectra of the acceptable batches and of the reference standard batch can then be used as acceptability range of values of new batches of the product in compliance validation processes at the industrial level.

According to the invention the product subjected to the batch to batch validation process of the invention and for which suitable acceptability values are provided with the method of the invention is a product, comprising or consisting of one or more natural matrices, for the treatment of a pathological condition i.e. a product with a therapeutic effect verified at least preclinically *in vitro* on cells and/or tissues and/or organoids and/or *in vivo* on animal models, and therefore a product expected to, when administered to a patient suffering from a given pathological condition, reduce the severity of the subject's condition (i.e. the severity is at least partially improved or ameliorated), and/or provide some alleviation, mitigation or decrease in at least one clinical symptom of said condition and/or delay in the progression of the said condition. The subject treated, according to the invention, is an animal including humans (hence the product is for human or for veterinary use), or even a plant.

The methods and processes provided in the present invention are, indeed, processes that can be carried out with the goal of validating batches of a given therapeutic product, comprising or consisting of natural matrices, in the production chain.

The product, as already stated above, is a product that comprises or consists of one or more natural matrices, i.e. of complex natural systems; a non-limiting example of said natural matrices is represented by one or more of: cut or pulverized plant parts, plant extracts, processed plant parts, fractions of plant extracts such as, for example, the fractions obtained by filtration on a semi-permeable membrane (microfiltration, ultrafiltration, nanofiltration) or by treatment on adsorption resins, microorganisms, honey, propolis, silk, wax, plant resins, plant gums, plant exudates, vegetable oils, vegetable essential oils, animal tissues lysates, plant or animal fluids.

Preferably, said microorganisms are inactivated microorganisms such as tyndallized organisms.

In a most preferred embodiment, the therapeutic product is a product consisting of 100% of natural components, intended as components that are not obtained by man through chemical synthesis reactions, therefore, when the product comprises one or more natural matrices, it can also comprise minerals, and in general any other organic or inorganic material found in nature. Preferably, the product subject to methods and processes of the invention, is a product obtained or obtainable according to a standardised protocol, more preferably through an eubiotic standardised protocol. When no standardised protocol is available, the skilled person can nevertheless minimise the differences between batches of the same product by using, for each natural matrix comprised in the product, a pool of a starting raw sources or of intermediate materials or of natural matrices. In this way, the inherent variability of natural matrices derived from different samples of the same kind of source (e.g., same plant from different cultivars), can be diminished by said pooling.

According to the invention said product can be e.g., a, a food supplement, a medical device, or a medicament.

In an embodiment said product is a medical device as defined in EU Regulation 2017/745 Article 2 (1) indents 1-3 wherein the medical purpose is the treatment or alleviation of a disease or the modification of a pathological process or state.

Article 2 (1) indents 1-3 of EU Regulation 2017/745 recite:
For the purposes of this Regulation, the following definitions apply:
(1) 'medical device' means any [...] material or other article intended by the manufacturer to be used, alone or in combination, for human beings for one or more of the following specific medical purposes:
   - diagnosis, prevention, monitoring, prediction, prognosis, treatment, or alleviation of disease,
   - diagnosis, monitoring, treatment, alleviation of, or compensation for, an injury or disability,
   - investigation, replacement, or modification of the anatomy or of a physiological or pathological process or state,
[...] and which does not achieve its principal intended action by pharmacological, immunological, or metabolic means, in or on the human body, but which may be assisted in its function by such means. The product may also be a product classified as a medical device by the FDA.

Per Section 201(h)(1) of the Food, Drug, and Cosmetic Act, a device is:
An instrument, apparatus, implement, machine, contrivance, implant, *in vitro* reagent, or other similar or related article, including a component part, or accessory which is:
(A) recognized in the official National Formulary, or the United States Pharmacopoeia, or any supplement to them,
(B) intended for use in the diagnosis of disease or other conditions, or in the cure, mitigation, treatment, or prevention of disease, in man or other animals, or
(C) intended to affect the structure or any function of the body of man or other animals, and which does not achieve its primary intended purposes through chemical action within or on the body of man or other animals and which is not dependent upon being metabolized for the achievement of its primary intended purposes. The term "device" does not include software functions excluded pursuant to section 520(o).

The spectroscopy analysis of method of the invention is preferably carried out on a dry form of the product of interest, so when the final product intended for use is a solution, a suspension, or other liquid forms, it can be carried out on the lyophilised product prior to its rehydration.

In its final form for administration the product may be in the form of a powder, a granulate, a tablet, a syrup, a solution, a suspension, a hard or soft gelatine, a capsule, a spray, a cream, or the like.

According to the invention, a pathological condition is a specific disease or a pathological state. Non-limiting examples of pathological conditions include mild cognitive impairment (MCI), osteoporosis (OP), including post or peri menopausal osteoporosis (PMO), osteoarthritis (OA), cancer.

Without limitation thereto, cancers of major interest such as head and neck cancer, melanoma, breast cancer, bladder and osteosarcoma are included.

Hallmarks of pathological conditions as well as of specific diseases are well-known in the art. The skilled person can easily retrieve in the scientific literature the hallmarks of pathological conditions of interest as well as the biological activities underlying said hallmarks. It evident that the skilled person will select the biological activities underlying a given hallmark considering the specific pathological condition of interest.

By way of example, the skilled person can investigate the patho-physiological state-of-the-art of the disease of interest using different prior art sources.

The skilled person wishing to define hallmarks of a disease of interest will be able to retrieve the desired information from the scientific literature. By way of example, non-limiting example of sources that can be used Robbins & Cotran Pathologic Basis of Disease (Robbins Pathology) 10th Edition; https://calgaryguide.ucalgary.ca/; Biomedical literature from PubMed Central (https://pubmed.ncbi.nlm.nih.gov/) and the like.

For each of said hallmarks several biological activities underlying them are also known in the art, the skilled person can select said functions from the ones disclosed in the art. When more biological activities are ascribed in the art to a given hallmark of a given pathology in the method of the invention, preferably at least two, at least three or more of said activities are selected.

When wishing to use specific software available for easily carrying out certain steps of the method of the invention such as finding biological activities underlying a hallmark of a disease, the skilled person may wish to adapt the retrieved hallmarks adapting their definitions to better interrogate the software used. By way of example, in case the software used is Qiagen IPA (IPA version 94302991 Qiagen), the information found in the aforementioned resources can be used, if necessary, to redefine said hallmarks to interrogate IPA if the IPA uses a different internal definition of said hallmarks. To rapidly identify the biological activities underlying a hallmark of a disease, when IPA is used, the following procedure can be followed:
Hallmarks can be written, one by one, in the "disease and functions" query box and the search is then launched.

The obtained resuming table allows the skilled person to filter disease/activity that come from many lines of evidence. By way of example, the source for the relationship can be the Ingenuity Knowledge Base, including curation from journal articles, OMIM, JAX and ClinicalTrials.gov.

The tool is therefore able to associate with each biological activity a defined number of genes whose regulation can influence the modulation of the biological activity itself. Examples of known hallmarks associated with pathological conditions are shown in figures 1-4, 8-12.

Merely by way of example, hallmarks of OA known in the art comprise: Proliferation (Skeletal and muscular system development and function such as articular dysfunction and joint space); Inflammation; Anatomical damage. Still by way of example, suitable biological activities underlying the OA hallmarks above can comprise the following:
Proliferation: the biological activities of interest being e.g., skeletal, and muscular system development and function such as articular dysfunction and joint space, articular disfunction, joint space and formation of the cartilage tissue,
Inflammation: the biological activities interested being inflammatory disease, inflammation and nociception, inflammation of the joint,
Protection from anatomical damage: the biological activities interested being organismal injury and abnormalities such as joint inflammation and swelling, difficulty moving a joint: osteoarthritis.
Possible state of the art keywords defining said activities are indicated in figures 1-4.
Further, by way of example, known hallmarks of MCI may comprise Cognition (impairment), activation and viability (neuronal, decrease), Myelination and branching (decrease), Inflammation (increase), Skeletal and muscular system function (decrease).
Cognition: the biological function interested being cognition and learning;
Activation and viability: the biological activity interested being development, differentiation of neurons, proliferation of neuronal cells;
Myelination and branching: the biological activity interested being branching of neurons, neuronal sprouting;
Inflammation: the biological activity interested being chronic inflammatory disorder;
Skeletal and muscular system function: the biological activity interested being proliferation of muscle cells and necrosis of muscle.

Possible state of the art keywords defining said activities are indicated in figures 8 and 9.

Still by way of example, known hallmarks of OP, including PMO may comprise mineralisation, Inflammation (increase), functionality adipose tissue (increase), remodelling of bone, osteoporosis, differentiation of osteoblasts (decrease).
Mineralisation: the biological activity interested being mineralisation of bone cell lines and osteoblasts, formation of bone;
Inflammation: the biological activity interested being inflammation of adipose, connective and white adipose tissue;
Functionality of adipose tissue: the biological activity interested being weight gain, transdifferentiation, differentiation of adipocytes;
Remodelling of bone: the biological activity interested being remodelling and resorption of bone;
Osteoporosis: the biological activity interested being osteoporosis itself;
Differentiation of osteoblasts: the biological activity interested being activation of alkaline phosphatase, differentiation of bone cells, differentiation of osteoblasts.
Possible keywords linked to said activities are indicated in figures 10 and 11.

For each of said hallmarks biological activities underlying them are known in the art.

For each of said biological activities the modulation causally related to the pathological condition is also known in the art, therefore the opposite modulation can be considered as the modulation concurring to the desired therapeutical effect, consequently, a panel indicating the desired modulation pattern of each of said activities can be designed, said panel being representative of "the modulation trend of said activities in a healthy physiological state" i.e., said panel being representative of the way in which said activities would need to be modulated in order to restore the healthy physiological state (see figures 1-4, 8-11). Modulation in the present description is intended as up or down modulation of a given activity and of a specific parameter that is associated to the development or presence of particular condition (e.g., pathological, altered but not yet pathological, or healthy). Up modulation refers to the modification in the activity, expression, of particular genes, proteins, cellular pathways or cellular components resulting in an enhancement of a given biological activity. Downregulation is the opposite of upregulation. It involves a modification in the activity, expression of genes, proteins, cellular pathways or cellular components, leading to a reduction in a given biological activity. A modulation trend for restoring a healthy physiological state is therefore intended as the directionality (up or down) of the modulation of a selected biological activity that is observable or expected when passing from a pathological or altered condition to a healthy state. Therefore, if a given activity underlying a pathological condition is up-modulated in a pathological or altered condition, the modulation trend for restoring a healthy physiological state is down-modulation. In other words, the modulation trend for restoring a healthy physiological state of the one or more activities underlying a pathological or altered condition selected according to the method of the invention, is the opposite to the one detectable in the pathological or altered condition.

Non-limiting examples of the modulations of biological activities that are causally linked to pathological conditions (OA ... PMO) are depicted in Figures 1-4 and 8-12.

Therefore, conversely, restoring the healthy physiological state from an existing pathology of interest can be designed as requiring the inverse modulation of the one or more selected biological activities. By way of example but not limited thereto, for OA, MCI, and OP, in particular PMO, the modulation leading to the healthy physiological state is depicted in figures 1-4 and 8-12

When the modulation pattern leading to the healthy physiological state is observed upon treatment with a given product, for one or more, preferably most and ideally all, of the biological activities causally related to the hallmarks of a given disease, then the product can be identified as a product having the desired therapeutic effect. Therefore, a product inducing the modulation of the biological activities causally related to the hallmarks of a given pathological condition in the direction toward the healthy physiological state, can be identified as a product having the desired therapeutic effect on the overall pathological state. When only some of the biological activities causally related to the hallmarks of a disease are altered in the direction of the physiological state as defined above, has only a partial therapeutic effect which does not result in an entirely healthy physiological state. Nevertheless, a partial therapeutic effect is considered to fall within the scope of the invention.

When gene expression is selected as a parameter, the genes, and the expression pattern of each of said genes underlying the modification of each biological activity causally linked to each selected hallmark of a pathological condition can be retrieved by the skilled person from the state of the art, this work can be facilitated by using ad hoc bioinformatics tools. The same Qiagen IPA indicated above is suitable for a fast sorting out of said information from scientific literature, as it is an aggregator of scientific references that allows to search for information on genes/proteins and the construction of networks that predict the behaviour of biological systems according to their gene expression status.

The pathophysiological features (hallmarks) available in the state-of-the-art for a given pathological condition are used to interrogate IPA via the "IPA Bioprofiler" tool, using them as key words, additional keywords related to said hallmarks can also be added.

The use of "IPA Bioprofiler" allows the skilled person to identify the expressed genes causally linked to each of the identified biological activities and the specific molecular pathway underpinning them. Information concerning the measured gene expression data (by way of example, following the manufacturer's instructions Fold change value cut-off ≤-2 and ≥ +2 and p-value≤0.05) induced by each batch was then superimposed on the networks obtained, to define influenced genes and modulation of the connected biofunction.

Once the most relevant genes and the expression pattern thereof underlying the modification detectable in the pathological condition of interest for each of the selected biological activities are identified, for each of said genes the expression pattern opposite to the one identified is set as the expression pattern indicative of the healthy physiological state of said biological activities.

As stated above, modulation of biological activity is divided in down-modulation or up-modulation depending on the regulation of the related genes The expression pattern induced by the reference batch of the product of interest can be used to calculate the expected impact on the one or more connected biological activities. Specifically, the resulting expected calculated impact, based on the literature, on the related biofunctions can be determined by "IPA Molecule Activity Predictor" tool (MAP) and resumed in a heatmap visualization using a colour code that can easily be transformed into numeric values by the user.

*In vitro* cell-based assays are well-known laboratory techniques that involve the use of isolated cells to study biological processes or test the effects of drugs, chemicals, or other substances.

According to the invention, cell culture models such as monolayer cultures, or three-dimensional (3D) systems can be used. When appropriate, disease-specific cell lines can be used, depending on the disease of interest (e.g., cancer) also proliferation assays can be used.

A suitable *in vitro* cell-based assay is an assay designed to mimic a disease e.g., due to the nature of the cells used, or by inducing a diseased phenotype in cells treated with specific compounds i.e., a "disease model assay" or "disease-in-a-dish" model.

Cell Type Selection: a cell line or primary cells relevant to the disease being modelled is selected. By way of example, if studying OA, a suitable and recognised cell-based assay is with chondrocytes that, upon treatment with IL1B, are a disease model of OA acknowledged in the art. Still by way of example, if studying neurodegenerative diseases, neuronal cell lines like SH-SY5Y, primary neurons may be selected or primary cells in which the desired phenotype can be induced by an "insult" with a given compound can be used.

For the study of Osteoporosis, in particular PMO, a suitable cell-based assay can be prepared by using Human, adipocyte-derived, mesenchymal stem cell lines (hADMSC), capable of differentiating into osteoblasts and mineralize the extracellular matrix (ECM) as described in the examples.

For the study of cancers, viability tests can be carried out on suitable (depending on the cancer of interest) cell-based assays.

In case, e.g., ROS scavenging activity is a selected biological activity, a suitable cell-based assay can be carried out with a human fibroblast (HuDe) cell line insulted with a generator of ROS (e.g., AAPH 2,2'-azobis-2-methyl-propanimidamide, dihydrochloride) as known in the art.

For cell-based assays wherein the pathological state must be induced by an insult, the assay cells can also be directly tested with the product of interest in order to verify that the modulation of the selected biological activities has the same trend of the modulation expected to lead to a healthy physiological state. This is particularly useful for assessing the beneficial (adjuvating homeostasis) effect of a product.

The cell-based assays preferably include appropriate control groups, such as untreated cells, vehicle-treated cells, and cells treated with compounds known to have no impact on the disease phenotype. These controls help distinguish the specific effects of the tested compounds.

In those embodiments in which the expression of one or more genes, e.g., a gene expression profile, is used as a parameter of one or more biological activities connected to a pathological condition of interest, a transcriptomics analysis on a suitable *in vitro* cell-based assay mimicking the pathological condition of interest can be carried out, and the genes, and the genes and the expression pattern thereof underlying the modification detectable in the pathological condition for each of the selected biological activities can be identified. In the case in which the disease phenotype is caused by the administration of a specific agent to the cultured cells, the modification representing the pathological condition is the modification insulted cells vs. pre-insulted, untreated cells and the modification induced by the reference standard is the modification insulted cells vs. insulted cells + reference standard.

The transcriptomic analysis can be carried out with any suitable technique known in the art, including next generation sequencing and gene expression microarrays and the transcriptomic expression profile in the basal cells as opposed to the cells treated to mimic the pathological condition can be evaluated in order to identify the significantly differentially expressed genes and their expression patterns. According to the method of the invention, once the expression pattern of the significantly differentially expressed genes of the cells representing the disease phenotype vs. the cells before the insult inducing said diseased phenotype is assessed, the opposite expression pattern is considered representative of the restoration of a healthy physiological state (herein also abbreviated as "representative of the healthy physiological state).

When gene expression is the selected parameter, for each biological activity in a) the genes and the expression pattern thereof underlying the modification detectable in said pathological condition for each of said biological activities can be identified from the state of the art using appropriate tools.

By way of example, the skilled person can derive this information using any suitable approach including using software specifically designed for this scope such as Ingenuity Pathway Analysis (IPA version 94302991 Qiagen).

The interpretation of high-throughput gene-expression data is greatly facilitated by the consideration of prior biological knowledge. This can be done using statistical gene-set-enrichment methods where differentially expressed genes are intersected with sets of genes that are associated with a particular biological activity or pathway (Abatangelo, L. et al. (2009) Comparative study of gene set enrichment methods. BMC Bioinform., 10, 275). One more recent approach involves the application of causal networks that integrate previously observed cause-effect relationships reported in the literature (Chindelevitch, L. et al. (2012a) Causal reasoning on biological networks: interpreting transcriptional changes. Bioinformatics, 28, 1114-1121; Felciano, R.M. et al. (2013) Predictive systems biology approach to broad-spectrum, host-directed drug target discovery in infectious diseases. Pac. Symp. Biocomput., 2013, 17-28; Kumar, R. et al. (2010) Causal reasoning identifies mechanisms of sensitivity for a novel AKT kinase inhibitor, GSK690693. BMC Genom., 11, 419; Martin, F. et al. (2012) Assessment of network perturbation amplitudes by applying high-throughput data to causal networks. BMC Syst. Biol., 6, 54; Pollard, J. Jr. et al. (2005) A computational model to define the molecular causes of type 2 diabetes mellitus. Diabetes Technol. Ther., 7, 323-336). While still depending on statistics, this is more powerful than gene-set enrichment since it leverages knowledge about the direction of effects rather than mere associations.

In a preferred embodiment, the skilled person can follow the protocol provided in the publication by Kramer et al, Bioinformatics vol 30 no 4 2014, pages 523-530 "Causal analysis approaches in Ingenuity Pathway Analysis provides and discuss a suite of algorithms and tools for inferring and scoring regulator networks upstream of gene expression data based on a large-scale causal network derived from the Ingenuity Knowledge Base" or the manufacturer's instructions of (IPA version 94302991 Qiagen). The method and algorithms disclosed in the paper enable the skilled person to predict downstream effects on biological activities and diseases.

In the article, the authors describe causal analysis approaches that have been implemented in Ingenuity Pathway Analysis (IPA) with particular focus on the details of the underlying algorithms, and the application to several real-world use cases. In particular, points a) b. and a) c. can be readily carried out by the skilled person by using Ingenuity Pathway Analysis (IPA version 94302991 Qiagen) which is well-known pathway analysis application among the life science research community cited in tens of thousands of articles that allows to understand causal connections between and among diseases, genes and networks of upstream regulators.

Step b) of the method of the invention comprises:
quantifying in terms of numerical values the modulation of said one or more biological activities induced by each batch of step a) for each cell-based assay read-out defining as reference values the values calculated for the reference standard batch;
more in detail the step can comprise analysing the modulation of each of the one of more biological activities and/or parameters thereof induced by a reference standard of said product in the *in vitro* cell-based assay performed in step a) and determining the quali-quantitative modulation of each of one of more biological activities and/or parameters thereof induced by said reference standard and calculating the modulation value for each of said biological activities induced by said reference standard thereby providing the reference standard modulation values for each of said biological activities indicative of said desired therapeutic effect (cut-off values).

From the results obtained with the cell based assay it is possible to determine the quali-quantitative modulation of each of the activities and/or parameters previously identified, induced by each product batch sample with respect to the control group and it is therefore possible to obtain values representing the qualitative (which activity is modulated and in which direction, i.e. up modulation positive value or down-modulation negative value) and quantitative (how much is each activity modulated with respect to the control group) modulation induced by each batch on each of said biological activity; the magnitude of the value representing the distance (in terms of %, folds etc.) with respect to the control group. The modulation of each activity can be expressed as a numerical value, such a statistical numerical value.

Depending on the parameters selected, the modulation of a biological activity can be defined and characterized by the skilled person by commonly used various quantitative values depending on the specific context and the type of biological activity being studied. Some common values used to define modulation include: fold change i.e. the ratio of the value of the biological activity under a particular condition or treatment to its value under a control or reference condition; by way of example, a fold change of 1 indicates no change, while values greater than 1 indicate upregulation and values less than 1 indicate downregulation; log fold change i.e. the logarithm (usually base 2 or base 10) of the fold change; percentage change, i.e. the percentage difference between the value of the biological activity under a particular condition and its value under a control or reference condition; z-score, which z-score represents the deviation of the observed value of the biological activity from its mean value, normalized by the standard deviation (a positive z-score indicates an increase in activity, while a negative z-score indicates a decrease); effect size, i.e. a measure of the magnitude of the difference between two groups, often standardized to facilitate comparisons across studies or datasets (Cohen's d is a common effect size measure, calculated as the difference in means divided by the standard deviation); area under the curve (AUC), for dynamic biological activities, such as signalling pathways or physiological responses, the AUC can be used to quantify the overall activity or response over a period of time.

These values can be used individually or in combination to provide a comprehensive characterization of the modulation of a biological activity under different conditions or treatments. The choice of which value(s) the skilled person decides to use depends on the specific research question, the nature of the biological activity, and the available data.

In the context of biological activities, a z-score can be used to express the modulation or alteration of a specific biological activity relative to its typical or baseline behaviour. This is often employed in fields like systems biology, where researchers analyse high-dimensional datasets to understand complex biological processes.

In this case, the z-score represents how much a particular biological activity deviates from its expected or average behaviour within a given context, often in response to some stimulus, treatment, or condition.

For example, in gene expression analysis, a z-score might be calculated to assess how much the expression level of a gene changes in response to a therapeutic treatment compared to its expression in control conditions. A positive z-score indicates upregulation, while a negative z-score indicates downregulation.

Mathematically, a suitable formula for calculating the z-score of a biological activity modulation might involve comparing its observed value under a specific condition to the mean and standard deviation of its values across multiple conditions or 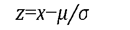 replicates:

Where x represents the observed value of the biological activity (e.g., gene expression level), µ is the mean value of the activity across all conditions, and σ is the standard deviation of the activity across all conditions.

This z-score approach allows researchers to identify and prioritize biological activities that are significantly altered under certain experimental conditions, providing insights into the underlying mechanisms of biological systems.

Once the desired modulation of the biological activity is defined (i.e. the one representing the healthy physiological state) and optionally the desired regulation of the related parameter (e.g. ROS scavenging activity, genes, etc.) is identified for the pathological condition of interest, an analysis of the modulation of the biological activity induced by the reference standard of the therapeutic product (optionally the modulation of one or more parameters thereof, e.g., gene expression as determined by transcriptomics analysis) is carried out on the *in vitro* cell-based assay that is representative of the pathological condition. As already clarified previously, the reference standard has a previously assessed therapeutic or beneficial effect. The quali-quantitative modulation of the selected biological activity/ies and/or parameter/s thereof (e.g. of the expression of the genes of interest) induced by the reference standard is determined and the modulation values for each of the selected biological activities are calculated and subsequently used as reference modulation values or "cut-off values" for each of said biological activities indicative of said desired therapeutic effect.

The reference standard of the product tested is, as stated above, a batch of the product of interest for which a therapeutic or beneficial effect has been assessed either *in vitro* and/or *in vivo,* preclinically, or clinically. In any case it is a batch of the product of interest for which the therapeutic or beneficial effectiveness has been previously verified and is hence defined as a batch with a known therapeutic or beneficial effect.

According to an embodiment, the reference standard modulation values for each biological activity are taken as reference modulation values that are representative of the desired therapeutic or beneficial effect. The reference modulation values can be adjusted, as explained below, when the cell-based assay is carried out also on one or more reference drugs (i.e. drugs that are known in the art as indicate for the treatment of the pathological condition of interest). Said drugs are expected to modulate with the desired trend at least some of the selected biological activities although, due to their different mechanism of action (SAR), said drugs are not expected to modulate with the desired trend all of said selected biological activities. Therefore, as explained in more detail below, the adjustment of the reference modulation values considering also the drug modulation values will be carried out only for said biological activities that are modulated by the tested drug in the with the same trend of the healthy physiological state.

The modulation values in step b) are calculated to represent the directionality and magnitude of the modulation for each biological activity exerted by a given batch of product. For transcriptomics, the Core analysis of the IPA version 94302991 Qiagen can be used by the skilled person to readily obtain the aforementioned modulation values in terms of Z-score values following the manufacturer's instructions.

In case the Core analysis does not yield sufficient relevant information, alternative approaches can be employed. An example of an alternative approach is provided below (overlay analysis). Step b) of the method of the invention comprises quantifying in terms of numerical values the modulation pattern of the parameters identified in a) c. induced by further different batches of said product in said *in vitro* cell-based assay by determining the quali-quantitative modulation of each of said parameters induced by each of said batches thereby calculating the Z-score modulation value for each of said biological activities induced by each of said batches.

Preferably at least three batches that are acceptable and one batch that is not acceptable with are desired, and so the skilled person must analyse at least four additional batches of the product of interest.

As stated above, the batches of that are considered acceptable are batches for which the modulation value for each biological activity is ≥ the reference modulation value when the desired modulation of the biological activity is an up modulation and ≤ the reference modulation value when the desired modulation of the biological activity is a down modulation. Conversely, when the modulation value for at least one biological activity does not fulfil the requirements above, the batch is considered non-compliant (not acceptable) and can be used as a reference of not acceptability for the final assessment of the acceptability values of spectroscopy.

According to a preferred embodiment of the invention, additional different batches of the product of interest can be analysed. In particular, when a batch X of the product of interest, that has not been analysed with the cell-based assay described herein, does not fall within the spectroscopy acceptability values in the process of batch-to-batch validation of the invention, steps a) to c) of the method for determining the acceptability values of the spectroscopy analysis can be performed on said batch. In case the batch complies with the reference modulation values (i.e. the batch exerts the desired modulation of the biological activities which are indicative of the desired therapeutic effect), batch X is considered acceptable and the acceptability values of the spectroscopy to use in the process of the invention can be adjusted accordingly. Additionally, if desired, steps a) and b) can be performed also with one or more reference drug (i.e., a drug conventionally administered for the treatment of the pathological condition of interest). For each biological activity modified also by said one or more drug in the direction of the healthy physiological state, the reference modulation value can be adjusted by comparing the drug modulation value and the reference standard modulation value, and selecting as reference modulation value the one associated to the weakest performance.

As conventional drugs are expected to modulate in the desired direction only some of the selected biological activities due to their mechanism of action (SAR), only the modulation values related to the activities that are significantly modulated with the same directionality of the healthy physiological state will be considered.

As known by the skilled person, reference drugs for the treatment of a pathological condition may be known a priori to provide a therapeutic effect only on one or few hallmarks of the disease. In this case, the information that will be integrated in the method of the invention will relate only to the interested hallmarks and related biological activities.

As stated above, the method of the invention may comprise determining one or more parameter and the modulation trend thereof underlying the modification detectable in said pathological condition for each of said biological activities in both diseased and healthy physiological state. In a preferred embodiment said parameters and are genes. Preferably, gene modulation is assessed by transcriptomic analysis.

In a not limiting embodiment the method of the invention can be carried with the aid of IPA version 94302991 Qiagen out as follows:
Definition of pathophysiological state of the art of the disease and of the pathophysiological hallmarks of the disease with which to interrogate IPA

The pathophysiological state-of-the-art of the disease of interest is investigated using different specific sources:
- Robbins & Cotran Pathologic Basis of Disease (Robbins Pathology) 10th Edition
- https://calgaryguide.ucalgary.ca/
- Biomedical literature from PubMed Central (https://pubmed.ncbi.nlm.nih.gov/) The information found in the aforementioned resources are used to identify hallmarks to interrogate IPA by the following procedure:
   - Hallmarks are written, one by one, in the "disease and functions" query box and the search is then launched.
   - The obtained resuming table allows you to filter disease/activity that come from many lines of evidence. The source for the relationship is the Ingenuity Knowledge Base, including curation from journal articles, OMIM, JAX and ClinicalTrials.gov.
   - In silico model will be limited to genes and mRNAs.

The tool can associate with each biological activity a defined number of genes whose modulation is able to influence the modulation of the biological activity itself.

The following applies to any step of the method of the invention in any embodiment herein described, wherein an *in vitro* cell-based assay is carried out treating the cells with the disease phenotype with a given compound, be it the reference standard, a different batch of the product of interest, a reference drug, or the like.

A whole transcriptome raw data analysis

Whole transcriptome expression profile is evaluated in the *in vitro* cellular model representing the disease for control cells and for model cells. A Human Clariom^{™} S Pico Assay HT (Applied Biosystems, ThermoFisher Scientific) on a GeneTitan MC Instrument (Applied Biosystems, ThermoFisher Scientific), can be used following the manufacturer's instructions. CEL Intensity files can be generated by Affymetrix GeneChip Command Console Software (AGCC, ThermoFisher Scientific). Data analyses can be performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package). This phase allows the user to obtain a list of differentially expressed genes (DEGs), identified based on their expression fold changes with respect to a relevant control experimental condition, in this case one that reproduces the pathological state *in vitro.*

The transcriptional modifications profile thus obtained is subjected to a functional pathway enrichment analysis. One of the commercial tools that can be used is Ingenuity Pathway Analysis (IPA version 94302991, Qiagen) [Krämer et al. (2014)]. The use of IPA allows the user to estimate how and to what extent the modulation of gene expression in the cellular system (cell-based assay) influences the biological activities related to the pathology of interest.

IPA pre-analysis filtering (contextual data analysis) of transcriptional profile

In preparation for subsequent analyses, transcriptomic profiles undergo a filtration process to identify relevant genes and their corresponding measurement values. This filtration aims to select only significantly perturbed genes, as indicated by their fold change compared to the pathological condition. Typically (e.g., as according to the manufacturer's instructions), the fold change threshold is set to encompass values ≤ -2 and ≥ +2, accompanied by a statistical significance denoted by a p-value of ≤ 0.05. However, the skilled operator has the flexibility to adjust the cut-off based on their expertise, considering the successful performance of negative controls (a sample representing the pathological state) or reference standard known to be able to counteract fully or partially the pathological state.

The possible methodological approaches to extract biological meaningfulness from a list of modifications of gene expression profiles through IPA can be of two types: "Core analysis" and "Overlay analysis of in silico model of patho-physiological state" (abbreviated as overlay analysis).

At this stage of the procedure, hence, two different, alternative, options can be pursued.

### Core Analysis

The list of Differentially expressed genes (DEGs) after administration of the reference standard or of any other product batch/reference drug or other substance, and corresponding data measurement values (fold change with respect to pathological state) identified in the different experimental conditions are uploaded into the application. Available identifiers are mapped to their corresponding entity in QIAGEN's Knowledge Base.

By launching a "Core Analysis", significantly perturbed DEGs, called Network Eligible molecules, are overlaid onto a global molecular network developed from information contained in the QIAGEN Knowledge Base. Networks of Network Eligible Molecules are then algorithmically generated based on their connectivity.

The core analysis provides a comprehensive list of approximately top 500 biological activities derived from the generated networks. The program's association "Biological functions-genes" is always supported by annotations corresponding to scientific peer review publications that substantiate, through the automatic association of a Z-score [Krämer et al. (2014)], the calculated directionality and magnitude of modulation of the biological activities. In essence, this value represents a statistical metric that assesses the similarity between the observed pattern of Differentially Expressed Genes (DEGs) and the expected pattern based on existing literature for a given annotation.

It is the responsibility of the proficient operator to carefully choose the biological activities that are relevant to the specific pathology under investigation. The selection of biological activities will be structured based on the identified hallmarks of the pathology of interest.

The associated Z-score values will then be used to indicate the directionality and magnitude of modulation for each biological activity.

### Overlay Analysis

If the core analysis does not yield sufficient relevant information, an alternative approach called "Overlay analysis" can be employed. This analysis focuses on the biological activities identified by the "in silico model of the patho-physiological state". The selection of biological activities is structured based on the identified hallmarks of the pathology of interest.

The "Overlay analysis" is structured by establishing a relationship between patterns of differentially expressed genes and selected biological activities (always supported by annotations corresponding to scientific peer review publications that substantiate the directionality and magnitude of modulation of the biological activities) using the following procedure:
□ Import the set of biological activities selected from the in-silico model of the patho-physiological state into a new sheet called "my pathway."
□ Utilize the "Build tool" and "Grow tool" to identify Differentially Expressed Genes (DEGs) belonging to the transcriptomic profile under investigation and are linked to regulation of the biological activities selected in the previous step.
□ Modulation of the identified DEGs is represented using green colour (indicating down-modulation) and red colour (indicating up-modulation).
□ To determine the expected calculated impact of such experimentally observed modulations of gene expression on the biological activities, the "Overlay" and "Molecule Activity Predictor" tool (MAP) are employed. The "Prediction" function is activated within MAP tool to calculate the resulting expected modulation of biological activities. Colour coding is thus established:
   - orange: increase in activity
   - blue: decrease in activity
   - white: not achievable/not predictable

As the "Overlay analysis" does not directly calculate the Z-score for each biological activity but provides results in terms of colours indicating the direction of the modification and intensity of the colour signal proportional to the magnitude of the modulation of interest, it is necessary to translate the intensity of the modulation signal (graphically represented in the "my pathway" tab) into a numerical value. This is achieved by converting the colour intensity obtained for each biological activity into a Z-score.

One of the possible tools that can be used for this purpose is "IPAmap_Parser" app, a web port app of Pipeline Pilot that aims to assign a score, called z-score, to genes and biofunctions based on their coloration within a biological pathway generated by QIAGEN's Ingenuity Pathway Analysis software. The key step of the algorithm is the conversion from the RGB colour model to the LAB model (https://www.xrite.com/it-it/blog/lab-color-space posted on October 2018 by Tim Mouw), a colorimetric coding that also allows the intensity of the colour to be recorded and not just the RGB composition. This conversion occurs within a Pipeline Pilot "component" that uses a procedure written using R software that is based on specific features of the colorspace package (https://cran.r-project.org/web/packages/colorspace/index.html, details can be found on Zeileis et al 2020journal of statistical software, doi:10.18637/jss.v096.i01).

The Z-score allows for an objective comparison of the impacts of different treatments.

When the above protocol is carried out with the reference standard, and optionally with one or more reference drugs, the modulation (Z-score) values obtained for each biological activity as disclosed above are considered the reference cut-off Z-score values for the desired modulation of said activity, therefore, when the desired modulation is an up modulation the cut-off Z-score value will correspond to ≥ the reference Z-score value obtained according to the present description , when the desired modulation is a down modulation the cut-off Z-score value will correspond to ≤ the reference Z-score value obtained according to the present description.

In case one or more reference drugs is also tested in the selected *in vitro* cell-based assay, the modulation Z-score values observed for the biological activities that are known to be modulated by said reference drugs, can be considered in order to establish the compliance criteria (acceptable or not acceptable batch).

A reference drug is a drug recommended by the clinical guidelines for treating the pathology or the hallmark of interest.

When a reference drug tested *in vitro* in a) modulates one or more biological activity associated with hallmarks of the pathological condition of interest in the desired direction (healthy physiological state), both the Z-score obtained with the reference standard and with the reference drug are considered effective for said one or more biological activities, therefore, the value associated to the weakest performance is assumed to identify the lower limit of compliance for each of said one or more biological activities and will be considered the reference (cut-off) Z-score to refer to for defining acceptable or not acceptable the test batches of the product.

Step d) of the method comprises:
(d) performing a is Near InfraRed Spectroscopy (NIR) or RAMAN spectroscopy on said reference standard batch and on said one or more different test batches; A nonlimiting example of spectroscopy analysis not part of the invention Fourier Transformed InfraRed (FTIR), Spectrophotometry, such as, e.g. UV-VIS (UV-Visible), fluorescence spectroscopy, light scattering. The skilled person, depending on the formulation of the product analysed (e.g., dry powder, liquid, etc.) will readily select the spectroscopy or spectrophotometry technique more suitable for the analysis of the selected product.

According to a preferred embodiment of the invention said spectroscopy analysis is carried out by NIR. Indeed, as the skilled person knows, NIR spectroscopy is a vibrational spectroscopy technique that provides qualitative information about the chemical species present in the material analysed together with information about its physical state. Performing NIR analysis means subjecting the material to light of different wavelengths and measuring the vibration of the material at each wavelength. The vibrations detected depend on the composition and on the interactions between the components, which affect the vibrational capabilities of each component placed within the matrix. This analysis generates a vibrational footprint specific to that material.

The analysis of the NIR Fingerprint allows the reconstruction of a chemical-physical profile characteristic of the molecular composition of each analysed sample that is influenced by the chemical surroundings. Indeed, the molecules within the sample can form bonds between them, in particular hydrogen bonds, both intermolecular and intramolecular. This leads to an alteration of the vibration frequencies of both stretching and bending of the hydrogen atom and result in a shift in vibrational frequencies with respect to the single isolated molecule. For this reason, NIR spectrometry can be an excellent means of characterizing complex matrices. This technique is therefore particularly suitable for the analysis of natural matrices as it provides a fingerprint of the whole matrix network and of the interactions between the components of the matrix.

An example of the NIR spectral profiles is shown Figure 6.

Step e) of the method comprises:
defining the acceptability values of the NIR or RAMAN spectroscopy spectra as the variability range of the NIR or RAMAN spectroscopy spectra values of all of said acceptable batches and of said reference standard batch, preferably refined by the spectra of said non-acceptable batches.

The variability range obtained will depend on the spectroscopy technique used and on the product tested.

Independently of the spectroscopy techniques used, the common feature is that each of the "acceptable" batches and the reference standard are set *a priori* as valid and acceptable products due to their assessed effect on the selected biological activities.

When NIR is used the NIR acceptability values obtained with the method of the invention are suitable for use in NIR conformity tests for batch-to-batch compliance (i.e., validation of the batch for production).

It is evident that the conformity tests must be carried out with the same spectroscopy or spectrophotometry technique used for the assessment of the acceptability values.

In order to define the acceptability values, the complete NIR spectrum of all the acceptable batches samples and of the reference standard is acquired, the spectra are aligned and normalised (Standard Normal Variate) and the wavelength (λ) regions of interest of the spectra are hence defined and the average spectrum of the spectra of all the compliant (positive) samples + reference standard is generated.

The average spectrum obtained will be the reference spectrum over the whole procedure.

In the execution of a NIR conformity test for pharmaceutical API-based products, pre-processing comprises (SNV) as indicated above, definition of the λ regions of interest, and the Max Conformity Index Value is normally used in the conformity tests and is conventionally imposed as 3.5 which means that the highest value of standard deviations accepted for pharmaceutical products in any point of the spectrum is normally 3.5.

In the method disclosed herein the acceptability values are not based on the quali-quantitative analysis of specific chemical substances in said product but on the modulation activity exerted by said product on selected biological activities, therefore, the Max Conformity Index (MCI) Value conventionally imposed in the quality controls for classical APIs, which is related to the quali-quantitative chemical composition, cannot be considered as *a priori* acceptable and the value is hence assigned based on the spectra of the batches selected in iv) and the reference standard.

In addition, Sum 2 (which derives from CI but also considers NIR spectra of one or more undesired samples), which is more suitable for heterogeneous samples, is preferably used as an acceptability parameter according to the present invention.

According to this embodiment of the invention, the CI (Conformity Index) limit to which the conformity test will be pre-processed, corresponds to the maximum value of CI MAX (also defined herein as CI limit) defined by the spectra of the batches selected in iv) and the reference standard.

Therefore, according to the present invention the acceptability values of the spectroscopy analysis are defined based on the modulation activity on each selected biological activity exerted by the reference standard and batches of the product that are compliant with the reference standard in said modulation.

In the case of a NIR spectroscopy, hence, the CI limit i.e., the acceptability cut-off is calculated according to the following formula:

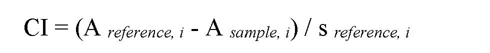

Where
A *_{reference, i}* = average absorbance at a given wavelength(*i*) of the reference (average spectrum)
A *_{sample, i}* = absorbance at a given wavelength(*i*) of the test sample
s *_{reference, i}*= standard deviation at a given wavelength(*i*) of the reference (average spectrum)

In conformity tests related to heterogeneous samples, as indicated above, the Sum2 parameter is more suitable.

Sum2= (Sum of All CIs > CI limit - CI limit)/(Sum of the number of points in the spectrum with CI > CI limit)

Selecting the appropriate parameter in a conformity test depends on the user-specific control problem that can be easily addressed by the skilled person, in the case of products comprising or consisting of one or more natural matrices, i.e., extremely heterogeneous samples, Sum 2 is a suitable parameter.

In one embodiment of the invention, Sum2 parameter is selected for determining the acceptability cut-off of the conformity test.

Depending on the spectroscopy used, the acceptability values are calculated mutatis mutandis, with the same ratio used for NIR spectroscopy, i.e., by defining as acceptable the spectra obtained from the acceptable batches according to the invention and the reference standard.

When carrying out the process for the compliance validation process of one or more batches of a product (conformity test) comprising one or more natural matrices for the treatment of a pathological condition, according to the present invention, a batch may result, in a first analysis, "not compliant" as its spectroscopy spectrum does not fall within the acceptability values defined with the method of the invention.

In that case, it may be of interest to verify whether the non-compliance result is caused by an effective non-compliance of the batch with the desired modulation of the biological activities (i.e., the therapeutic effect) or is due to the fact that the acceptability parameters can be broadened, in that case, an adjustment of said parameters can be made.

According to the method of the invention, steps a) to c) can be repeated for said batch and, if the batch results as acceptable, the acceptability values of the spectroscopy can be recalculated also including the spectrum of the new batch.

The higher the number of acceptable batches, the more accurate the spectroscopy acceptability values obtained with the method of the invention will be.

Furthermore, the method of the invention may also further comprise:
in step d) performing a NIR or RAMAN spectroscopy on one or more *a priori* undesired batches of said product, and verifying that the spectra of said one or more undesired batches does not fall within the acceptability values defined in e), and, in the negative (i.e., when the batch results within the acceptability values defined in e))
e') defining new, narrower, acceptability values of the spectroscopy spectra resulting by considering the variability range of the spectroscopy spectra values of all of said acceptable batches and of said reference standard batch and of all said undesired batches as not acceptable.

The additional features above can be desired by the product manufacturer to make sure that batches that may result from a formulative error expectable from the production chain, such as the absence of an ingredient or the like are excluded *a priori* from the acceptability values of the spectroscopy spectra in the compliance batch-to-batch control. According to an example not part of the invention spectrophotometry may be used instead of spectroscopy.

The specification also describes a method for determining a reference standard of a therapeutic product for the treatment of a pathological condition, wherein the product comprises one or more natural matrices; said method comprising:
(1) performing at least one *in vitro* cell-based assay designed to simulate conditions related to said pathological condition on several batches of said product, and on one or more reference drug for the treatment of said pathological condition, wherein the read-out of said cell-based assay is representative of the modulation of one or more biological activity associated with one or more hallmarks of said pathological condition and determining for each of said one or more biological activities the modulation trend thereof for restoring a healthy state;
(2) quantifying in terms of numerical values the modulation of said one or more biological activities induced by each batch and drug of step (1) for each cell-based assay read-out, and defining as reference value for each biological activity modified by said one or more reference drug according to said modulation trend for restoring a healthy state, the value associated to the weakest performance;
(3) selecting the batches whose values calculated in (1) induce a modulation of each measured biological activity in said cell-based assay whose modulus is ≥ to the modulus of the reference value calculated in (2) and defining as product reference standard batch the one with the highest modulus values on the larger number of biological activities.

Steps (1) and (2) are carried out, mutatis mutandis, as steps a) and b) as described above and in the examples.

The parameters and modulation patterns thereof as described above for the method for defining the acceptability values of a spectroscopy or spectrophotometry analysis for the compliance validation of one or more batches of a product apply mutatis mutandis to the method for defining a reference standard. Hence, in an example, said parameters are genes and the modulation thereof is their expression pattern.

Finally, the specification describes a process for the compliance validation (i.e., production quality control) of one or more batches of a product for the treatment of a pathological condition or of a beneficial product, said product comprising one or more natural matrices, said method comprising the following steps:
a. performing a spectroscopy or spectrophotometry analysis of each batch,
b. validating each batch for which the obtained spectrum satisfies the acceptability values defined according to the method of the invention. Given the fact that the method of the invention provides reliable parameters for use in the batch to batch validation for the compliance of products comprising or consisting of one or more natural matrices, the skilled person will readily understand that the method of the invention can also be applied, *mutatis mutandis*, for defining the acceptability values of a spectroscopy or spectrophotometry analysis for the compliance validation of one or more batches of a product adjuvating the homeostasis of a subject's system, organ or apparatus comprising or consisting of one or more natural matrices; comprising a step in which said acceptability values are calculated on a spectroscopy or spectrophotometry spectra of a reference standard of said product, said reference standard having an ascertained homeostatic adjuvant effect in the maintenance of a healthy physiological state of said system organ or apparatus, and one or more different batches of said product; wherein said spectra are defined as acceptable on the basis of the biological activity exerted in a cellular-based assay by said reference standard and said one or more different batches of said product on one or more biological activities underlying said healthy physiological state.

The method as defined in claim 1 can be described also as follows:
A method for defining the acceptability ranges or cut-offs of a spectroscopy analysis for the validation of one or more batches of a product, for use in the treatment of a pathological condition, comprising or consisting of one or more natural matrices; comprising calculating said acceptability ranges or cut-offs from the spectroscopy spectra of a gold standard (reference standard) of said product, said gold standard having an ascertained therapeutic effect in the treatment of said pathological condition, and of one or more different batches of said product; wherein said spectra are defined as acceptable or not-acceptable on the basis of the biological activity exerted in at least one cell-based assay by said gold standard (reference standard) and said one or more different batches on one or more hallmarks of said pathological condition.

The method as defined above can comprise the following steps:
i)
   a. retrieving from the state of the art a list of the hallmarks of said pathological condition;
   b. identifying for each of said hallmarks a set of biological activities modifications detectable in said pathological condition and determining the modulation of each of said activities concurring to the desired therapeutic effect thereby designing the modulation pattern of each of said activities representing a healthy physiological state;
   c. identifying the parameters and the modulation pattern thereof underlying the modification detectable in said pathological condition for each of said biological activities and setting for each of said parameters the modulation pattern opposite to the one identified as the modulation pattern indicative of said healthy physiological state;
ii) analysing the expression pattern of each of the parameters identified in i) c. induced by a gold standard (reference standard) of said product in a suitable *in vitro* cell-based assay, determining the quali-quantitative modulation of each of said parameters induced by said gold standard (reference standard) with respect to the pathophysiological state control of said *in vitro* cell based assay, and calculating the gold standard (reference standard) Z-score value (i.e. a modulation value) of the modulation of each of said biological activities induced by said gold standard and selecting each of said gold standard (reference standard) Z-score values as reference cut-off Z-score values indicative of said desired therapeutic effect,
iii) analysing the modulation pattern of the parameters identified in i) c. induced by further different batches of said product in said *in vitro* cell-based assay and determining the quali-quantitative modulation of each of said parameters induced by each of said batches thereby calculating the Z-score value of the modulation of each of said biological activities induced by each of said batches
iv) comparing the Z-score value of the modulation of each of said biological activities induced by each of said batches calculated in iii) with the corresponding reference cut-off Z-score values and selecting at least three positive batches for which each Z-score value calculated in iii) complies with the corresponding reference cut-off Z-score values and at least one negative batch for which at least one Z-score value calculated in iii) does not comply with the corresponding reference cut-off Z-score value
v) carrying out a spectroscopy analysis of said gold standard and of the batches selected in iv)
vi) defining the variability spectroscopy ranges resulting by considering each of the results obtained in v) as acceptable for each positive batch and non-acceptable for each negative batch thereby providing said acceptability spectroscopy ranges or cut-offs.

The method may further comprise in step ii) analysing the modulation pattern of the parameters identified in i) c. induced by one or more reference drug for the treatment of said pathological condition in said *in vitro* cell-based assay and determining the quali-quantitative modulation of each of said parameters induced by each drug and calculating the drug Z-score value (modulation value) of the modulation of each of said biological activities induced by each of said drug and, for each biological function modified also by said one or more drug in the direction of the healthy physiological state, comparing the drug Z-score value and the gold standard (reference standard) Z-score value, and selecting as reference cut-off-Z score value, the Z-score value associated to the weakest performance.

In an embodiment the parameters and modulation pattern thereof correspond to the genes and the expression pattern thereof underlying the modification detectable in said pathological condition for each of said biological activities.

By way of example, the product analysed with the method of the invention can be in dry or lyophilised form.

In addition, in the method as described in the paragraphs above, step iii) can be carried out on one or more additional different batch of said product and the one or more additional batch for which the Z-score (modulation value) value of the modulation of each of said biological activities complies with said corresponding cut-off Z-score values is subjected to the same spectroscopy analysis carried out in v) and the acceptability ranges or cut-offs defined in vi) are recalculated by defining also each of said batches as acceptable.

Further, the method may comprise the steps of:
vii)
   carrying out a spectroscopy analysis of one or more a priori undesired batch of said product and verifying that said one or more undesired batch does not result within the acceptability ranges or cut-offs defined in vi), and, in the negative
viii)
   defining new, narrower, variability spectroscopy ranges or cut-offs resulting by considering each the results obtained in vii) as non-acceptable.

In any part of the description and of the claims the term "comprising" can be replaced by "consisting of" and "reference modulation value/s" can be substituted with "modulation cut-off value/s" or "reference modulation cut-off value/s".

The examples below are not intended as limiting the present invention.

### EXAMPLES

### 1. Tested Products composition

### Product A (Arté GX) (figures 2-6)

| | |
|---|---|
| Centella Asiatica dry Leaves | 90% w/w |
| Echinacea purpurea dry Flowers | 10% w/w |
| Coextracted in water | |

### Product B (figure 9)

| | |
|---|---|
| Melissa officinalis leaves dry extract | 2.5% w/w |
| Royal jelly lyophilised | 2.5% w/w |
| Blueberry dry extract | 0.29% w/w |
| Concentrated blueberry juice | 5% w/w |
| Cynara scolymus L. leaves dry extract | 0.05% w/w |
| Curcuma longa L. radix dry extract | 0.16% w/w |
| Medicago sativa seeds dry extract | 1.6% w/w |
| Panax ginseng radix dry extract | 1.6% w/w |
| Honeycomb dry extract | 1.6% w/w |
| Concentrated apple juice | 44.35% w/w |
| Clarified lemon juice | 0.5% w/w |
| Honey | 30% w/w |
| Deionised water | 6.45% w/w |
| Malpighia emarginata juice | 1% w/w |
| Sambucus nigrum juice | 2% w/w |

### Product C (figure 11)

| | |
|---|---|
| Coral calcium powder | 32% w/w |
| Egg shell calcium powder | 30.2% w/w |
| Coral calcium citrate powder | 13% w/w |
| Agaricus bisporum powder | 4.65% w/w |
| Equisetum arvense flowering tops dry extract | 2% w/w |
| Malpighia emarginata carried by inulin dry extract | 1.50% w/w |
| Cetraria islandica powder | 2% w/w |
| Agave sisalana leaves powder | 12% w/w |
| Acacia senegal powder | 2.15% w/w |

In the examples, the word compliant, when referred to the cell-based assay samples indicates the acceptability of the batches with reference to the modulation values of the reference standard, when referred to the spectroscopy or spectrophotometry analysis it refers to the conformity of the batch with the desired quality of the product for final commercialisation.

### 2. In vitro cell assay representative of Osteoarthritis

An *in vitro* cellular model capable of recapitulating features of osteoarthritis [1-3] was established by exposing primary human chondrocytes (HC, Cell Application INC 402K-05) to IL1B [5 ng/ml] for 6 hours, once the cellular model was established it was exposed over 24 hours to five different batches of "Arté-GX" [1.4 mg/ml]:
□ Batch 20B1955 (reference standard)
□ Batch 20I1279
□ Batch 20J1770
□ Batch 20B0596
□ in a successive moment, Batch 21E1640 as well as an aliquot of said batch that was treated to induce destabilisation of the product, herein referred as Batch DEST 21E1640, were also analysed separately (see figures 3 and 4).

Each time one of the batch solutions was added, fresh IL1B [5 ng/ml] was also added to the medium.

### 2.1.1. Time schedule on chondrocyte experiment

The time schedule used for experimental setting is as follows:

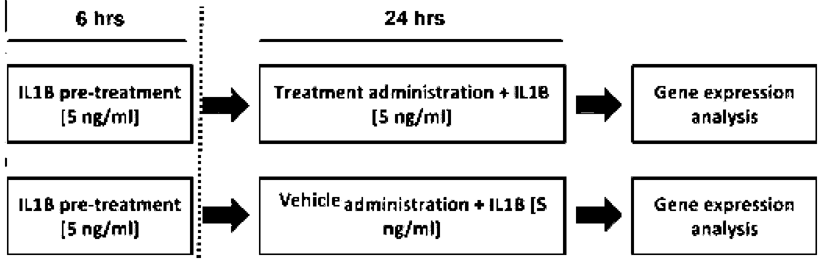

### 2.1.2. Gene expression analysis

At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene expression analysis experiments. Total RNA was extracted from cells lysates using an QIAsymphony RNA Kit (Qiagen,) with the QIAsymphony SP instrument (Qiagen).

The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained, and then scanned using the GeneTitan MC Instrument (Applied Biosystems, ThermoFisher Scientific) and CEL Intensity files were generated by Affymetrix GeneChip Command Console Software (AGCC, ThermoFisher Scientific).

### 2.1.3. Transcriptomics data analysis

Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05).

2.1.4. Bioinformatic modelling of experimentally observed transcriptomics data Ingenuity Pathways Analysis (IPA) (QIAGEN \Inc., https://www.qiagenbioinformatics.com/products/ingenuitypathway-analysis) [4] was used, for each investigated batch to evaluate modulations of gene expression relevant for effects of interest.

IPA is an aggregator of scientific references that allows to search for information on genes/proteins and the construction of networks that predict the behaviour of biological systems according to their gene expression status.

The patho-physiological features of state-of-the-art "Osteoarthritis condition" were considered with particular attention to the following areas involved:

| **Keywords used to interrogate IPA** | **Chosen biological function** |
|---|---|
| Osteoarthritis [osteoarthrosis] | Non-traumatic arthropathy:Osteoarthritis |
| Arthralgia/arthritis arthropathy | |
| Damage of cartilage tissue cartilage tissue | Formation of cartilage tissue |
| Inflammation of joint | Inflammation of joint |
| Pain of joint | |

This knowledge was used to interrogate IPA via the "IPA Bioprofiler" tool, using the above key words.

The use of "IPA Bioprofiler" allowed to identify clusters of expressed genes causally linked to each of the identified biological activities and the specific molecular pathway underpinning them. Information concerning the measured gene expression data (Fold change value cut-off ≤-2 and ≥ +2 and p-value≤0.05) induced by each batch was then superimposed on the networks obtained, to define influenced genes and modulation of the connected biofunction.

Modulation of expressed genes were shown in different intensities of blue (signifying down-modulation) or red (signifying up-modulation). The resulting expected calculated impact, based on the literature, on the related biofunctions was determined by "IPA Molecule Activity Predictor" tool (MAP) and resumed in a heatmap visualization.

The colour and intensity thereof were transformed into numerical values.

### 2.1.5. Results

The results of these tests yielded a comparative study of the performance and of the mechanism of action of five different batches of Arté GX. The analysis revealed that while all the different batches were capable of returning reproducible biological effects, it was also possible to identify batch-specific fluctuations in the induced transcriptional pattern. Evidently the induction of slightly different transcriptional patterns still results in the same desirable regulation of biological activities. This is due to a functional redundancy in the interactions between the components of the product and the body, whereby, by virtue of the multifocal mechanism of action, slightly different compositions elicit the same effect (Figs.3 and 4).

The five batches are therefore considered as having equivalent biological outputs since the induction and repression patterns are conserved.

From the results summarised in figure 4 it is evident that the observed transcriptional patterns of the five different batches elicit a very reproducible biological effect leading to a general modification of the pathological processes and of the overall pathological state equivalence for all the batches reported in Figure 4.

### 3.1. Targeted metabolomics

To appreciate whether the final matrix constituting "Arté GX" is characterized by the matrix effect, a series of analyses to grasp the product's features on different aspects was carried out on the batches reported above. A targeted metabolomics analysis capable of identifying most of such molecular components, was carried out together with the other analysis reported herein.

The products, as described above, consist of two vegetal matrices assembled and resulting in a final new vegetal matrix. Several analytical techniques have been used to identify and quantify compounds belonging to the main classes present in plants. Although metabolomic analysis does not allow to appreciate the dynamic changes within the components of the matrix, it allows a "picture" of the composition in the moment the analysis is carried out.

In the following analysis each individual component (plant metabolite) is specifically researched, for this reason the analysis is called "targeted metabolomics". This analysis allows to capture a frame on the qualitative data, by determining the chemical compounds present in the material, and quantitative data, by defining the concentrations of each compound in the material.

For Arté GX, a qualitative and quantitative characterization of as many primary and secondary metabolites as possible was carried out using an "omic" approach, the targeted metabolomics analysis, based on the use of multiple analytical methodologies. The analytical methods used for the chemical characterization of each batch are described below. The most appropriate analytical techniques have been adopted based on the chemical nature of the classes of compounds present. The analysis with chromatographic methods combined with different detection techniques (e.g., GC and LC each combined with a suitable detector), made it possible to identify and quantify, as appropriate, the organic compounds. The inductively coupled plasma analysis using a single quadrupole mass spectrometer (ICP-MS) or an optical emission spectrometer (ICP-OES) made it possible to establish the levels of elements present, while the anions were determined by ion chromatography and conductivity detector. Other gravimetric methods were used for the determination of classes of substances non-quantifiable by means of chromatographic methods.

The table below summarizes all the methods used.

| **Class of Compounds** | **Characteristics** | **Type of Method** | **Short description** |
|---|---|---|---|
| **Fibres** | Insoluble Fibers | Gravimetric | Method AOAC 991.42 **(A1)** |
| | Soluble fibres | Gravimetric | Method AOAC 993.19 **(A2)** |
| | FOS, Fructans | HPAEC-PAD | Extraction of FOS and Fructans followed by enzymatic digestion; analysis by ion chromatographer equipped with a pulsed amperometric detector. **(A3)** |
| **Polysaccharides** | Polysaccharides ≥ 20.000 Dalton | HPLC-RID | Extraction of water-soluble polysaccharides and analysis by HPLC equipped with molecular exclusion column and refractive index detector. **(B)** |
| **Water** | Loss on drying | Gravimetric | Method ISTISAN 1996/34 (pages 7-10). **(C)** |
| **Phenols** | Phenols polar | UHPLC-qToF | Sample extraction and reverse phase chromatography analysis by UHPLC coupled to a quadrupole-time-of-flight mass spectrometer. **(D1)** |
| | | HPLC-UV | Sample extraction and reverse phase chromatography analysis by HPLC coupled to UV-VIS detector. **(D2)** |
| **Terpenes** | Terpenes polar | UHPLC-qToF | Sample extraction and reverse phase chromatography analysis by UHPLC coupled to a quadrupole-time-of-flight mass spectrometer. **(E1)** |
| | | HPLC-UV | Sample extraction and reverse phase chromatography analysis by HPLC coupled to UV-VIS detector. **(E2)** |
| | Terpenes apolar | GC-TQ | Headspace analysis of the sample by gas chromatography coupled to a triple quadrupole mass spectrometer. **(E3)** |
| | Sterols | GC-FID | Analysis of the sample by gas chromatograph coupled to a flame ionization detector, after derivatization and transformation into trimethylsilyl ethers. **(E4)** |
| **Organic** acids | Organic acids mono-, di-, tri-carboxylic | HPLC-UV | Sample extraction and analysis by HPLC coupled to UV detector. **(F)** |
| **NITROGEN Compounds** | Nitrogen-containing substances, total | Kjeldahl | Method AOAC 920.53. **(G)** |
| **Lipids** | Fats, total | Gravimetric | Method ISTISAN 1996/34 (pag 39-40). **(H1)** |
| | Fatty acids derivatives | GC-TQ | Headspace analysis of the sample by gas chromatograph coupled to a triple quadrupole mass spectrometer. **(H2)** |
| | Fatty acids | HPLC-UV | Sample extraction and analysis by HPLC coupled to UV detector. **(H3)** |
| | Fatty acids | GC-FID | Analysis of the sample by gas chromatograph coupled to a flame ionization detector, after derivatization. **(H4)** |
| **Sugars and Derivatives** | Monosaccharides | IC-PAD | Sample extraction and analysis by ion chromatographer coupled to pulsed amperometric detector. **(I)** |
| | Disaccharides | IC-PAD | Sample extraction and analysis by ion chromatographer coupled to pulsed amperometric detector. **(I)** |
| **Other Organic Compounds** | Other organic compounds, apolar | GC-TQ | Headspace analysis of the sample by gas chromatographer coupled to a triple quadrupole mass spectrometer. **(L)** |
| **Inorganic Compounds** | Elements | ICP-MS, ICP-OES | Acid mineralization of the sample in a microwave oven and analysis of the conductively induced plasma by means of a single quadrupole mass spectrometer, or optical emission spectrometer. **(M1)** |
| | | IC-CD | Sample extraction and analysis by ion chromatographer coupled to conductometric detector. **(M2)** |
| | Anions | IC-CD | Sample extraction and analysis by ion chromatographer coupled to conductometric detector. **(M2)** |
| **Total RNA** | Nucleosides | UHPLC-qToF | After extraction and digestion of RNA from the sample a reverse phase chromatography analysis was applied by UHPLC coupled to a quadrupole-time-of-flight mass spectrometer. **(N)** |

The results, that are summarised in the table below, show an appreciable composition variability between the different batches and underline the impossibility to recapitulate the properties of the matrix as the sum of its single components. The work performed and reported herein (see cell-based assay results) together with the data below, demonstrates that the biological effect elicited by a product comprising or consisting of one or more natural matrices cannot be recapitulated by the sum of the effects elicited by the single molecular components but is the result of interconnections and interactions among the components: the matrix effect. This translates into the impossibility to formally define a structure-activity relationship (SAR) according to the principles canonically applied to APIs.

| **Method** | **COMPOUNDS** | **21E1640 (%)** | **20B1955 (%)** | **20I1279 (%)** | **20J1770 (%)** | **20B0596 (%)** | ***21E1640 (d%)*** | ***20I1279 (d%)*** | ***20J1770 (d%)*** | ***20B0596 (d%)*** |
|---|---|---|---|---|---|---|---|---|---|---|
| G | **NITROGEN-CONTAINING SUBSTANCES, Total** | 5,1 | 5,1 | 5 | 4,7 | 0,76 | 0,00 | 1,96 | 7,84 | 85,10 |
| | **FIBERS, Total** | 5,4 | 2,3 | 1,5 | 1,7 | 1,8 | 134,78 | 34,78 | 26,09 | 22,26 |
| A2 | **SOLUBLE FIBERS, Total** | 5,4 | 2,3 | 1,5 | 1,1 | 1,5 | 134,78 | 34,78 | 52,17 | 34,78 |
| A3 | **FRUCTOLIGOSAC CHARIDES, Total** | <LdQ | <LdQ | <LdQ | 0,6 | 0,2880 | / | / | / | / |
| A1 | **INSOLUBLE FIBERS, Total** | <LdQ | <LdQ | <LdQ | <LdQ | <LdQ | / | / | / | / |
| B | **MACROMOLECUL ES > 20000 (POLYSACCHARID ES), Total** | <LdQ | <LdQ | <LdQ | <LdQ | <LdQ | / | / | / | / |
| C | **WATER, Total** | 3,4 | 5,4 | 4 | 4,10 | 4,50 | 37,04 | 25,93 | 24,07 | 16,67 |
| | **PHENOLS, Total** | 4,57 | 3,45 | 3,20 | 3,33 | 2,97 | 32,30 | 7,37 | 3,48 | 13,95 |
| | **FLAVONOIDS, Total** | 1,80 | 1,49 | 1,49 | 1,74 | 1,18 | 20,48 | 0,47 | 16,39 | 20,81 |
| D1 | Rottlerin | <LdQ | <LdQ | <LdQ | <LdQ | / | / | / | / | / |
| D1 | Phloridzin | nq | nq | nq | / | / | / | / | / | / |
| | **FLAVANONES, Total** | 0,01 | 0,0052 | 0,0096 | nq | nq | 92,31 | 84,62 | / | / |
| D1 | Flavanomarein | 0,01 | 0,0052 | 0,0096 | nq | nq | 92,31 | 84,62 | / | / |
| | **FLAVONES, Total** | 0,0258 | 0,02 | 0,0297 | 0,023858 | 0,0134 | 29,00 | 48,50 | 19,29 | 33,00 |
| D1 | 3',4',5,5',6,7-Hexamethoxyflav one | <LdQ | 0,0002 | 0,0002 | 0,000119 | 0,0002 | / | 0,00 | 40,55 | 5,52 |
| D1 | Diosmin | nd | <LdQ | <LdQ | <LdQ | / | / | / | / | / |
| D1 | Homoorientin | 0,009 | 0,0063 | 0,0128 | 0,003715 | 0,0037 | 42,86 | 103,17 | 41,03 | 41,65 |
| D1 | Isoschaftoside | 0,0008 | 0,001 | 0,0009 | 0,004852 | 0,0022 | 20,00 | 10,00 | 385,20 | 124,34 |
| D1 | Isovitexin | 0,0012 | 0,001 | 0,0011 | 0,000779 | 0,0006 | 20,00 | 10,00 | 22,10 | 40,89 |
| D1 | Linarin | nd | nd | nd | <LdQ | / | / | / | / | / |
| D1 | Luteolin-7-O-beta-D-glucoside (Cynaroside) | nd | nd | nd | <LdQ | / | / | / | / | / |
| D1 | Luteolin-7-O-glucuronide | nd | nd | nd | <LdQ | <LdQ | / | / | / | / |
| D1 | Nobiletin | <LdQ | <LdQ | <LdQ | <LdQ | <LdQ | / | / | / | / |
| D1 | Orientin | 0,0025 | 0,0026 | 0,0018 | nq | nq | 3,85 | 30,77 | / | / |
| D1 | Schaftoside | 0,0067 | 0,0044 | 0,0081 | 0,008796 | 0,0036 | 52,27 | 84,09 | 99,91 | 19,06 |
| D1 | Vicenin-2 | 0,0038 | 0,0032 | 0,0029 | 0,004069 | 0,0021 | 18,75 | 9,38 | 27,16 | 33,32 |
| D1 | Vitexin | 0,0018 | 0,0013 | 0,0019 | 0,001528 | 0,0010 | 38,46 | 46,15 | 17,55 | 25,67 |
| | **FLAVONOLS, Total** | 1,7619 | 1,4669 | 1,4458 | 1,707743 | 1,1682 | 20,11 | 1,44 | 16,42 | 20,36 |
| D1 | Isorhamnetin-3-O-glucoside | 0,0022 | 0,0018 | 0,002 | 0,001514 | 0,0016 | 22,22 | 11,11 | 15,92 | 11,28 |
| D1 | Isorhamnetin-3-O-rutinoside | <LdQ | <LdQ | <LdQ | <LdQ | / | / | / | / | / |
| D1 | Kaempferol | 0,0062 | 0,0136 | 0,0104 | 0,002918 | 0,0030 | 54,41 | 23,53 | 78,54 | 77,76 |
| D1 | Kaempferol-3-O-glucoside | 0,0342 | 0,1187 | 0,0743 | 0,032471 | 0,0729 | 71,19 | 37,41 | 72,64 | 38,56 |
| D1 | Kaempferol-3-O-glucuronide | 0,3409 | 0,2751 | 0,2855 | 0,618309 | 0,5180 | 23,92 | 3,78 | 124,76 | 88,31 |
| D1 | Kaempferol-3-O-rutinoside | 0,0076 | 0,0119 | 0,0092 | 0,00195 | 0,0035 | 36,13 | 22,69 | 83,61 | 70,69 |
| D1 | Quercetin | 0,0201 | 0,0352 | 0,0159 | 0,012575 | 0,0192 | 42,90 | 54,83 | 64,27 | 45,36 |
| D1 | Quercetin-3-O-glucopyranoside (Isoquercetin) | 0,0694 | 0,0871 | 0,0694 | 0,025885 | 0,0598 | 20,32 | 20,32 | 70,28 | 31,36 |
| D1 | Quercetin-3-O-glucuronide | 1,2456 | 0,8651 | 0,9198 | 0,985214 | 0,4558 | 43,98 | 6,32 | 13,88 | 47,31 |
| D1 | Rutin | 0,0357 | 0,0584 | 0,0593 | 0,026907 | 0,0313 | 38,87 | 1,54 | 53,93 | 46,34 |
| | **ISOFLAVONES, Total** | <LdQ | <LdQ | <LdQ | <LdQ | <LdQ | / | / | / | / |
| D1 | Genistin | <LdQ | <LdQ | <LdQ | <LdQ | <LdQ | / | / | / | / |
| | **PHENOLIC ACIDS, Total** | 0,0745 | 0,0707 | 0,0923 | 0,077983 | 0,1091 | 5,37 | 30,55 | 10,30 | 54,35 |
| D1 | Protocatechuic acid | 0,0745 | 0,0707 | 0,0923 | 0,077983 | 0,1091 | 5,37 | 30,55 | 10,30 | 54,35 |
| D1 | Vanillic acid | nd | nd | nd | <LdQ | / | / | / | / | / |
| | **PHENYLPROPAN OIDS, Total** | 2,682 | 1,872 | 1,606 | 1,490576 | 0,929 | 43,25 | 14,23 | 20,39 | 50,38 |
| | **COUMARINS, Total** | 0,0149 | 0,0168 | 0,0164 | 0,007515 | 0,0022 | 11,31 | 2,38 | 55,27 | 86,90 |
| D1 | Aesculin | 0,0051 | 0,0043 | 0,0112 | 0,006231 | 0,0047 | 18,60 | 160,47 | 44,90 | 8,63 |
| D1 | Esculetin | 0,0098 | 0,0125 | 0,0052 | 0,001284 | 0,0053 | 21,60 | 58,40 | 89,73 | 57,65 |
| D1 | Fraxin | <LdQ | <LdQ | <LdQ | / | <LdQ | / | / | / | / |
| | **HYDROXYCINNA MIC ACIDS, Total** | 2,667 | 1,856 | 1,590 | 1,442046 | 0,9068 | 43,75 | 14,33 | 22,29 | 51,13 |
| D1 | 3,5-Dicaffeoylquinic acid | 1,043 | 0,5573 | 0,516 | 0,4833 | 0,2254 | 87,15 | 7,41 | 13,28 | 59,56 |
| D1 | 4,5-Dicaffeoylquinic acid | 0,1822 | 0,1601 | 0,1568 | 0,058033 | 0,0547 | 13,80 | 2,06 | 63,75 | 65,85 |
| D1 | Caffeic acid | 0,0219 | 0,0168 | 0,0274 | 0,008865 | 0,0089 | 30,36 | 63,10 | 47,23 | 47,04 |
| D2 | Caftaric acid | 0,2338 | 0,2825 | 0,2005 | 0,1949 | 0,2198 | 17,24 | 29,03 | 31,01 | 22,19 |
| D2 | Chicoric acid | 0,282 | 0,2948 | 0,17 | 0,1748 | 0,2142 | 4,34 | 42,33 | 40,71 | 27,34 |
| D2 | Chlorogenic acid | 0,8213 | 0,4702 | 0,4644 | 0,4276 | 0,2931 | 74,67 | 1,23 | 9,06 | 37,66 |
| D2 | Echinacoside | <LdQ | <LdQ | <LdQ | / | / | / | / | / | / |
| D1 | Ferulic acid | 0,0081 | 0,0047 | 0,0055 | 0,003602 | nq | 72,34 | 17,02 | 23,36 | / |
| D1 | Neochlorogenic acid | 0,0626 | 0,0658 | 0,0445 | 0,090946 | 0,0598 | 4,86 | 32,37 | 38,22 | 9,18 |
| D1 | Rosmarinic acid | 0,0082 | 0,0034 | 0,0026 | nq | / | 141,18 | 23,53 | / | / |
| D1 | Verbascoside | 0,0043 | <LdQ | 0,0019 | / | nq | / | / | / | / |
| | **PHLOROGLUCIN OLS, Total** | nq | nq | nq | <LdQ | / | / | / | / | / |
| D1 | Phloroglucinol carboxylic acid | nq | nq | nq | <LdQ | / | / | / | / | / |
| | **SALICYLATES, Total** | 0,0118 | 0,0162 | 0,0136 | 0,0145 | 0,006522 | 27,16 | 16,05 | 10,50 | 59,74 |
| D1 | Salicylic acid | 0,0118 | 0,0162 | 0,0136 | 0,0145 | 0,006522 | 27,16 | 16,05 | 10,50 | 59,74 |
| | **TERPENES, Total** | 13,63 | 15,80 | 16,59 | 14,92 | 14,93 | 13,74 | 5,02 | 5,57 | 5,49 |
| | **MONOTERPENES, Total** | 0,000408 | 0,000329 | 0,00042 | 0,000305 | 0,0008 | 23,96 | 29,09 | 7,15 | 143,18 |
| | **MONOTERPENE ALCOHOLS, Total** | 0,000355 | 0,00028 | 0,00037 | 0,000278 | 0,000309 | 26,59 | 32,76 | 0,95 | 10,17 |
| E3 | alpha-Terpineol | <LdQ | <LdQ | <LdQ | <LdQ | <LdQ | / | / | / | / |
| E3 | Carvacrol | 4,49E-05 | 5,08E-05 | 5,1E-05 | 2,61E-05 | 9,34E-05 | 11,67 | 0,85 | 48,61 | 83,83 |
| E3 | Eucalyptol | <LdQ | nd | nd | <LdQ | <LdQ | / | / | / | / |
| E3 | Linalool | 0,00031 | 0,00023 | 0,00032 | 0,000252 | 0,000216 | 35,06 | 39,82 | 9,59 | 6,13 |
| | **MONOTERPENE HYDROCARBONS, Total** | 5,27E-05 | 4,85E-05 | 5,2E-05 | 2,77E-05 | <LdQ | 8,70 | 7,90 | 42,97 | / |
| E3 | 4-Cymene | 5,27E-05 | 4,85E-05 | 5,2E-05 | 2,77E-05 | <LdQ | 8,70 | 7,90 | 42,97 | / |
| E3 | alpha-Pinene | nd | nd | <LdQ | <LdQ | <LdQ | / | / | / | / |
| E3 | alpha-Terpinene | <LdQ | <LdQ | <LdQ | <LdQ | <LdQ | / | / | / | / |
| E3 | beta-Pinene | <LdQ | <LdQ | <LdQ | <LdQ | <LdQ | / | / | / | / |
| E3 | Camphene | <LdQ | <LdQ | <LdQ | <LdQ | <LdQ | / | / | / | / |
| E3 | gamma-Terpinene | <LdQ | <LdQ | <LdQ | <LdQ | <LdQ | / | / | / | / |
| E3 | Myrcene | <LdQ | <LdQ | <LdQ | <LdQ | <LdQ | / | / | / | / |
| E3 | Sabinene | <LdQ | nd | <LdQ | <LdQ | <LdQ | / | / | / | / |
| E3 | Terpinolene | <LdQ | <LdQ | <LdQ | <LdQ | <LdQ | / | / | / | / |
| | **TRITERPENES, Total** | 13,627 | 15,799 | 16,592 | 14,919 | 14,9314 | 13,74 | 5,02 | 5,57 | 5,49 |
| | **PHYTOSTEROLS, Total** | 0,001141 | 0,001289 | <LdQ | / | / | 11,45 | / | / | / |
| E4 | 24-Methylenecholesterol | 0,000056 | <LdQ | <LdQ | / | / | / | / | / | / |
| E4 | 7-Dehydrocholesterol | <LdQ | <LdQ | <LdQ | / | / | / | / | / | / |
| E4 | beta-Sitosterol | 0,000435 | 0,000578 | <LdQ | / | / | 24,65 | / | / | / |
| E4 | Brassicasterol | <LdQ | <LdQ | <LdQ | / | / | / | / | / | / |
| E4 | Campestanol | 0,000181 | 6,19E-05 | <LdQ | / | / | 192,73 | / | / | / |
| E4 | Campesterol | 0,000149 | 0,000125 | <LdQ | / | / | 19,35 | / | / | / |
| E4 | Cholesterol | 8,78E-05 | 0,000075 | <LdQ | / | / | 17,32 | / | / | / |
| E4 | Clerosterol | <LdQ | <LdQ | <LdQ | / | / | / | / | / | / |
| E4 | delta-5-Avenasterol | <LdQ | 7,1E-05 | <LdQ | / | / | / | / | / | / |
| E4 | delta-5,23-Stigmastadienol | <LdQ | <LdQ | <LdQ | / | / | / | / | / | / |
| E4 | delta-5,24-Stigmastadienol | <LdQ | <LdQ | <LdQ | / | / | / | / | / | / |
| E4 | delta-7-Avenasterol | <LdQ | <LdQ | <LdQ | / | / | / | / | / | / |
| E4 | delta-7-Campesterol | <LdQ | <LdQ | <LdQ | / | / | / | / | / | / |
| E4 | delta-7-Stigmastenol | 0,000074 | 0,000107 | <LdQ | / | / | 30,79 | / | / | / |
| E4 | delta-7,9(11)-Stigmastadienol | <LdQ | <LdQ | <LdQ | / | / | / | / | / | / |
| E4 | Sitostanol | 0,000041 | 9,55E-05 | <LdQ | / | / | 57,01 | / | / | / |
| E4 | Stigmasterol | 0,000116 | 0,000175 | <LdQ | / | / | 33,72 | / | / | / |
| | **SAPOGENINS, Total** | 0,0091 | 0,0154 | 0,0176 | <LdQ | 0,0054 | 40,91 | 14,29 | / | 64,94 |
| E1 | Asiatic acid | 0,001 | 0,0033 | 0,0024 | <LdQ | <LdQ | 69,70 | 27,27 | / | / |
| E1 | Madecassic acid | 0,0081 | 0,0121 | 0,0152 | <LdQ | 0,005364 | 33,06 | 25,62 | / | 55,67 |
| | **SAPONINS, Total** | 13,617 | 15,782 | 16,574 | 14,919 | 14,926 | 13,72 | 5,02 | 5,47 | 5,42 |
| E2 | Asiaticoside | 5,526 | 7,121 | 6,935 | 6,143 | 6,801 | 22,40 | 2,61 | 13,73 | 4,49 |
| E2 | Madecassoside | 8,091 | 8,661 | 9,639 | 8,776 | 8,125 | 6,58 | 11,29 | 1,33 | 6,19 |
| | **SESQUITERPENES** , **Total** | <LdQ | nd | <LdQ | <LdQ | nd | / | / | / | / |
| E3 | Farnesol | <LdQ | nd | <LdQ | / | / | / | / | / | / |
| | **ORGANIC ACIDS, Total** | 10,37 | 14,48 | 9,94 | 9,03 | 15,07 | 28,38 | 31,31 | 37,66 | 4,07 |
| | **MONOCARBOXYL IC ACIDS, Total** | <LdQ | 2,03 | <LdQ | 1,431 | 1,627 | / | / | 29,51 | 19,85 |
| F | Acetic acid | <LdQ | 1,07 | <LdQ | 0,151 | 0,177 | / | / | 85,89 | 83,46 |
| F | Formic acid | <LdQ | <LdQ | <LdQ | <LdQ | <LoQ | / | / | / | / |
| F | Lactic acid | <LdQ | 0,96 | <LdQ | 1,28 | 1,45 | / | / | 33,33 | 51,04 |
| | **DICARBOXYLIC ACIDS, Total** | 8,2088 | 10,4075 | 7,6748 | 4,7048 | 10,05 | 21,13 | 26,26 | 54,79 | 3,44 |
| F | Fumaric acid | 0,067 | 0,0446 | 0,067 | 0,13 | 0,136 | 50,22 | 50,22 | 191,48 | 204,93 |
| F | Malic acid | 8,08 | 2,2 | 7,54 | 4,55 | 5,75 | 267,27 | 242,73 | 106,82 | 161,36 |
| F | Succinic acid | <LdQ | 8,14 | <LdQ | <LdQ | 4,13 | / | / | / | 49,26 |
| F | Tartaric acid | 0,0618 | 0,0229 | 0,0678 | 0,0248 | 0,034 | 169,87 | 196,07 | 8,30 | 48,47 |
| | **TRICARBOXYLIC ACIDS, Total** | 2,16 | 2,04 | 2,27 | 2,89 | 3,39 | 5,88 | 11,27 | 41,67 | 66,18 |
| F | Citric acid | 2,16 | 2,04 | 2,27 | 2,89 | 3,39 | 5,88 | 11,27 | 41,67 | 66,18 |
| | **SUGARS AND DERIVATIVES, Total** | 11,42 | 1,85 | 10,78 | 13,34 | 7,46 | 516,13 | 481,66 | 619,61 | 302,37 |
| | **MONOSACCHARI DES, Total** | 11,423 | 1,854 | 10,784 | 10,0247 | 7,16 | 516,13 | 481,66 | 440,71 | 286,19 |
| I | Fructose | 5,707 | 1,854 | 5,38 | 6,20591 | 4,5300 | 207,82 | 190,18 | 234,73 | 144,34 |
| I | Galactose | 1,369 | <LdQ | 1,315 | 1,30558 | 1,1000 | / | / | / | / |
| I | Glucose | 4,347 | <LdQ | 4,089 | 2,51321 | 1,2900 | / | / | / | / |
| | **DISACCHARIDES, Total** | <Ldq | <Ldq | <Ldq | 3,31694 | 0,30 | / | / | / | / |
| I | Lactose | <Ldq | <Ldq | <Ldq | <LdQ | / | / | / | / | / |
| I | Maltose | <Ldq | <Ldq | <Ldq | 1,54 | / | / | / | / | / |
| I | Sucrose | nq | nq | nq | 1,77694 | 0,30 | / | / | / | / |
| | **LIPIDS, Total** | 0,200 | 0,170 | 0,190 | 4,03E-05 | 5,88E-05 | 17,64 | 11,76 | 99,98 | 99,97 |
| | **FATTY ACIDS, Total** | 0,2 | 0,17 | 0,19 | <LdQ | 5,88E-05 | 17,65 | 11,76 | / | 99,97 |
| H4 | Decanoic acid | nd | nd | 0,01 | / | / | / | / | / | / |
| H4 | Dodecanoic acid | 0,02 | nd | nd | / | / | / | / | / | / |
| H4 | Linoleic acid | nd | nd | nd | / | / | / | / | / | / |
| H4 | Linolenic acid | nd | nd | nd | / | / | / | / | / | / |
| H4 | Myristic acid | nd | nd | nd | / | / | / | / | / | / |
| H4 | Octanoic acid | nd | nd | 0,01 | / | / | / | / | / | / |
| H4 | Oleic acid | nd | nd | nd | / | / | / | / | / | / |
| H4 | Palmitic acid | 0,1 | 0,1 | 0,09 | / | / | 0,00 | 10,00 | / | / |
| H3 | Propionic acid | <LdQ | <LdQ | <LdQ | / | / | / | / | / | / |
| H4 | Stearic acid | 0,08 | 0,07 | 0,08 | / | / | 14,29 | 14,29 | / | / |
| | **FATTY ACID DERIVATIVES, Total** | 9,38E-05 | 9,37E-05 | 9,5E-05 | 4,03E-05 | 5,88E-05 | 0,09 | 1,20 | 57,05 | 37,24 |
| | **FATTY ACID ESTERS, Total** | 9,38E-05 | 9,37E-05 | 9,5E-05 | 4,03E-05 | 5,88E-05 | 0,09 | 1,20 | 57,05 | 37,24 |
| H2 | Ethyl palmitate | 9,38E-05 | 9,37E-05 | 9,5E-05 | 4,03E-05 | 5,88E-05 | 0,09 | 1,20 | 57,05 | 37,24 |
| | **OTHER ORGANIC COMPOUNDS, Total** | 0,00069 | 0,00048 | 0,00069 | 0,00064 | 0,000361 | 44,12 | 44,78 | 33,41 | 24,64 |
| | **AROMATIC COMPOUNDS, Total** | 0,000691 | 0,00048 | 0,00069 | 0,00064 | 0,000361 | 44,12 | 44,78 | 33,41 | 24,64 |
| | **AROMATIC ALCOHOLS, Total** | 0,000665 | / | 0,00067 | 0,000622 | 0,000361 | / | / | / | / |
| L | para,alpha,alpha-Trimethylbenzyl alcohol | 0,000665 | 0,000466 | 0,00067 | 0,000622 | 0,000361 | 42,67 | 44,25 | -33,35 | 22,46 |
| L | Benzaldehyde | <LdQ | <LdQ | <LdQ | <LdQ | <LdQ | / | / | / | / |
| | **STYRENES, Total** | 2,62E-05 | 1,35E-05 | 2,2E-05 | 1,82E-05 | <LdQ | 94,07 | 62,94 | 35,28 | / |
| L | Dimethylstyrene | 2,62E-05 | 1,35E-05 | 2,2E-05 | 1,82E-05 | <LdQ | 94,07 | 62,94 | 35,28 | / |
| | **INORGANIC COMPOUNDS, Total** | 21,85 | 26,21 | 21,45 | 22,98 | 27,44 | 16,63 | 18,18 | 12,34 | 4,68 |
| | **ANIONS, Total** | 4,6313 | 6,34 | 4,4114 | 5,463733 | 5,53 | 26,95 | 30,42 | 13,82 | 12,78 |
| M2 | Nitrate | 0,0013 | <LdQ | 0,0014 | 0,092379 | <IdQ | / | / | / | / |
| M2 | Nitrite | <LdQ | <LdQ | <LdQ | <LdQ | <IdQ | / | / | / | / |
| M2 | Phosphate | 1,63 | 1,74 | 1,55 | 1,529767 | 1,4700 | 6,32 | 10,92 | 12,08 | 15,52 |
| M2 | Sulfate | 3 | 4,6 | 2,86 | 3,841587 | 4,0600 | 34,78 | 37,83 | 16,49 | 11,74 |
| | **MACROELEMENT S, Total** | 17,05 | 19,66 | 16,87 | 17,32551 | 21,9985 | 13,28 | 14,18 | 11,88 | 11,89 |
| M1 | Calcium | 1,745388 | 2,544027 | 1,75523 | 1,847967 | 2,8998 | 31,39 | 31,01 | 27,36 | 13,98 |
| M2 | Chloride | 4,67 | 5,32 | 4,36 | 5,496178 | 4,8700 | 12,22 | 18,05 | 3,31 | 8,46 |
| M1 | Magnesium | 1,270564 | 1,533499 | 1,25715 | 1,233837 | 1,6827 | 17,15 | 18,02 | 19,54 | 9,73 |
| M1 | Phosphorus | 0,624007 | 0,619255 | 0,60815 | 0,579524 | 0,7223 | 0,77 | 1,79 | 6,42 | 16,64 |
| M1 | Potassium | 5,728856 | 6,134733 | 5,80766 | 5,657116 | 7,6743 | 6,62 | 5,33 | 7,79 | 25,10 |
| M1 | Sodium | 2,326488 | 2,741546 | 2,40561 | 2,510884 | 3,8194 | 15,14 | 12,25 | 8,41 | 39,31 |
| M1 | Sulfur | 0,683772 | 0,767168 | 0,6784 | 1,56 | <LdQ | 10,87 | 11,57 | 103,35 | / |
| | **MICROELEMENTS** , **Total** | 0,137432 | 0,165006 | 0,12715 | 0,134181 | 0,1918 | 16,71 | 22,94 | 18,68 | 16,24 |
| M1 | Chromium | 3,09E-05 | 0,000102 | 0,00011 | 8,77E-05 | 0,0007 | 69,77 | 9,86 | 14,21 | 567,60 |
| M1 | Cobalt | 0,000127 | 0,000122 | 0,00013 | 0,000149 | 0,0001 | 4,08 | 8,87 | 22,19 | 7,45 |
| M1 | Copper | 0,000718 | 0,000226 | 0,00091 | 0,000945 | 0,0009 | 217,47 | 303,07 | 317,91 | 287,93 |
| M2 | Fluoride | NQ | NQ | NQ | <Ldq | nq | / | / | / | / |
| M1 | Iron | 0,000372 | 0,000777 | 0,00326 | 0,002209 | 0,0027 | 52,15 | 319,33 | 184,10 | 243,53 |
| M1 | Manganese | 0,128456 | 0,156247 | 0,11479 | 0,121585 | 0,1722 | 17,79 | 26,53 | 22,18 | 10,20 |
| M1 | Molybdenum | <LdQ | <LdQ | <LdQ | <LdQ | <LdQ | / | / | / | / |
| M1 | Nickel | 0,000873 | 0,000727 | 0,00106 | 0,00111 | 0,0008 | 20,11 | 45,89 | 52,65 | 3,32 |
| M1 | Selenium | <LdQ | <LdQ | <LdQ | <LdQ | <LdQ | / | / | / | / |
| M1 | Tin | <LdQ | <LdQ | <LdQ | <LdQ | <LdQ | / | / | / | / |
| M1 | Vanadium | <LdQ | <LdQ | <LdQ | <LdQ | <LdQ | / | / | / | / |
| M1 | Zinc | 0,006855 | 0,006804 | 0,00688 | 0,008096 | 0,0145 | 0,75 | 1,09 | 18,99 | 112,71 |
| | **OTHER ELEMENTS, Total** | 0,035826 | 0,049164 | 0,03758 | 0,056604 | 0,0508 | 27,13 | 23,57 | 15,13 | 3,33 |
| M1 | Aluminum | 0,00085 | 0,001453 | 0,00176 | <LdQ | 0,0021 | 41,48 | 20,92 | / | 45,49 |
| M1 | Antimony | <LdQ | <LdQ | <LdQ | <LdQ | <LdQ | / | / | / | / |
| M1 | Arsenic | <LdQ | <LdQ | <LdQ | <LdQ | <LdQ | / | / | / | / |
| M1 | Barium | 0,00455 | 0,005308 | 0,00493 | 0,005361 | 0,0055 | 14,29 | 7,16 | 0,98 | 4,28 |
| M1 | Boron | 0,002366 | 0,002735 | 0,00244 | 0,002466 | 0,0028 | 13,49 | 10,73 | 9,84 | 3,87 |
| M3 | Bromide | 0,00019 | 0,00027 | 0,00018 | 0,016442 | / | 29,63 | 33,33 | 5989,72 | / |
| M1 | Cadmium | <LdQ | <LdQ | <LdQ | <LdQ | <LdQ | / | / | / | / |
| M1 | Gadolinium | <LdQ | <LdQ | <LdQ | <LdQ | <LdQ | / | / | / | / |
| M1 | Gallium | <LdQ | <LdQ | <LdQ | <LdQ | <LdQ | / | / | / | / |
| M1 | Gold | <LdQ | <LdQ | <LdQ | <LdQ | <LdQ | / | / | / | / |
| M1 | Iridium | <LdQ | <LdQ | <LdQ | <LdQ | <LdQ | / | / | / | / |
| M1 | Lead | <LdQ | <LdQ | <LdQ | <LdQ | nd | / | / | / | / |
| M1 | Lithium | 2,79E-05 | 4,26E-05 | 3,6E-05 | 3,27E-05 | 0,0000 | 34,59 | 16,02 | 23,32 | 12,05 |
| M1 | Lutetium | <LdQ | <LdQ | <LdQ | <LdQ | <LdQ | / | / | / | / |
| M1 | Mercury | <LdQ | <LdQ | <LdQ | <LdQ | <LdQ | / | / | / | / |
| M1 | Rubidium | 0,016844 | 0,022168 | 0,01703 | 0,019779 | 0,0230 | 24,02 | 23,20 | 10,78 | 3,70 |
| M1 | Silver | <LdQ | <LdQ | <LdQ | <LdQ | <LdQ | / | / | / | / |
| M1 | Strontium | 0,008375 | 0,013771 | 0,00873 | 0,01 | 0,0125 | 39,19 | 36,62 | 27,38 | 9,23 |
| M1 | Tellurium | <LdQ | <LdQ | <LdQ | <LdQ | <LdQ | / | / | / | / |
| M1 | Thallium | <LdQ | <LdQ | <LdQ | <LdQ | <LdQ | / | / | / | / |
| M1 | Thorium | <LdQ | <LdQ | <LdQ | <LdQ | <LdQ | / | / | / | / |
| M1 | Titanium | 0,002622 | 0,003414 | 0,00248 | 0,002523 | 0,0047 | 23,20 | 27,35 | 26,09 | 39,02 |
| M1 | Tungsten | <LdQ | <LdQ | <LdQ | <LdQ | <LdQ | / | / | / | / |
| M1 | Uranium | <LdQ | <LdQ | <LdQ | <LdQ | <LdQ | / | / | / | / |
| M1 | Ytterbium | <LdQ | <LdQ | <LdQ | <LdQ | <LdQ | / | / | / | / |

| | | | | | | | | | | |
|---|---|---|---|---|---|---|---|---|---|---|
| *Note 1. Gray boxes indicate chemical macro classes.* *Note 2. %= compound concentration expressed as percentage of the composition.* *Note 3. (d%)= percentage deviation: (*\|*Reference standard (%)-Test(%)*\|/*(Reference standard (%) ) x 100).* *Note 4. The term "Total" refers to the sum of the values of the various compounds which forms the corresponding group.* *Note 5. <LdQ= below the limit of quantification.* *Note 6. nd* = *compound not detected* *Note 7. nq or NQ= compound not quantifiable.* *Note 8.* /*= compound not reported.* | | | | | | | | | | |

The results show that, between the five batches of co-extract, there are quantitative fluctuations (up or down) in the individual chemical classes of components. These fluctuations, if used to predict the performance [or effect] of the individual batches, would lead to an *a priori* view that these batches have different biological function and to the rejection of the batches that are not comparable to the reference standard, if the criteria applied were those valid for an API acting via the keylock paradigm thanks to the presence of an evident SAR. The analysis here reported argues the fact that, despite the biological function being maintained across all the different batches assessed, none of the single molecular components identified would respect the criteria set for a single API, thus demonstrating that the matrix should not be considered as a compilation of APIs.

As seen above, the biological function is preserved in every batch.

Thus, figure 5 shows that it is not representative and therefore not correct to entrust the estimation of the reproducibility of the activity profile of a complex matrix purely on the quantitative analysis of the individual constituents. In fact, given the very nature of a complex matrix, quali-quantitatively different chemical profiles, which, on a chemical standpoint should be considered different, evoke instead, in biological systems, the same reaction relevant for the intended use. This should not be a surprising observation but another demonstration that biological function of a complex matrix cannot be traced back to the sum of the activity of each single molecule within the matrix itself (i.e., the chemical standpoint). Therefore, the activity of the matrix cannot be predicted solely based on the identity and quantity of the molecules which constitute it.

This also highlights the fact that there are both structural and functional redundancy mechanisms that give the matrix particular resilience, i.e., as shown above, the ability to mediate the same activity despite different quali-quantitative compositions. In other words, the study of a matrix from an exclusively molecular point of view is not correct because the identity of its individual components cannot predict its features. This confirms the need for methods capable of describing the intrinsic characteristics of the matrix rather than looking exclusively at its molecular components. Those methods, as the one provided in the present invention, should monitor the preservation of those parameters on which the maintenance of biological function really depends.

### 3.2. Near Infrared Spectroscopy (NIR)

### 3.2.1. Introduction

To create the control chart and provide the NIR acceptability cut-offs, four batches (comprising the reference standard) of Arté GX made on a prototype and industrial scale selected as positive batches according to the method of the invention, steps i) to iv), were analysed and formed the library of good quality samples (training set). The batches used to build the training set were selected after passing the conformity test performed through the biological assay of the invention (see also examples above). Once the library was defined, a batch of Arté GX DEGR 21E1640 selected as negative batch according to the method of the invention, steps i) to iv), was also analysed.

The five batches were used to set the NIR acceptability parameters.

Once the acceptability parameters were defined, two poor formulative qualities as defined above (R19L4299 and R19L4298) as well as an unknown Arté GX batch (21E1640) as test samples were analysed using the same operating methods. Following the result obtained after analysis with conformity tests, the test samples are confirmed as being of good quality or not depending on whether they have passed the conformity test performed via a biological assay to confirm or, possibly, modify the acceptability criteria identified by the control chart" as above.

The aim of this study was to build a control map of NIR spectra, defined a priori through evaluation of conformity in biological function of the batches that are part of it, as a fingerprint to be used to discriminate valid conformity batches of Arté GX from samples of poor biological and/or formulation quality.

Below is the list of the four batches used for the creation of the NIR library and the list of samples analysed as tests:
□ 4 batches Arté GX for the construction of the library (Tab. 1) freeze dried
□ 1 poor quality batch of Arté GX (degraded, poor biological activity quality)
□ 1 batch of Arté GX as library test (Tab. 2) freeze dried
□ 2 poor formulative qualities (Tab. 2)

**Table 1. (Training set).**

| **Product** | **Batch** |
|---|---|
| Arté GX Reference standard | 20B1955 |
| Arté GX | 20I1279 |
| Arté GX | 20J1770 |
| Arté GX | 20B0596 |
| Arté GX | DEGR 21E1640 |

**Table 2. (Test set).**

| **Product** | **Batch** |
|---|---|
| Arté GX | 21E1640 |
| Water extract Centella Asiatica leaves | R19L4299 |
| Water extract Echinacea Purpurea leaves | R19L4298 |

### 3.2.2. instruments

Bruker NIR spectrometers, model MPA (Multi-Purpose Analyzer):
- Resolution: 16 cm-1
- Wavenumber Reproducibility: Better than 0.04 cm-1
- Wavenumber Accuracy: Better than 0.1 cm-1
- Photometric Accuracy: 0.1% T
- Wavenumber range: from 4000 to 12500 cm-1
- Background scans: 64
- Scans for sample acquisition: 64

### 3.3. Statistics tests

### Compliance test

Compliance testing is a simple method for testing deviations of measured NIR spectra within certain limits. To set these limits (acceptability values of NIR spectra), samples of good and bad quality belong to at least one batch or production cycle of the final product to be identified as reference spectra are needed that. According to the present invention, the reference samples are identified through a biological assay deemed appropriate (cell-based assay) and are studied via NIR, to assess the minimum range of specifications such as to include the batches themselves. According to the invention, this range is imposed as acceptable in order to recognize whether a batch of unknown conformity (not tested in the cell-based assay hence not known whether acceptable or not acceptable on the basis of its biological activity) is compliant or not. The NIR spectra of these samples reflect the different variations of the sample capable of achieving compliant and non-compliant performance in terms of modulation of one or more selected biological activity (therapeutic or beneficial activity) and form a confidence band in the spectral range. To pass the NIR conformity test, the spectrum of a new sample must fall within this confidence band. First, the mean and standard deviation of the absorbance values for each wavelength (i) must be calculated. The mean value plus/minus the standard deviation determines the confidence band within the spectral range and defines what amount of variation over each spectral wavelength is acceptable for the product analysed.

Secondly, it is necessary to check whether the spectrum of a sample to be tested falls within the confidence band defined in the spectral range. The difference between this sample and the average of the reference samples is calculated at each wavelength (i). This absolute deviation is then weighted by the corresponding standard deviation "s" on the respective wavelength, which results in a relative deviation called the Conformity Index (CI) equation (1). $CI = \left({A}_{reference , i}-{A}_{sample , i}\right) / {s}_{reference , i}$
A *_{reference, i}* = average absorbance at a given wavelength(*i*) of the reference (average spectrum)
A *_{sample, i}* = absorbance at a given wavelength(*i*) of the test sample
s *_{reference, i}*= standard deviation at a given wavelength(*i*) of the reference (average spectrum)
In conformity tests another parameter can be used to evaluate the batches against the reference library applying the limit of the CI.

This parameter called Sum2 is represented by equation (2): 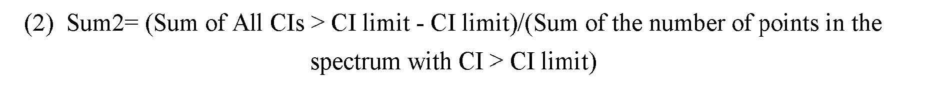 Sum2= (Sum of All CIs > CI limit - CI limit)/(Sum of the number of points in the spectrum with CI > CI limit)

Selecting the appropriate parameter in a conformity test depends on the user-specific control problem that can be easily addressed by the skilled person, in the case of products comprising or consisting of one or more natural matrices, i.e., extremely heterogeneous samples, Sum 2 is a suitable parameter.

Hence, in the present case, Sum2 parameter was selected for determining the acceptability cut-off of the conformity test.

### 3.3.1 Preparation of the sample

Each sample was transferred to the sample holder suitable for the NIR analysis of inhomogeneous solids. Before the analysis, it was checked that the bottom of the sample holder was completely covered.

### 3.3.2 Sample acquisition

The NIR spectra were acquired in reflection mode using a rotating sample holder suitable for the analysis of samples such as inhomogeneous solids, as well as powders, to ensure high reproducibility of the data.

Quality Control and background subtraction was performed before each acquisition. The samples, also reported in the previous tables (Tab. 1 and Tab 2), were prepared as described above and analysed at NIR.

### 3.3.3. Data pre-processing

Pre-processing is a mathematical manipulation to extrapolate spectral features and reduce sources of variability.

For the development of the preprocessing method, the pre-processing method involving the use of SNV normalization was selected and the region of the spectrum from 4200 to 9000 cm-¹ was selected as the region of the spectrum of greatest relevance for the model.

The OPUS Software (Opus 8.5, Bruker) was used for carrying out the conformity test. By applying the pre-processing method the following parameters in the OPUS Conformity Index method were set:
a) Pre-processing: SNV;
b) Regions: From 4200 to 9000 cm-1;
c) Conformity Test parameters: Max Conformity Index Value; Sum2

### 3.3.4. Data acquisition

Four batches indicated in Tab 1 as well as the batches in tab 2 were analysed.

The spectra of the first four batches above were used as reference spectra in the creation of the Conformity Index (CI) as they had the desired biological activity (acceptable according to the cell-based assay).

SNV normalization pre-processing was carried out and the region from 4200 to 9000 cm⁻¹ was selected as the region for data reprocessing. The CI MAX threshold and Sum 2 values of the batches of the card are shown below:

### CIMAX experimental assay (interval based on the biological data (steps i) to iv) of the method of the invention

| **Batch type** | **Batch ID-** | **Cell-based assay** | **CI Max** | **Sum 2** |
|---|---|---|---|---|
| Reference | 20B1955 | REFERENCE STANDARD, COMPLIANT | 1.5 | 0.0 |
| Reference | 20I1279 | COMPLIANT | 1.5 | 0.0 |
| Reference | 20J1770 | COMPLIANT | 1.5 | 0.0 |
| Reference | 20B0596 | COMPLIANT | 1.3 | 0.0 |

### (NIR spectra in Figure 6a)

When referred to the cell-based assay, the tables indicate "compliant" when the batch resulted acceptable according to step c) of the method of the invention.

As reported above, the Max Conformity Index Value is assigned based on the maximum CI MAX value defined by the training consisting of compliant (positive) samples according to the cell-based assay. From the first data (reported above), the CI limit was set to 1.5, this value was hence used to calculate the Sum 2 of the training set and of the test set.

To define the conformity limit of Sum 2, the negative batch (i.e., non-compliant with the biological data (cell-based assay) according to the method of the invention) DEST 21E160 was tested against the control chart. The table below shows the results in terms of CI and Sum 2 for each batch.

| **Batch type** | **Batch ID-** | **Formulative conformity** | **Cell-based assay** | **CI Max** | **Sum 2** |
|---|---|---|---|---|---|
| Reference | 20B1955 | COMPLIANT | COMPLIANT | 1.5 | 0.0 |
| Reference | 20I1279 | COMPLIANT | COMPLIANT | 1.5 | 0.0 |
| Reference | 20J1770 | COMPLIANT | COMPLIANT | 1.5 | 0.0 |
| Reference | 20B0596 | COMPLIANT | COMPLIANT | 1.3 | 0.0 |
| Reference | DEG 21E1640 | COMPLIANT | NOT COMPLIANT | 5.8 | 1.3 |

### (NIR average spectrum of compliant batches plus NIR spectrum of non-compliant batch figure 6b)

Based on the Sum 2 values of batch DEG 21E1640 and its non-compliance with the cell-based assay, the threshold Sum 2 value to define a batch as not compliant is hence set as ≤1.2. Once this acceptability cut-off was set following the method of the invention, the spectra of a new unknown (not verified through cell-based assay) batch of Arté GX and two known formulative non-compliant batches were tested to verify the reliability of the method of the invention and its suitability for a process of quality control according to the description and to the claims.

### Results:

| **Sample type** | **Batch** | **Formulative compliance** | **Cell-based assay** | **CI Max** | **Sum 2 (≤ 1.2)** | **Expected** | **NIR Result** |
|---|---|---|---|---|---|---|---|
| Reference | 20B1955 | COMPLIANT | COMPLIANT | 1.5 | 0.0 | COMPLIANT | COMPLIANT |
| Reference | 20I1279 | COMPLIANT | COMPLIANT | 1.5 | 0.0 | COMPLIANT | COMPLIANT |
| Reference | 20J1770 | COMPLIANT | COMPLIANT | 1.5 | 0.0 | COMPLIANT | COMPLIANT |
| Reference | 20B0596 | COMPLIANT | COMPLIANT | 1.3 | 0.0 | COMPLIANT | COMPLIANT |
| Reference | DEST 21E1640 | COMPLIANT | NOT COMPLIANT | 5.8 | 1.3 | NOT COMPLIANT | NOT COMPLIANT |
| Test | 21E1640 | COMPLIANT | Not assessed | 3.6 | 0.6 | UNKNOWN | COMPLIANT |
| Test | R19L4299 | NOT COMPLIANT | Not assessed | 5.9 | 1.6 | NOT COMPLIANT | NOT COMPLIANT |
| Test | R19L4298 | NOT COMPLIANT | Not assessed | 49.4 | 8.1 | NOT COMPLIANT | NOT COMPLIANT |

Therefore, according to the NIR assay with the acceptability cut-off assessed with the method of the invention, batch 21E1640 resulted compliant according to the validation process of the invention.

In order to confirm the validity of the validation process of the invention, the modulation of the biological activities identified for the product Arté GX described above was carried out also for batch 21E1640 and the product resulted acceptable, i.e. compliant with the reference modulation values according to the method of the invention.

Hence, processes and methods have been identified that allow defining the acceptability criteria of a product comprising or consisting of one or more natural matrices based on the conservation of a number of selected biological activities and regardless of the notion of its composition at the molecular level.

### 3.4 RAMAN spectroscopy

Samples of the same batches of 3.2 were used.

Each sample was deposited between two microscope slides so that the powder created a thickness of approximately 1 mm. The measurements were conducted with Rigaku's Xantus-2 spectrometer in the 2000-200 cm⁻¹ interval, using the using the 1064 nm laser line as an exciter, an excitation power of approximately 100 mW, and collecting the radiation scattered at 180° with respect to the excitation (back-scattering).

### 3.4.1 Acquisition conditions

The following measurement conditions were selected for the acquisition of each spectrum:
Method of acquisition of the sample being analyzed.

| **Sample** | **Sample treatment** | **Spectrum range** | **N° scans** | **Spectrum resolution** |
|---|---|---|---|---|
| Product A | Approximately 100mg were deposited and pressed between two microscope slides | 2000-200 cm⁻¹ | 30 x1s integration time | 7 cm⁻¹ |

For each sample, three samplings were repeated to check the reproducibility of the data: the three replicates were then averaged to obtain a representative spectrum of the sample to be characterized. Average spectrum and standard deviation were obtained with Bruker Optics Opus 8.1 software.

### 3.4.2 Pre-processing

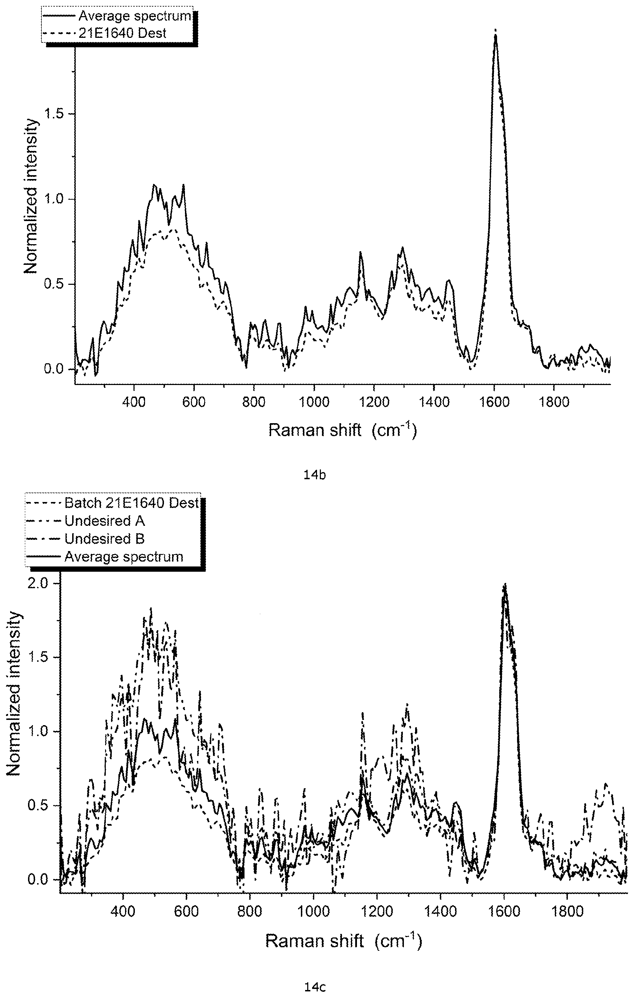

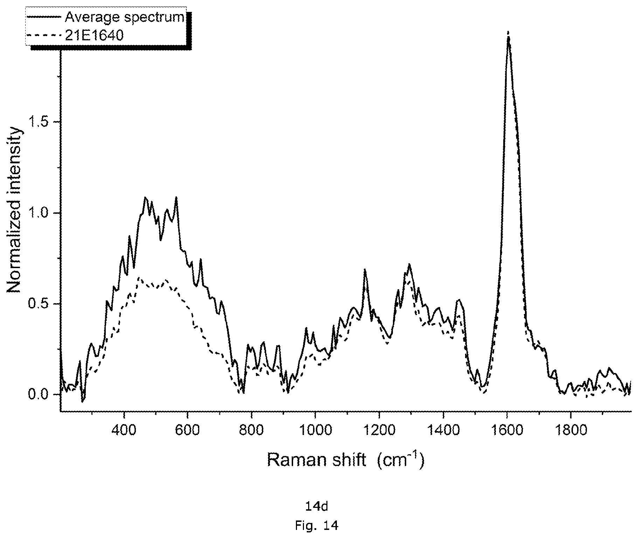

The average spectra thus obtained for all samples were processed to subtract the baseline; for this purpose, the intensity at 200, 790,910, 1520 and 1800 cm⁻¹ was set to zero. Subsequently the spectral profiles were normalized to the intensity of the signal centered at approximately 1610 cm⁻¹ (Figure 14). These manipulations were carried out using Opus 8.1 software from Bruker Optics.

As shown in Figure 14, the spectra show an enlarged signal of notable intensity in the low frequency region: this signal could derive from a residual luminescence of the sample cover glass for which in subsequent processing it was decided to limit the analysis range to region between 780 and 2000 cm⁻¹.

### 3.4.3 Statistical analysis

The statistical analysis of the spectroscopic data was conducted using the Origin2023 Software from OriginLab and following all the steps necessary to obtain the parameters to be used in the conformity tests such as: the CI Conformity Index and the Sum2 parameter using equations (1) and (2) in 3.3 above.

### 3.4.4 Definition of the Control Chart

The construction of the control chart was done using the same criterion adopted for the NIR and ATR-FTIR spectra.

Using the Origin 2023 software, the spectra of 20B1955, 20I1279, 20J1770 and 20B0596 were used as reference spectra: from these the average spectrum with relative standard deviation was obtained. The intensity values (normalized Raman) and standard deviation as a function of frequency constituted the data indicated with I *_{reference, i}* and s *_{reference, i}* in equation (1) of 3.3.

### 3.4.5 Execution of the conformity test

The maximum value of CI as the frequency varied was calculated using equation (1) for each of the reference spectra: in this way it was possible to determine the values of (CI limit) to be used for the calculation of Sum 2 using equation (2). This value was set equal to the maximum CI value obtained for the reference spectra: CI limit = CI Max = 1.50

Using this value, via equation (2) the two conformity tests were applied to the spectra of all the analyzed samples.

The table below summarizes the values found for the Co-extract samples used as Reference Spectra (R) in the creation of the Conformity Index method since they have the desired biological activity, the degraded 21E1640 sample necessary to define the compliance limit of Sum 2, and those of the samples used as method tests (T).

| **Type** | **Batch** | **Formulative compliance** | **Cell-based assay** | **CI Max** | **Sum 2 (≤1.5)** | **Expected** | **RAMAN result** |
|---|---|---|---|---|---|---|---|
| R | _20B1955 | COMPLIANT | COMPLIANT | 1.47 | 0.0 | COMPLIANT | COMPLIANT |
| R | _2011279 | COMPLIANT | COMPLIANT | 1.50 | 0.0 | COMPLIANT | COMPLIANT |
| R | _20J1770 | COMPLIANT | COMPLIANT | 1.50 | 0.0 | COMPLIANT | COMPLIANT |
| R | _20B0596 | COMPLIANT | COMPLIANT | 1.49 | 0.0 | COMPLIANT | COMPLIANT |

| **Type** | **ID** | **Conformità formulativa** | **Cell-based assay** | **CI Max** | **Sum 2 (≤2.09)** | **Expected** | **RAMAN result** |
|---|---|---|---|---|---|---|---|
| R | DEST_21E1640 | COMPLIANT | NON COMPLIANT | 4.95 | 2.10 | NOT COMPLIANT | NOT COMPLIANT |
| T | _21E1640 | COMPLIANT | UNKNOWN | 5.34 | 2.06 | UNKNOWN | COMPLIANT |
| T | _R19L4299 | NOT COMPLIANT | NOT ASSESSED | 6.98 | 2.43 | NOT COMPLIANT | NOT COMPLIANT |
| T | R19L4298 | NOT COMPLIANT | NOT ASSESSED | 29.46 | 5.84 | NOT COMPLIANT | NOT COMPLIANT |

In this case the value of Sum 2 for the degraded sample is slightly higher than those obtained for the non-degraded sample of the same batch. On the basis of this conformity criterion, the unknown sample is compliant as required by the formulation of the co-extract.4. Radical scavenger activity

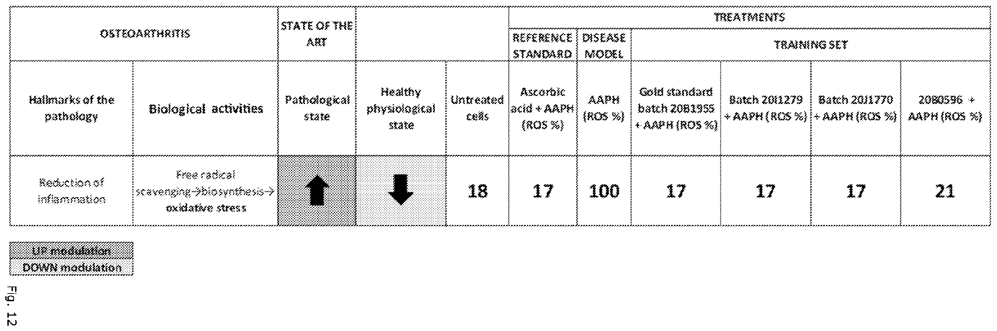

As additional control of the validity of the method and process of the invention, all the Arté GX batches above were also tested for their radical scavenger activity as this activity is known to have a notable promoting effect in osteogenesis. Figure 12 summarises the hallmark and biological activity related to this parameter.

### 4.1. Assay method

The assay involves the use of Human fibroblast (HuDe) cell line as a model to test the ability of products to have an antioxidant activity based on a scavenger activity.

Five different batches of Arté GX were tested at the concentration of 1,4 mg/ml, calculated by applying the dilution factor of 2,8 reflecting product dilution in synovial fluid in an in vivo scenario.

The ROS scavenger activity test is based on the use of the reactive oxygen species (ROS) generator AAPH (2,2'-azobis-2-methyl-propanimidamide, dihydrochloride) which can simulate the appearance of exogenous pro-oxidant damage and thereby induces production of endogenous ROS. Fluorescent probe 6-carboxy-2',7'-dichlorodihydrofluorescein diacetate (H2DCFDA, Life Technologies) was used as indicator of the presence of ROS in cells. The fluorescence emission of H2DCFDA was measured at regular time intervals (every 10 minutes for a total of 90 minutes) with a fluorometer (Varioskan Lux, Thermo-Scientific) and quantitatively correlated to the production of free radicals in cells. To account for cell number at the end of the assay, all of the calculated fluorescence values are normalized with respect to relevant cell viability measured by MTT (tetrazolium salt, [3-(4,5-dimethylthiazol-2yl)-2,5-diphenyltetrazolium bromide, Sigma Aldrich) assay performed according to the manufacturer's instructions. The degree of protection from the generation of ROS provided by the tested products is compared to those obtained by protecting cells with ascorbic acid (considered as benchmark of antioxidant molecule). The results are shown as comparison of integrated area under fluorescence versus time curve (AUC) calculated versus AAPH (considered as 100% of ROS production).

### 4.2. Results

Figure 7 shows equivalent radical scavenger activity of all five batches of Arté GX. All batches show an equivalently highly significant protective effect on fibroblasts.

The above discussed data further qualify the five batches as equivalent in terms of induction of the same biological effect, due to influence on gene expression as well as to radical scavenging activity.

The modulation value (percentage) was calculated as in Figure 12.

All the batches were acceptable with respect to the modulation values identified for the reference standard and therefore the NIR acceptability values calculated as above confirmed the suitability of the methods and process of the invention also using different parameters for monitoring the modulation of the selected biological activity/ies.

### 4. ISOTOPIC ABUNDANCE

### 4.1. Introduction

The analysis of isotopic abundance is a way to describe matter from an atomic point of view. The isotopic distribution characterizing the starting materials may be influenced by phenomena of different nature which in turn may lead to significant variations in the final products [ISPRA, Quaderni - Laboratorio 2/2018. ISBN 978-88-448-0873-0]. The isotopic composition of a sample is equal to the ratio between the abundance of the heavy and light isotopic forms (example the relationship 13C/12C) and is expressed as a deviation, in parts per thousand, from an internationally identified standard reference material. A positive value of δ indicates that the heavy isotope is enriched in the sample compared to the standard, while a negative value indicates that the heavy isotope in the sample is impoverished.

A marked difference in isotopic abundance ratios of a sample compared to samples known to be of good quality can account for different intra- and intermolecular interactions between the phytochemical classes that make up the matrix regardless of the quantitative profile of the individual species and the different chemical reactivity kinetics. In the first case this phenomenon is defined as geometric isotope effect (GIE) and is due in particular to hydrogen binding. In fact, the length of the hydrogen bond with oxygen is smaller than that between deuterium and oxygen. This may involve a different structural rearrangement of both intra- and intermolecular structures.

Isotope abundance also changes the kinetics of reactions, known as Kinetic isotope effect (KIE), which can be either primary or secondary, depending on whether the isotope changes the reaction making it faster or slower than the process of interest. It is therefore clear that the KIE establishes a link between a given isotopic abundance of a material and its ability to interact in a reproducible manner with biological systems. It therefore seems plausible that the analysis of isotopic abundance is a possible tool for monitoring the conformity of the product from a physical-chemical and potentially biological point of view.

In a batch, the presence of alterations in the isotopic abundance ratios may indicate adulteration, poor quality of the product and, when samples representing different intermediates along the production process are considered, possibly a general loss of its desirable native conformation.

In this context, the acquisition of several good-quality batches of the product, together with established conformity verification techniques such as NIR, may lead to the generation of a reference library where individual batches can be evaluated, and isotopic reproducibility verified within established ranges.

In addition, the 14C activity assessment can define the system as 100% natural. This is because the 14C is an unstable isotope (half-life of 5730 years) and, therefore, tends to accumulate in living material while petroleum derivatives have a very low presence/absence of this unstable carbon isotope.

### 4.1.1. Results and discussion

The analysis of isotopic abundance was carried out on the five batches included in the development and validation of the NIR method and thus constituting good quality batches (20B0596, 20B1955, 20I1279, 20J1770, 21E1640), batches 20B0596, 20B1955 were prepared from different batches of starting materials with respect to batches 20I1279, 20J1770, 21E1640.

The samples were sent to the Chelab (Tentamus Company) laboratory and tested for stable isotopes as follows:
- δ18O: Method IRMS, UNIT ‰ V-SMOW.
- δ13C: Method QMA-M-01, EA-IRMS, UNIT ‰ V-PDB.

C14- activity was also tested:
- 14C-activity: Method ISO-16620-2;2015 (AMS), UNIT % modern carbon (pMC).

The results were as follows.

δ ratio of the main isotopes of the co-extract centella-echinacea.

| **Arté GX Batch** | **δ18O** | **δ13C** | **14C-activity (% modern carbon Pmc)** |
|---|---|---|---|
| | | | |
| **20B0596** | 22,6 | -25,54 | 102,35 |
| **20I1279** | 20,3 | -28,64 | 102,53 |
| **20B1955** | 22,3 | -28,83 | 102,28 |
| **20J1770** | 20,6 | -28,54 | 103,09 |
| **21E1640** | 20,8 | -28,51 | 102,56 |

(the values in the table includes the percent error according to the official method used)

The measured values for the C14 activity of the samples of Pmc correspond to those for substances from purely bio-based carbon. There is no evidence of a synthetic source in the analysed material. The C14-activity value perfectly overlaps among batches as it is not affected by the biological variability of the starting materials, thus identifying high reproducibility of the production process according to conservation of this parameter.

On two batches of co-extract (20J1770 e 21E1640) a study of the isotopic abundance during different steps of the manufacturing process was conducted.

The results are reported in the table below depicting the δ ratio of the main isotopes of the coarse plant raw plant parts of the co-extract centella-echinacea.

| **Description** | **δ¹⁸O/¹⁶O** | **δ¹³C/¹²C** |
|---|---|---|
| **Centella leaves, Batch 20H1282** | +20.8 | -28.57 |
| **Echinacea roots, Batch 20F0840** | +22.7 | -28.17 |
| **Mixture Centella leaves + Echinacea roots (batches above)** | +20.9 | -28.86 |
| **Batch 21E1640 (Manufactured in presently used as clinical experimental batch)** | +20.8 | -28.51 |
| **Batch 20J1770** | +20.6 | -28.54 |

The assessment of the isotopic abundance of the materials along the production process shows that the production process does not alter the abundance ratios, thus substantiating the fact that the process conserves the native biophysical characteristics of the starting materials.

Analysis for the batches under study showed substantial similarity of values and maintenance of ratios during the manufacturing process. This is consistent with the NIR results previously described, according to which all batches were similar and merged without outliers to similar spectra.

### 5. RNA evaluation in production intermediate

Biophysical characterization of biological vegetal material includes the evaluation of biological material in production intermediates.

"RIC199EL0, extract_BLEND CENT_ECH EL, batch R20I4716" which corresponds to a production intermediate of Arté GX, i.e., the Centella asiatica and Echinacea water coextract in the proportions depicted in example 1, before ultrafiltration. The presence of RNA has been evaluated both quantitatively and qualitatively. RNA was extracted using a plant matrix specific kit (RNeasy PowerPlant kit) after homogenization with the use of QIAshredder columns before proceeding with the kit extraction protocol. The dimensional distribution of the RNA obtained was performed using Bioanalyzer 2100 with RNA 6000 Nano, RNA 6000 Pico and small RNA kits.

In Figure 13, the electropherogram "A" shows the size distribution of the total RNA and the electropherogram "B" shows the RNA size distribution between 4 and 150 nt.

Then, to quantitatively assess the total RNA extracted from the sample, a nucleic acid digestion was performed using the New England Biolabs nucleoside digestion Mix kit.The RNA concentration was expressed as total nucleosides by UHPLC-qToF analysis. The Table below reports RNA expressed as total nucleosides.

### BLEND CENT_ECH EL

| | **µg/g** | **%** |
|---|---|---|
| **Guanosine** | 9,20 | 41,39 |
| **Adenosine** | 3,51 | 15,79 |
| **Uridine** | 2,06 | 9,27 |
| **Cytidine** | 7,46 | 33,56 |
| **Total** | 22,23 | 100 |

These observations, in addition to providing a method for validating the biological origin of a matrix, identify an additional degree of both structural and functional complexity to be considered in product management.

For a prompt reference and confrontation, the experimental protocols used for the three different products described in example 1 are summarised in the table hereinbelow.

| | **Arté GX (reference model)** | **PRODUCT B** | **PRODUCT C** | |
|---|---|---|---|---|
| **CELL BASED ASSAY** | *2. In vitro cell assay representative of Osteoarthritis* | Human SH-SY5Y (ATCC^{®} No. CRL-2266^{™}) neuroblastoma cells are a commonly used as a neuronal model, as they maintain several neuron markers [Barbosa, D.J.; Capela, J.P.; de Lourdes Bastos, M.; Carvalho, F. In vitro models for neurotoxicology research. [Toxicol. Res. 2015, 4, 801-842*]*. | Human, adipocyte-derived, mesenchymal stem cell lines (hADMSC), capable of differentiating into osteoblasts and mineralize the extracellular matrix (ECM) were used in this study. These cells were obtained during general surgery from three different patients (PA42, PA59 and PA69) (Romagnoli et al, "In vitro Behavior of Human Adipose Tissue-Derived Stem Cells on Poly(ε-caprolactone) Film for Bone Tissue Engineering Applications", BioMed Research International, vol. 2015, Article ID 323571, 12 pages, 2015. https://doi.org/10.1155/2015/323571). | |
| | An *in vitro* cellular model capable of recapitulating features of osteoarthritis [1-3] was established by exposing primary human chondrocytes (HC, Cell Application INC 402K-05) to IL1B [5 ng/ml] for 6 hours. | | | |
| | insulted cells were exposed for 24 hours to five different batches of "Arté-Gx" [1.4 mg/ml]: | | These cell lines have been characterized with respect to the main stemness markers of mesenchymal stem cells (CD44, CD105, and STRO1) and by studying their multipotency toward osteogenic phenotypes at the Department of Surgery and Translational medicine of the University of Florence. | |
| | | The cells were exposed for 24 hours to product B at 1.07 mg/ml in the appropriate cell culture medium. | | |
| | Batch 20B1955 (reference standard) | | | |
| | Batch 2011279 | | | |
| | Batch 20J1770 | | hADMSCs were cultured in a growth medium (GM) and grown to 70-80% confluence. Afterwards, the cells were seeded in 24-well plate at a concentration of 1 × 10⁵ cells/well. After a week, the GM was replaced with osteogenic medium (OM) containing the | |
| | Batch 21E1640 | | | |
| | Batch 20B0596 | | | |
| | Batch 21E1640 Dest | | | |
| | Each time one of the batch solutions was added, fresh IL1B [5 | | | |
| | ng/ml] was also added to the medium. | | fluorophore calcein 1µg/mL and incubated for 28 days with or without product C. The medium with or without the product C was refreshed twice a week. | |
| **DEFINITION OF THE PATHO-PHYSIOLOGICAL HALLMARKS OF THE DISEASE WITH WHICH TO INTERROGATE IPA** | The patho-physiological features of state-of-the-art "Osteoarthritis condition" were considered with particular attention to the following areas involved: | The alteration of healthy physiological state features of state-of-the-art "Mild Cognitive Impairment" were considered with particular attention to the following areas involved: | The alteration of healthy physiological state features of state-of-the-art "Osteoporosis" were considered with particular attention to the following areas involved: | |
| | | | | - Remodelling of bone |
| | | | | - Osteoporosis |
| | | | | - Differentiation of osteoblasts |
| | | | | - Mineralization |
| | -inflammatory process, | | | - Inflammation |
| | | -Cognition | | - Funcitonality of adipose tissue |
| | -cellular proliferation, | -Activation and viability | This knowledge was used to interrogate IPA via the "IPA Bioprofiler" tool, using the following keywords: osteoporosis, postmenopausal osteoporosis, calcification of bone, osteoblast and osteoclast differentiation, bone mineral density. | |
| | -anatomical damage, | | | |
| | -oxidative stress | -Myelination and branching | | |
| | This knowledge was used to interrogate IPA via the "IPA Bioprofiler" tool, using the following keywords: | | | |
| | | -Reduction of inflammation | | |
| | | -Skeletal and muscular system function | | |
| | osteoarthritis, arthropathy, formation of cartilage tissue, destruction of cartilage tissue, damage of cartilage, connective tissue disorder, inflammation of joint, immune cell trafficking. | This knowledge was used to interrogate IPA via the "IPA Bioprofiler" tool, using the following keywords: | | |
| | | neurodegeneration, cognitive deficit, memory deficit, function of muscle, oxidative stress and inflammatory process. | | |

| | **Arté GX (reference model)** | **PRODUCT B** | **PRODUCT C** |
|---|---|---|---|
| **GENE EXPRESSION ANALYSIS** | *2.1.2. Gene expression analysis* | At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene | *2.1.2. Gene expression analysis* |
| | At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene expression analysis experiments. Total RNA was extracted from cells lysates | | At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene expression |
| | using an QIAsymphony RNA Kit (Qiagen,) with the QIAsymphony SP instrument (Qiagen). | expression analysis experiments. Total RNA was extracted from cells lysates using an QIAsymphony RNA Kit (Qiagen,) with the QIAsymphony SP instrument (Qiagen). The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using RNA-Seq data obtained with Illumina NextSeq, sequenced in paired-end mode. | analysis experiments. Total RNA was extracted from cells lysates using an QIAsymphony RNA Kit (Qiagen,) with the QIAsymphony SP instrument (Qiagen). The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific). |
| | The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific). | | |

| | **Arté GX (reference model)** | **PRODUCT B** | **PRODUCT C** |
|---|---|---|---|
| **TRANSCRIPTOMICS DATA ANALYSIS** | *2.1.3. Transcriptomics data analysis* | Sample was mapped on reference genome using the bioinformatics tool | *2.1.3. Transcriptomics data analysis* |
| | Data analysis was performed using Transcriptomic Analysis | | Data analysis was performed using |
| | Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05). | STAR (version 2.7.0f), with the standard parameters for paired reads. The reference track was the assembly Human obtained from GenCode (HG38). The quantification of transcripts expressed for each sequenced sample was performed using featureCount algorithm. | Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05). |
| | | R was used to create a matrix of all genes expressed in all samples with the corresponding read-counts and the Bioconductor package DESeq2 was used to normalize the data, using the median of ratio, to perform the differential expression analysis. Quality control check such as Euclidean distances (Heatmap Distances) and Principal component analysis (PCA) were performed among all samples in each condition considered. | |

### 5. CONCLUSIONS

Taken together, the data here reported allow to identify acceptability parameters which are key to guarantee the quality of the production process of a product comprising or consisting of one or more matrix of biological origin (natural matrix). Intriguingly, the data shows that monitoring exclusively the reproducibility of the composition at the molecular level does not represent a rewarding strategy, as such a strategy ignores key features of the matrix that impact its ability to induce a reproducible effect when this is used to treat a biological system. Presumably due to the presence of redundancy effects between molecular components at both the structural and functional levels, and due to the presence of a network of physical and functional interactions within the complex matrix, its biological function strongly depends on features better monitored by analysing carefully selected biophysical properties of the matrix.

Very eloquently, the exclusive use of targeted metabolomics data, especially when judged according to the principles usually reserved to single APIs, is not capable of predicting the capability of different batches of the same product to elicit reproducible biological effects (see figure 5 vs figures 3 and 4). By applying targeted metabolomics alone, such batches are incorrectly perceived as unacceptably different from one another. In contrast, the application of techniques monitoring features of the matrix that are emergent and deriving from networks of interactions, therefore different from and only partially dependent on conservation of the single molecular components, as disclosed in the present application, correctly identifies a satisfactory degree of similarity between the different batches analysed and express consistency with the fact that they indeed elicit reproducible, desirable biological effects.

## Claims

1. A method for the compliance validation of one or more batches of a product for the treatment of a pathological condition or for adjuvating homeostasis in an altered physiological state, said product consisting of one or more natural matrices, the method comprising
(a) performing at least one *in vitro* cell-based assay on a reference standard batch of said product having a known therapeutic effect for treatment of said pathological condition, and on one or more test batches of said product, wherein the read-out of said cell-based assay is representative of the modulation of one or more biological activity associated with one or more hallmarks of said pathological condition or of a pathological condition that can develop from said altered physiological state,
(a1) determining for each of said one or more biological activities the modulation trend thereof for restoring a healthy physiological state and,
(a2) performing a transcriptomic analysis determining the genes and modulation trend thereof underlying the modification detectable in said pathological condition in both diseased and healthy physiological state for each of said biological activities;
(b) quantifying in terms of numerical values the modulation of said one or more biological activities induced by each batch of step (a) for each cell-based assay read-out and defining as reference values the values calculated for said reference standard batch;
(c) defining as acceptable the test batches whose values calculated in (b) induce a modulation of each measured biological activity in said cell-based assay whose modulus is ≥ to the modulus of the reference value calculated in (b) and defining as non-acceptable the batches whose values calculated in (b) induce a modulation of at least one of said biological activities in said cell-based assay whose modulus is < to the modulus of the reference value calculated in (b);
(d) performing a NIR or RAMAN spectroscopy on said reference standard batch and on said one or more different test batches; and
(e) defining the acceptability values of the NIR or RAMAN spectroscopy spectra thereby obtained, as the variability range of the NIR or RAMAN spectroscopy spectra values of all of said acceptable batches and of said reference standard batch, preferably refined by the spectra of said non-acceptable batches;
(f) performing a NIR or RAMAN spectroscopy analysis of further batches of said product, and
(g) validating each of said further batches for which the obtained spectrum satisfies the acceptability values defined in step (e).

2. The method according to claim 1 further comprising
in step (a) performing said *in vitro* cell-based assay on one or more reference drug for the treatment of said pathological condition,
in step (b) quantifying in terms of numerical values the modulation induced in step (a) by each reference drug for each cell-based assay read-out and, for each biological activity modified by said reference standard and by said one or more reference drug according to said modulation trend for restoring a healthy state, comparing the value calculated for each reference drug and for the reference standard and selecting as reference value, the value associated to the weakest performance.

3. The method of any one of claim 1-2, wherein the cell-based assay is designed to simulate conditions related to said pathological condition or to said altered physiological state.

4. The method according to anyone of claims 1 to 3 wherein said one or more test batches are at least three test batches.

5. The method according to anyone of claims 1 to 4 wherein said product comprises one or more of: cut or pulverized plant parts, plant extracts , fractions of said extracts, fractions obtained by filtration on a semi-permeable membrane, or those obtained by treatment on adsorption resins, or microorganisms, honey, propolis, silk, wax, plant resins, plant gums, plant exudates, vegetable oils, vegetable essential oils, animal tissues lysates, plant or animal fluids.

6. The method according to anyone of claims 1 to 5 wherein said product is: a medical device, a food supplement, or a medicament.

7. The method according to anyone of claims 1 to 6 wherein said pathological condition comprises osteoarthritis, mild cognitive impairment, post menopausal osteoporosis or cancer.

## Patentansprüche

1. Verfahren zur Konformitätsvalidierung einer oder mehrerer Chargen eines Produkts zur Behandlung eines pathologischen Zustands oder zur Unterstützung der Homöostase in einem veränderten physiologischen Zustand, wobei das Produkt aus einer oder mehreren natürlichen Matrizen besteht, wobei das Verfahren umfasst
(a) Durchführen mindestens eines zellbasierten *In-vitro*-Assays an einer Referenzstandardcharge des Produkts, das eine bekannte therapeutische Wirkung zur Behandlung des pathologischen Zustands aufweist, und an einer oder mehreren Testchargen des Produkts, wobei das Ausleseergebnis des zellbasierten Assays repräsentativ für die Modulation einer oder mehrerer biologischer Aktivitäten ist, die mit einem oder mehreren Kennzeichen des pathologischen Zustands oder eines pathologischen Zustands, der sich aus dem veränderten physiologischen Zustand entwickeln kann, verbunden sind,
(a1) Bestimmen des Modulationstrends für jede der einen oder mehreren biologischen Aktivitäten zur Wiederherstellung eines gesunden physiologischen Zustands und,
(a2) Durchführen einer Transkriptomanalyse zum Bestimmen der Gene und deren Modulationstrends, die der in dem pathologischen Zustand nachweisbaren Modifikation sowohl im erkrankten als auch im gesunden physiologischen Zustand für jede der biologischen Aktivitäten zugrunde liegen;
(b) Quantifizieren der durch jede Charge von Schritt (a) induzierten Modulation der einen oder der mehreren biologischen Aktivitäten in Form von numerischen Werten für jedes Ausleseergebnis des zellbasierten Assays und Definieren der für die Referenzstandardcharge berechneten Werte als Referenzwerte;
(c) Definieren der Testchargen als akzeptabel, deren in (b) berechnete Werte eine Modulation jeder gemessenen biologischen Aktivität in dem zellbasierten Assay induzieren, deren Betrag ≥ dem Betrag des in (b) berechneten Referenzwerts ist, und Definieren der Chargen als nicht akzeptabel, deren in (b) berechnete Werte eine Modulation mindestens einer der biologischen Aktivitäten in dem zellbasierten Assay induzieren, deren Betrag < dem Betrag des in (b) berechneten Referenzwerts ist;
(d) Durchführen einer NIR- oder RAMAN-Spektroskopie an der Referenzstandardcharge und an der einen oder den mehreren verschiedenen Testchargen; und
(e) Definieren der Akzeptanzwerte der dadurch erhaltenen NIR- oder RAMAN-Spektroskopiespektren als den Variabilitätsbereich der NIR- oder RAMAN-Spektroskopiespektrenwerte aller der akzeptablen Chargen und der Referenzstandardcharge, vorzugsweise verfeinert durch die Spektren der nicht akzeptablen Chargen;
(f) Durchführen einer NIR- oder RAMAN-Spektroskopieanalyse weiterer Chargen des Produkts, und
(g) Validieren jeder der weiteren Chargen, für die das erhaltene Spektrum die in Schritt (e) definierten Akzeptanzwerte erfüllt.

2. Verfahren nach Anspruch 1, ferner umfassend
in Schritt (a) Durchführen des zellbasierten *In-vitro*-Assays an einem oder mehreren Referenzarzneimitteln zur Behandlung des pathologischen Zustands,
in Schritt (b) Quantifizieren der in Schritt (a) durch jedes Referenzarzneimittel induzierten Modulation für jedes Ausleseergebnis des zellbasierten Assays in Form von numerischen Werten und, für jede biologische Aktivität, die durch den Referenzstandard und durch das eine oder die mehreren Referenzarzneimittel gemäß dem Modulationstrend zur Wiederherstellung eines gesunden Zustands modifiziert wird, Vergleichen des für jedes Referenzarzneimittel und für den Referenzstandard berechneten Werts und Auswählen des mit der schwächsten Leistung verbundenen Werts als Referenzwert.

3. Verfahren nach einem der Ansprüche 1 bis 2, wobei der zellbasierte Assay dazu ausgelegt ist, Bedingungen zu simulieren, die sich auf den pathologischen Zustand oder den veränderten physiologischen Zustand beziehen.

4. Verfahren nach einem der Ansprüche 1 bis 3, wobei die eine oder die mehreren Testchargen mindestens drei Testchargen sind.

5. Verfahren nach einem der Ansprüche 1 bis 4, wobei das Produkt eines oder mehrere umfasst von: geschnittene oder pulverisierte Pflanzenteile, Pflanzenextrakte, Fraktionen der Extrakte, durch Filtration an einer semipermeablen Membran erhaltene Fraktionen, oder solche, die durch Behandlung an Adsorptionsharzen erhalten werden, oder Mikroorganismen, Honig, Propolis, Seide, Wachs, Pflanzenharze, Pflanzengummis, Pflanzenexsudate, pflanzliche Öle, pflanzliche ätherische Öle, tierische Gewebelysate, pflanzliche oder tierische Flüssigkeiten.

6. Verfahren nach einem der Ansprüche 1 bis 5, wobei das Produkt: ein Medizinprodukt, ein Nahrungsergänzungsmittel oder ein Arzneimittel ist.

7. Verfahren nach einem der Ansprüche 1 bis 6, wobei der pathologische Zustand Osteoarthritis, leichte kognitive Beeinträchtigung, postmenopausale Osteoporose oder Krebs umfasst.

## Revendications

1. Procédé permettant la validation de conformité d'un ou de plusieurs lots d'un produit destiné au traitement d'un état pathologique ou au maintient de l'homéostasie dans un état physiologique altéré, ledit produit étant constitué d'une ou de plusieurs matrices naturelles, le procédé comprenant
(a) la réalisation d'au moins un essai *in vitro* basé sur des cellules sur un lot étalon de référence dudit produit ayant un effet thérapeutique connu pour le traitement dudit état pathologique, et sur un ou plusieurs lots d'essai dudit produit, dans lequel la lecture dudit essai basé sur des cellules est représentative de la modulation d'une ou de plusieurs activités biologiques associées à une ou plusieurs caractéristiques dudit état pathologique ou d'un état pathologique qui peut se développer à partir dudit état physiologique altéré,
(a1) la détermination, pour chacune desdites une ou plusieurs activités biologiques, de la tendance de modulation de celles-ci afin de rétablir un état physiologique sain et,
(a2) la réalisation d'une analyse transcriptomique déterminant les gènes et la tendance de modulation de ceux-ci sous-jacents à la modification détectable dans ledit état pathologique à la fois dans un état physiologique malade et sain pour chacune desdites activités biologiques ;
(b) la quantification, en termes de valeurs numériques, de la modulation de ladite ou desdites activités biologiques induites par chaque lot de l'étape (a) pour chaque lecture d'essai basé sur des cellules et la définition, comme valeurs de référence, des valeurs calculées pour ledit lot étalon de référence ;
(c) la définition, comme acceptables, des lots d'essai dont les valeurs calculées à l'étape (b) induisent une modulation de chaque activité biologique mesurée dans ledit essai basé sur des cellules dont le module est ≥ au module de la valeur de référence calculée à l'étape (b) et la définition, comme non acceptables, des lots dont les valeurs calculées à l'étape (b) induisent une modulation d'au moins l'une desdites activités biologiques dans ledit essai basé sur des cellules dont le module est < au module de la valeur de référence calculée à l'étape (b) ;
(d) la réalisation d'une spectroscopie NIR ou RAMAN sur ledit lot étalon de référence et sur ledit ou lesdits lots d'essai différents ; et
(e) la définition des valeurs d'acceptabilité des spectres de spectroscopie NIR ou RAMAN ainsi obtenus, comme plage de variabilité des valeurs de spectres de spectroscopie NIR ou RAMAN de tous lesdits lots acceptables et dudit lot étalon de référence, de préférence affinée par les spectres desdits lots non acceptables ;
(f) la réalisation d'une analyse par spectroscopie NIR ou RAMAN d'autres lots dudit produit, et
(g) la validation de chacun desdits lots supplémentaires pour lesquels le spectre obtenu satisfait aux valeurs d'acceptabilité définies à l'étape (e).

2. Procédé selon la revendication 1, comprenant en outre
à l'étape (a) la réalisation dudit essai *in vitro* sur les cellules sur un ou plusieurs médicaments de référence pour le traitement dudit état pathologique,
à l'étape (b) la quantification en termes de valeurs numériques de la modulation induite à l'étape (a) par chaque médicament de référence pour chaque lecture d'essai basé sur des cellules et, pour chaque activité biologique modifiée par ledit étalon de référence et par ledit ou lesdits médicaments de référence selon ladite tendance de modulation pour le rétablissement d'un état sain, la comparaison de la valeur calculée pour chaque médicament de référence et pour l'étalon de référence et le choix comme valeur de référence, de la valeur associée à la performance la plus faible.

3. Procédé selon l'une quelconque des revendications 1 à 2, dans lequel l'essai basé sur des cellules est conçu pour simuler des conditions liées audit état pathologique ou audit état physiologique altéré.

4. Procédé selon l'une quelconque des revendications 1 à 3, dans lequel lesdits un ou plusieurs lots d'essai sont au moins trois lots d'essai.

5. Procédé selon l'une quelconque des revendications 1 à 4 dans lequel ledit produit comprend un ou plusieurs parmi : des parties de plantes coupées ou pulvérisées, des extraits de plantes, des fractions desdits extraits, des fractions obtenues par filtration sur une membrane semi-perméable, ou celles obtenues par traitement sur résines d'adsorption, ou des micro-organismes, du miel, du propolis, de la soie, de la cire, des résines végétales, des gommes végétales, des exsudats de plantes, des huiles végétales, des huiles essentielles végétales, des lysats de tissus animaux, des fluides végétaux ou animaux.

6. Procédé selon l'une quelconque des revendications 1 à 5, dans lequel ledit produit est : un dispositif médical, un complément alimentaire ou un médicament.

7. Procédé selon l'une quelconque des revendications 1 à 6, dans lequel ledit état pathologique comprend de l'arthrose, une déficience cognitive légère, une ostéoporose post-ménopausique ou un cancer.
